# EUROPEAN PATENT APPLICATION

(11) **EP 4 603 489 A1**
(43) Date of publication of application: **20.08.2025**
(21) Application number: 23877362.6
(22) Date of filing: 13.10.2023
(51) Int. Cl.: C07D 409/14, A61K 31/4178, A61K 31/427, A61K 31/4436, A61K 31/4439, A61K 31/497, A61K 31/506, A61P 9/02, C07D 401/12, C07D 407/12, C07D 409/12, C07D 417/12, C07D 417/14

(54) **IMIDAZOLE DERIVATIVE**

(30) Priority: 14.10.2022 JP 2022165485
(71) Applicant: SANWA KAGAKU KENKYUSHO CO., LTD., Aichi 461-8631 (JP)
(72) Inventor: SAKAI, Keita, Nagoya-shi, Aichi 461-8631 (JP); KAWAKAMI, Yuriko, Nagoya-shi, Aichi 461-8631 (JP); TAKASU, Noriaki, Nagoya-shi, Aichi 461-8631 (JP); MAEDA, Hiroyuki, Nagoya-shi, Aichi 461-8631 (JP); TANAKA, Gaku, Nagoya-shi, Aichi 461-8631 (JP); NAGANO, Hiroki, Nagoya-shi, Aichi 461-8631 (JP); HASHIMOTO, Shinji, Nagoya-shi, Aichi 461-8631 (JP); TORII, Masafumi, Nagoya-shi, Aichi 461-8631 (JP); MORIMOTO, Nobutaka, Nagoya-shi, Aichi 461-8631 (JP); MIYAZATO, Kenji, Nagoya-shi, Aichi 461-8631 (JP)
(74) Representative: M. Zardi & Co S.A.
(86) International application number: PCT/JP2023/037190
(87) International publication number: WO 2024/080361

(57) **Abstract**

The present invention relates to a compound represented by the following general formula (I) or pharmacologically acceptable salt thereof. The present compound has an excellent adrenergic α1A receptor agonistic action, and is useful as a preventive or therapeutic drug for orthostatic hypotension, essential hypotension, acute hypotension associated with various diseases or conditions, and urinary incontinence. 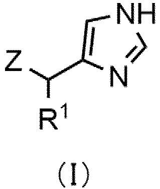 [In the formula, R¹ represents a hydrogen atom or a C₁-C₃ alkyl group; and Z is selected from the group consisting of a substituted phenylamino group, a substituted phenyloxy group, a substituted phenylthio group, a substituted thienylamino group, and the like.]

## Description

### [Technical Field]

The present invention relates to imidazole derivatives and pharmaceutical application thereof.

### [Background Art]

Epinephrine and norepinephrine, a type of neurotransmitters collectively referred to as catecholamines, are mainly secreted from the adrenal medulla and sympathetic nerve endings, and are involved in sympathetic nerve signaling. Adrenergic receptors involved in the onset of action are roughly divided into α receptor and β receptor, and the α receptor has two subtypes: α1 and α2. Of these, the α1 receptor further has three subtypes: α1A receptor, α1B receptor, and α1D receptor; all of which are involved in smooth muscle contraction. Among them, it has been reported that the activation of the α1A receptor strongly contributes to increasing blood pressure (Non Patent Literatures 1 and 2).

Since the activation of the α1 receptor causes the vascular smooth muscle to contract, α1 receptor agonists are clinically used as hypertensive drugs. Currently, midodrine, etilefrine, phenylephrine, and the like are known as the clinically used α1 receptor agonists; however, their types are limited.

As the α1 receptor agonists, N-((1H-imidazol-4-yl)methyl)-2-(oxazol-5-yl)aniline and 5-(2-((1H-imidazol-4-yl)methoxy)phenyl)isoxazole (Non Patent Literature 3) have been reported. These compounds have α1A agonistic action; however, they are different in structure from the compounds of the invention of the present application, which have no oxazole rings nor an isoxazole rings in the biaryl structure.

### [Citation List]

### [Non Patent Literature]

[Non Patent Literature 1] Circulation 100 2336-2343 1999
[Non Patent Literature 2] J Pharmacol Exp Ther 274 97-103 1995
[Non Patent Literature 3] Bioorg Med Chem Lett 12 3449-3452 2002

### [Summary of Invention]

### [Technical Problems]

Since the activation of α1A receptor plays an important role in increasing blood pressure, the present invention aims to provide a novel compound that can be used as a hypertensive drug and that exhibits a strong agonistic action on the α1A receptor. In other words, the present invention aims to provide a compound that can be an excellent therapeutic or preventive drug for orthostatic hypotension, essential hypotension, acute hypotension associated with various diseases or conditions, and urinary incontinence, based on activation of the α1A receptor.

### [Solution to Problems]

To solve the above problems, the present inventors have repeated extensive researches to develop an adrenergic α1A receptor agonist with a novel structure. As a result, the present inventors have found that a compound represented by the following general formula (I) and a pharmacologically acceptable salt thereof have significantly excellent adrenergic α1A receptor agonistic action, leading to the completion of the present invention.

According to the present invention, provided is the compound represented by the following general formula (I) or a pharmacologically acceptable salt thereof. As used herein, the compound and the pharmacologically acceptable salt thereof are referred to as the "present compound". The present invention can be shown as the following embodiments (1) to (18) or the like.

(1) A compound represented by the following general formula (I) or a pharmacologically acceptable salt thereof: 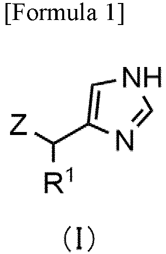
   [wherein R¹ represents a hydrogen atom or a C₁-C₃ alkyl group,
   Z is selected from the group consisting of the following formulas: 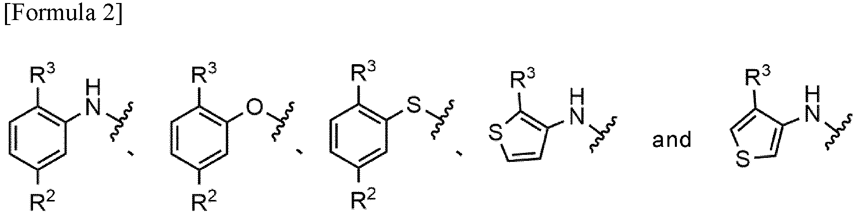
   wherein R² represents a hydrogen atom, a fluorine atom, or a C₁-C₃ alkyl group,
   R³ is selected from the group consisting of the following formulas: 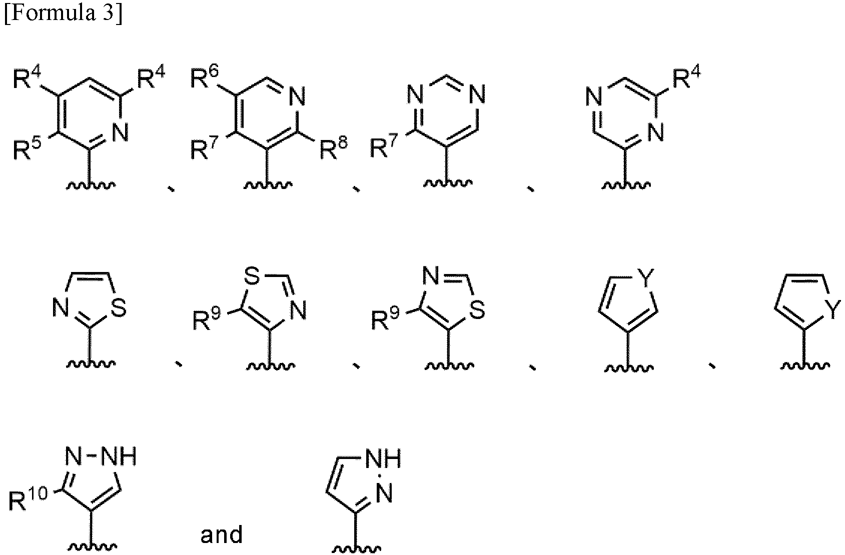
   wherein Y represents an oxygen atom or a sulfur atom,
   R⁴ each independently represents a hydrogen atom or a C₁-C₃ alkoxy group,
   R⁵ represents a hydrogen atom, a fluorine atom, a C₁-C₃ alkyl group, or a C₁-C₃ alkoxy group,
   R⁶ represents a hydrogen atom, a fluorine atom, or a C₁-C₃ alkoxy group,
   R⁷ represents a hydrogen atom, a C₁-C₃ alkyl group, a C₁-C₃ alkoxy group, or a cyclopropyl group,
   R⁸ represents a hydrogen atom or a C₁-C₃ alkoxy group,
   R⁹ represents a hydrogen atom or a C₁-C₃ alkyl group,
   R¹⁰ represents a hydrogen atom, a C₁-C₃ alkyl group, a C₁-C₃ alkoxy group, or a cyano group, and
   when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom].
(2) A compound represented by the following general formula (I) or a pharmacologically acceptable salt thereof: 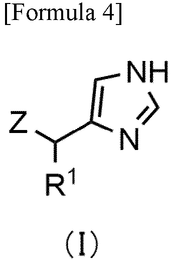
   [wherein R¹ represents a hydrogen atom or a C₁-C₃ alkyl group,
   Z is selected from the group consisting of the following formulas: 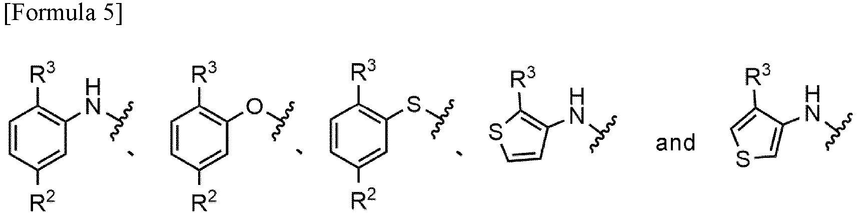
   wherein R² represents a hydrogen atom, a fluorine atom, or a C₁-C₃ alkyl group,
   R³ is selected from the group consisting of the following formulas: 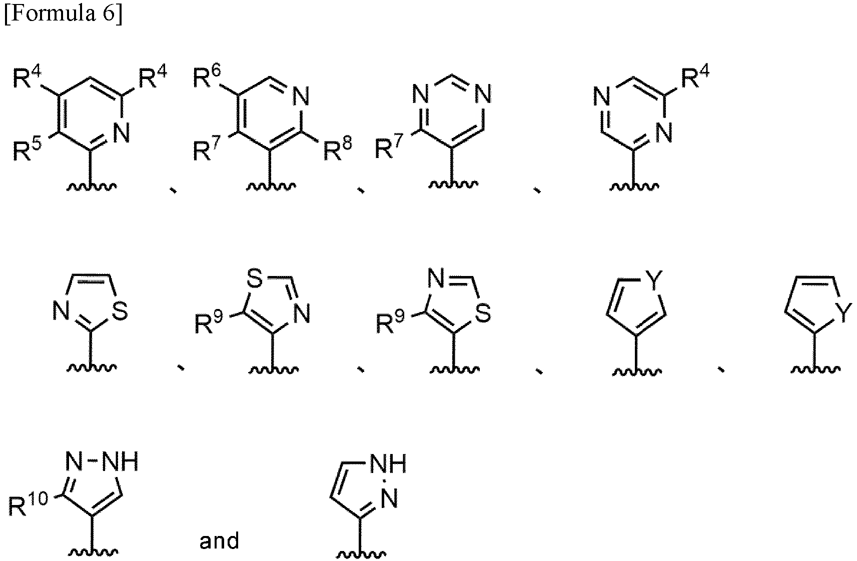
   wherein Y represents an oxygen atom or a sulfur atom,
   R⁴ each independently represents a hydrogen atom or a C₁-C₃ alkoxy group,
   R⁵ represents a hydrogen atom, a fluorine atom, a C₁-C₃ alkyl group, or a C₁-C₃ alkoxy group,
   R⁶ represents a hydrogen atom, a fluorine atom, or a C₁-C₃ alkoxy group,
   R⁷ represents a hydrogen atom, a C₁-C₃ alkyl group, a C₁-C₃ alkoxy group, or a cyclopropyl group,
   R⁸ represents a hydrogen atom or a C₁-C₃ alkoxy group,
   R⁹ represents a hydrogen atom or a C₁-C₃ alkyl group,
   R¹⁰ represents a hydrogen atom, a C₁-C₃ alkyl group, a C₁-C₃ alkoxy group, or a cyano group, and
   when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom,
   provided that when Z is: 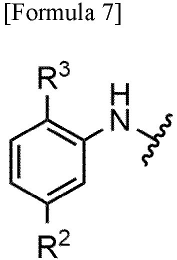 , and R² is a hydrogen atom, R³ is not 3-fluoropyridine bound at position 2;
   when Z is: 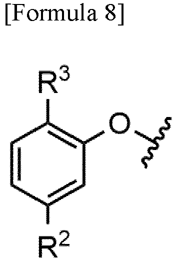 , and R² is a hydrogen atom, R³ is neither non-substituted pyridine bound at position 2, non-substituted pyrazole bound at position 3, nor 3-methoxypyrazole bound at position 4;
   when Z is: 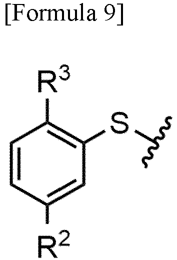 , and R² is a hydrogen atom, R³ is not non-substituted furan bound at position 2;
   when Z is: 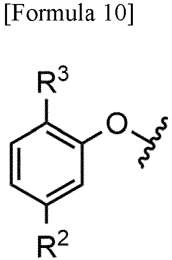 , R¹ is a C₁-C₃ alkyl group and R² is a hydrogen atom, R³ is not non-substituted pyrazole bound at position 4; and
   when Z is: 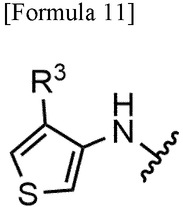 , R³ is not non-substituted thiazole bound at position 5, and
   of two enantiomers that occur when R¹ is a C₁-C₃ alkyl group, the enantiomer having a weaker α1A agonistic action than 4-(2-((1H-imidazol-4-yl)methoxy)phenyl)-1H-pyrazole is excluded].
(3) The compound or pharmacologically acceptable salt thereof according to (2), wherein R³ is selected from the group consisting of the following formulas: 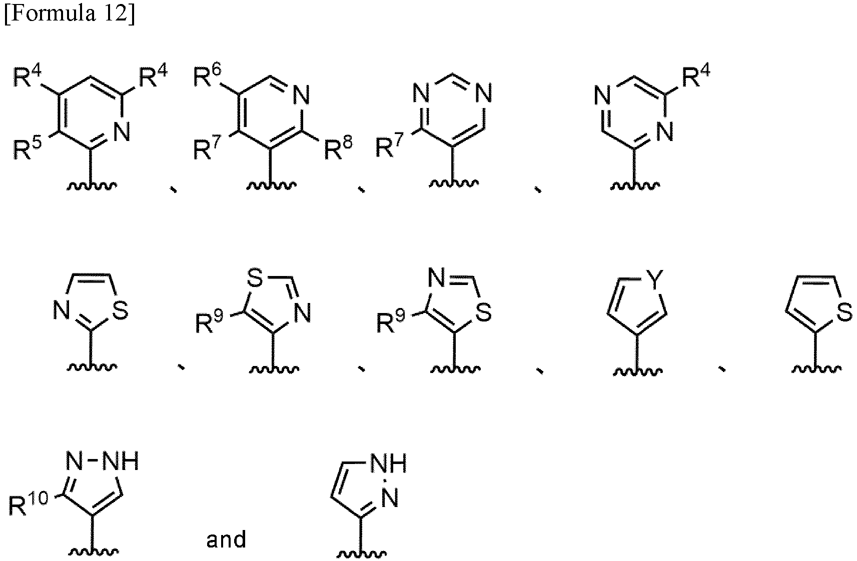
   [wherein Y represents an oxygen atom or a sulfur atom,
   R⁴ each independently represents a hydrogen atom or a C₁-C₃ alkoxy group,
   R⁵ represents a hydrogen atom, a fluorine atom, or a C₁-C₃ alkoxy group,
   R⁶ represents a hydrogen atom, a fluorine atom, or a C₁-C₃ alkoxy group,
   R⁷ represents a hydrogen atom, a C₁-C₃ alkyl group, a C₁-C₃ alkoxy group, or a cyclopropyl group,
   R⁸ represents a hydrogen atom or a C₁-C₃ alkoxy group,
   R⁹ represents a hydrogen atom or a C₁-C₃ alkyl group,
   R¹⁰ represents a hydrogen atom, a C₁-C₃ alkyl group, a C₁-C₃ alkoxy group, or a cyano group, and
   when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom,
   provided that when Z is: 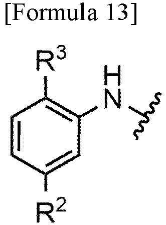 , R³ is neither thiophene bound at position 4 and substituted with R⁹ at position 3, pyridine bound at position 3 and substituted with a C₁-C₃ alkyl group at position 4, nor non-substituted pyrazole bound at position 4;
   when Z is: 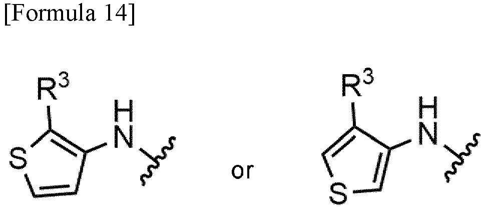 , R³ is neither thiophene bound at position 4 and substituted with R⁹ at position 3, pyridine bound at position 3 and substituted with a C₁-C₃ alkyl group at position 4, non-substituted pyrazole bound at position 4, non-substituted pyrimidine bound at position 5, nor pyrimidine bound at position 5 and substituted with a C₁-C₃ alkyl group at position 4;
   when Z is: 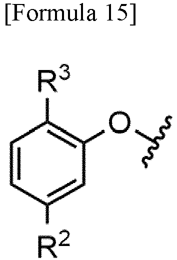 , and R² is a C₁-C₃ alkyl group, R³ is neither thiophene bound at position 4 and substituted with R⁹ at position 3, non-substituted pyrimidine bound at position 5, nor pyrimidine bound at position 5 and substituted with a C₁-C₃ alkyl group at position 4;
   when Z is the same as described above, and R² is a hydrogen atom, R³ is neither pyridine bound at position 3 and substituted with a C₁-C₃ alkyl group at position 4, nor non-substituted pyrazole bound at position 4;
   when Z is the same as described above, R¹ is a C₁-C₃ alkyl group and R² is a hydrogen atom, R³ is neither 4-cyclopropylpyridine bound at position 3, nor non-substituted pyrimidine bound at position 5; and
   when Z is: 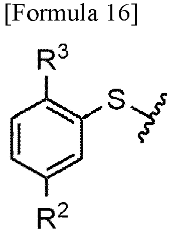 , R³ is neither thiophene bound at position 4 and substituted with R⁹ at position 3, pyridine bound at position 3 and substituted with a C₁-C₃ alkyl group at position 4, non-substituted pyrazole bound at position 4, non-substituted pyrimidine bound at position 5, nor pyrimidine bound at position 5 and substituted with a C₁-C₃ alkyl group at position 4].
(4) The compound or pharmacologically acceptable salt thereof according to (3), wherein R³ is selected from the group consisting of the following formulas: 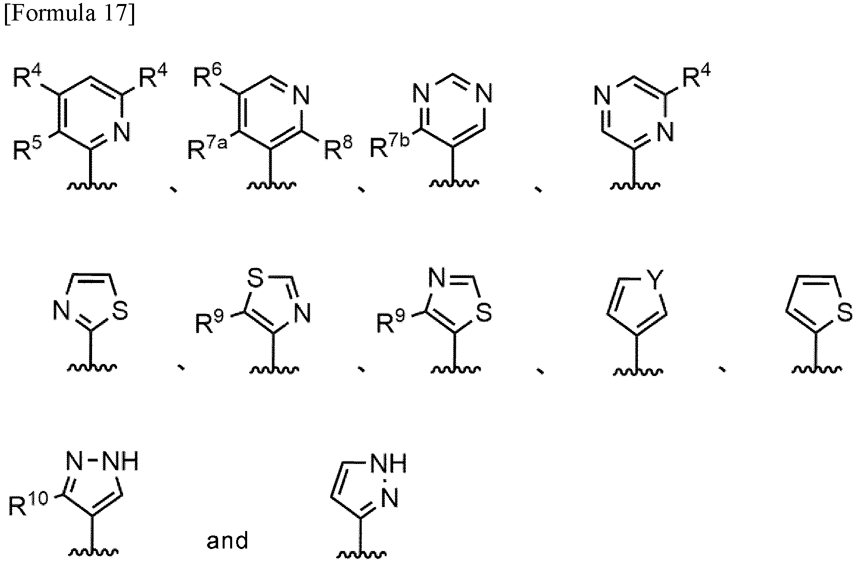
   [wherein Y represents an oxygen atom or a sulfur atom,
   R⁴ each independently represents a hydrogen atom or a C₁-C₃ alkoxy group,
   R⁵ represents a hydrogen atom, a fluorine atom, or a C₁-C₃ alkoxy group,
   R⁶ represents a hydrogen atom, a fluorine atom, or a C₁-C₃ alkoxy group,
   R^{7a} represents a hydrogen atom, a C₁-C₃ alkoxy group, or a cyclopropyl group,
   R^{7b} represents a hydrogen atom, a C₁-C₃ alkyl group, a C₁-C₃ alkoxy group, or a cyclopropyl group,
   R⁸ represents a hydrogen atom or a C₁-C₃ alkoxy group,
   R⁹ represents a hydrogen atom or a C₁-C₃ alkyl group,
   R¹⁰ represents a hydrogen atom, a C₁-C₃ alkyl group, a C₁-C₃ alkoxy group, or a cyano group, and
   when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom,
   provided that when Z is: 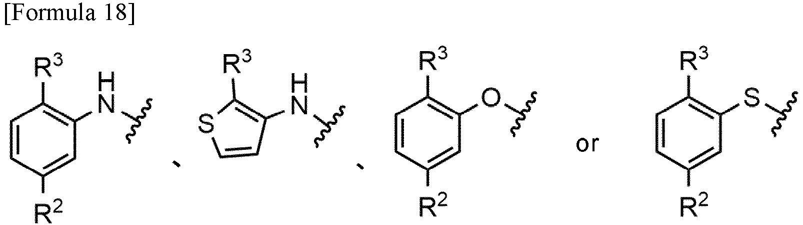 , R³ is neither pyridine bound at position 2 and substituted with a C₁-C₃ alkoxy group at position 3 nor pyridine bound at position 5 and substituted with a C₁-C₃ alkoxy group at position 3].
(5) The compound or pharmacologically acceptable salt thereof according to (4), wherein R³ is selected from the group consisting of the following formulas: 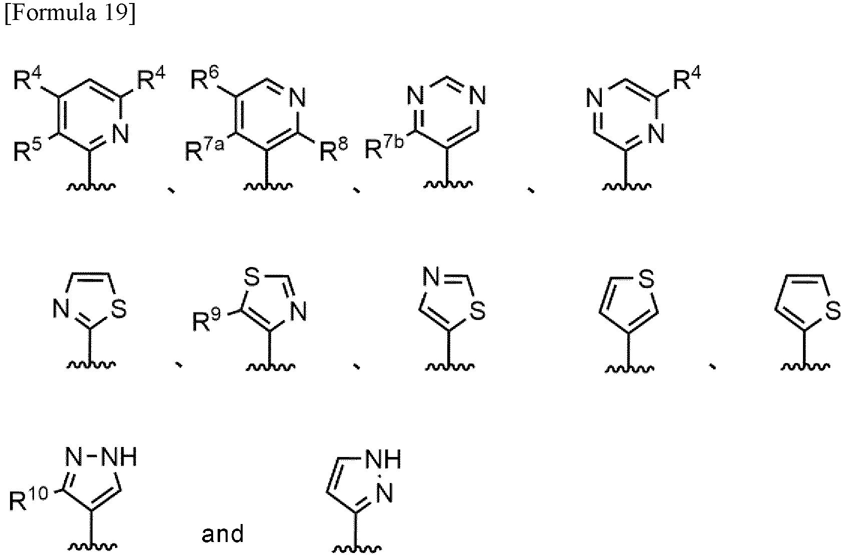
   [wherein R⁴ each independently represents a hydrogen atom or a methoxy group,
   R⁵ represents a hydrogen atom, a fluorine atom, or a methoxy group,
   R⁶ represents a hydrogen atom, a fluorine atom, or a methoxy group,
   R^{7a} represents a hydrogen atom, a methoxy group, or a cyclopropyl group,
   R^{7b} represents a hydrogen atom, a methyl group, or a methoxy group,
   R⁸ represents a hydrogen atom or a methoxy group,
   R⁹ represents a hydrogen atom or a methyl group,
   R¹⁰ represents a hydrogen atom, a methyl group, a methoxy group, or a cyano group, and
   when R⁶ is a methoxy group, R^{7a} and R⁸ are hydrogen atoms; when R⁶ is a fluorine atom, R⁸ is a hydrogen atom; and when R⁸ is a methoxy group, R⁶ is a hydrogen atom or a fluorine atom, and R^{7a} is a hydrogen atom,
   provided that when Z is: 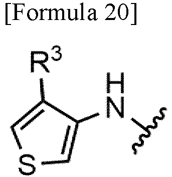 , R³ is not 3-methylpyrazole bound at position 4;
   when Z is: 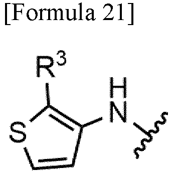 , R³ is neither non-substituted pyridine bound at position 3, 3-methylpyrazole bound at position 4, 2-methoxypyridine bound at position 3, 4-methoxypyridine bound at position 3, nor non-substituted thiophene bound at position 2;
   when Z is: 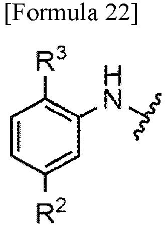 , R³ is neither non-substituted pyrimidine bound at position 5, 3-methylpyrazole bound at position 4, 2-methoxypyridine bound at position 3, 4-methoxypyridine bound at position 3, nor non-substituted thiophene bound at position 2;
   when Z is: 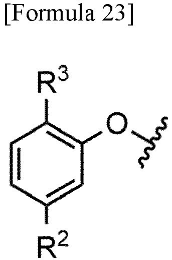 , and R² is a methyl group, R³ is neither non-substituted pyridine bound at position 3, 2-methoxypyridine bound at position 3, 4-methoxypyridine bound at position 3, nor non-substituted thiophene bound at position 2;
   when Z is the same as described above, and R² is a hydrogen atom, R³ is neither non-substituted pyridine bound at position 3, 3-methylpyrazole bound at position 4, nor non-substituted thiophene bound at position 2;
   when Z is the same as described above, and R² is a fluorine atom, R³ is neither non-substituted pyridine bound at position 3, 4-methylpyrimidine bound at position 5, 2-methoxypyridine bound at position 3, nor non-substituted thiophene bound at position 2; and
   when Z is: 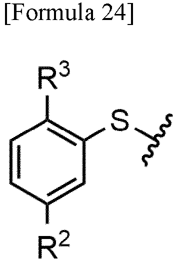 , R³ is neither non-substituted pyridine bound at position 3, 3-methylpyrazole bound at position 4, 2-methoxypyridine bound at position 3, 4-methoxypyridine bound at position 3, nor non-substituted thiophene bound at position 2].
(6) The compound or pharmacologically acceptable salt thereof according to (5), wherein R³ is selected from the group consisting of the following formulas: 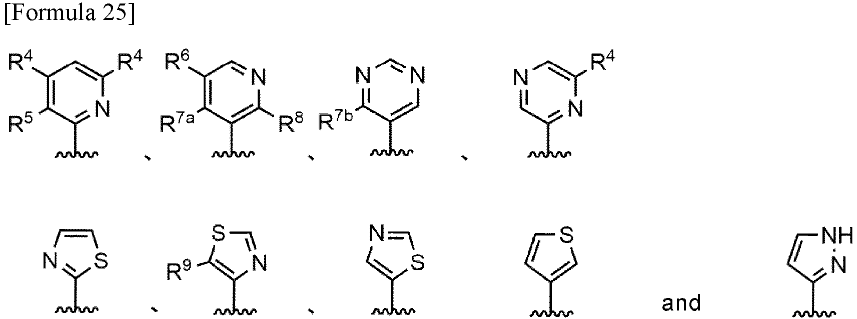
   [wherein R⁴ each independently represents a hydrogen atom or a methoxy group,
   R⁵ represents a hydrogen atom, a fluorine atom, or a methoxy group,
   R⁶ represents a hydrogen atom, a fluorine atom, or a methoxy group,
   R^{7a} represents a hydrogen atom, a methoxy group, or a cyclopropyl group,
   R^{7b} represents a methoxy group,
   R⁸ represents a hydrogen atom or a methoxy group,
   R⁹ represents a hydrogen atom or a methyl group,
   when R⁶ is a methoxy group, R^{7a} and R⁸ are hydrogen atoms; when R⁶ is a fluorine atom, R⁸ is a hydrogen atom; and when R^{7a} is a methoxy group, R⁶ is a fluorine atom, and R⁸ is a hydrogen atom,
   provided that when Z is: 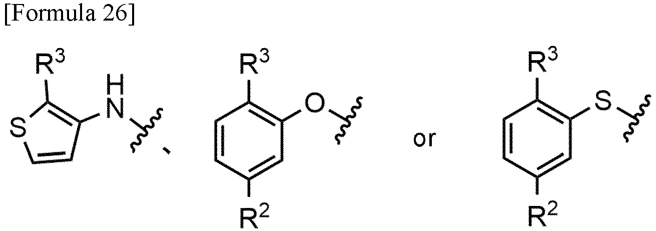 , R³ is not non-substituted pyridine bound at position 3].
(7) The compound or pharmacologically acceptable salt thereof according to (2) to (6), wherein in the above general formula (I), Z is selected from the group consisting of the following formulas. 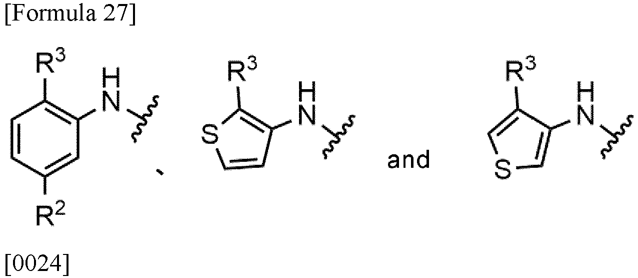
(8) The compound or pharmacologically acceptable salt thereof according to (2) to (6), wherein in the above general formula (I), Z is selected from the group consisting of the following formulas. 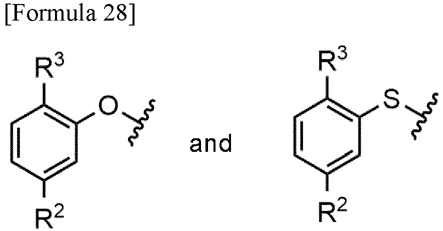
(9) The compound or pharmacologically acceptable salt thereof according to (7), wherein in the above general formula (I), Z is the following formula: 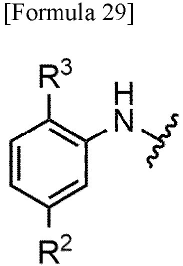
   [wherein R² represents a hydrogen atom, a methyl group, or a fluorine atom, and
   R³ is selected from the group consisting of the following formulas: 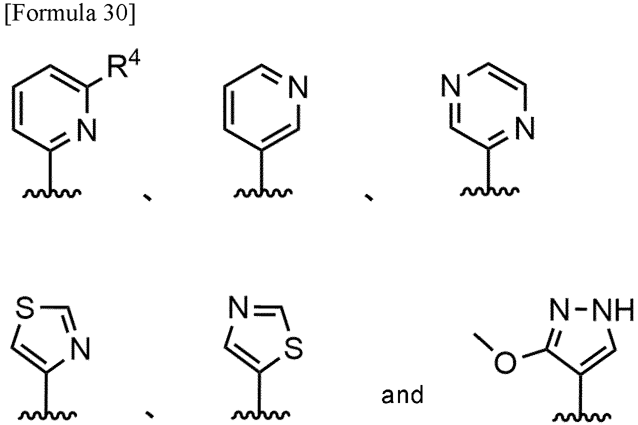
   wherein R⁴ represents a hydrogen atom or a methoxy group].
(10) The compound or pharmacologically acceptable salt thereof according to (8), wherein in the above general formula (I), Z is the following formula: 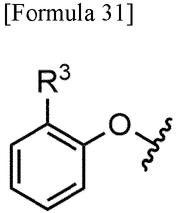
   [wherein R³ is selected from the group consisting of the following formulas: 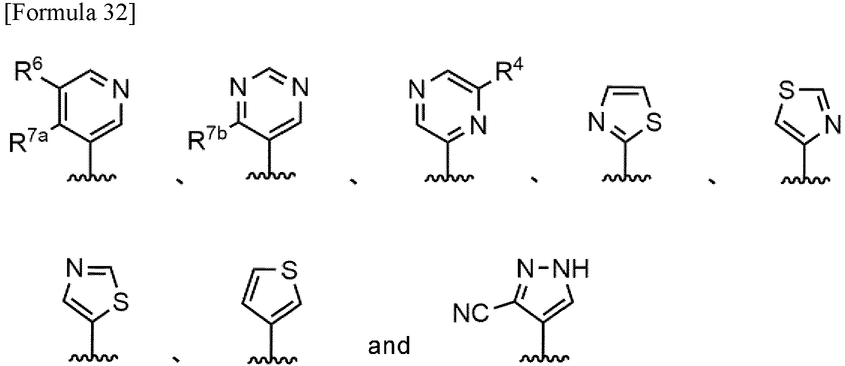
   [wherein R⁴ represents a hydrogen atom or a methoxy group,
   R⁶ represents a hydrogen atom or a fluorine atom,
   R^{7a} represents a hydrogen atom, a methyl group, a methoxy group, or a cyclopropyl group,
   R^{7b} represents a hydrogen atom, a methyl group, a methoxy group,
   when R⁶ is a hydrogen atom, R^{7a} represents a methoxy group or a cyclopropyl group; when R^{7a} is a methyl group, R⁶ is a fluorine atom; and when R^{7a} is a cyclopropyl group, R⁶ is a hydrogen atom].
(11) The compound or pharmacologically acceptable salt thereof according to (2), selected from the group consisting of the following compounds:
   N-((1H-imidazol-4-yl)methyl)-[2,3'-bithiophen]-4'-amine;
   N-((1H-imidazol-4-yl)methyl)-[2,2'-bithiophen]-3-amine;
   N-((1H-imidazol-4-yl)methyl)-4-(pyridin-3-yl)thiophen-3-amine;
   N-((1H-imidazol-4-yl)methyl)-4-(4-methoxypyridin-3-yl)thiophen-3-amine;
   N-((1H-imidazol-4-yl)methyl)-4-(5-methoxypyridin-3-yl)thiophen-3-amine;
   N-((1H-imidazol-4-yl)methyl)-4-(5-fluoropyridin-3-yl)thiophen-3-amine;
   N-((1H-imidazol-4-yl)methyl)-2-(pyridin-3-yl)thiophen-3-amine;
   N-((1H-imidazol-4-yl)methyl)-4-(1H-pyrazol-5-yl)thiophen-3-amine;
   N-((1H-imidazol-4-yl)methyl)-2-(pyridin-3-yl)aniline;
   N-((1H-imidazol-4-yl)methyl)-2-(pyrimidin-5-yl)aniline;
   N-((1H-imidazol-4-yl)methyl)-2-(thiazol-5-yl)aniline;
   N-((1H-imidazol-4-yl)methyl)-4-(thiazol-2-yl)thiophen-3-amine;
   N-((1H-imidazol-4-yl)methyl)-4-(pyrazin-2-yl)thiophen-3-amine;
   N-((1H-imidazol-4-yl)methyl)-4-(thiazol-4-yl)thiophen-3-amine;
   N-((1H-imidazol-4-yl)methyl)-2-(thiazol-4-yl)aniline;
   N-((1H-imidazol-4-yl)methyl)-4-(3-methylpyridin-2-yl)thiophen-3-amine;
   N-((1H-imidazol-4-yl)methyl)-4-(3-methoxypyridin-2-yl)thiophen-3-amine;
   N-((1H-imidazol-4-yl)methyl)-4-(4-methylthiazol-5-yl)thiophen-3-amine;
   N-((1H-imidazol-4-yl)methyl)-2-(4-methylpyrimidin-5-yl)aniline;
   N-((1H-imidazol-4-yl)methyl)-5-methyl-2-(4-methylpyrimidin-5-yl)aniline;
   N-((1H-imidazol-4-yl)methyl)-4-(4-methoxypyridin-2-yl)thiophen-3-amine;
   N-((1H-imidazol-4-yl)methyl)-4-(6-methoxypyridin-2-yl)thiophen-3-amine;
   N-((1H-imidazol-4-yl)methyl)-4-(4-methoxypyrimidin-5-yl)thiophen-3-amine;
   N-((1H-imidazol-4-yl)methyl)-4-(5-methylthiazol-4-yl)thiophen-3-amine;
   N-((1H-imidazol-4-yl)methyl)-5-fluoro-2-(4-methylpyrimidin-5-yl)aniline;
   N-((1H-imidazol-4-yl)methyl)-2-(pyrazin-2-yl)aniline;
   N-((1H-imidazol-4-yl)methyl)-2-(pyridin-2-yl)aniline;
   N-((1H-imidazol-4-yl)methyl)-2-(3-methoxypyridin-2-yl)aniline;
   N-((1H-imidazol-4-yl)methyl)-4-(3-fluoropyridin-2-yl)thiophen-3-amine;
   N-((1H-imidazol-4-yl)methyl)-4-(pyridin-2-yl)thiophen-3-amine;
   N-((1H-imidazol-4-yl)methyl)-4-(6-methoxypyrazin-2-yl)thiophen-3-amine;
   N-((1H-imidazol-4-yl)methyl)-2-(6-methoxypyridin-2-yl)aniline;
   N-((1H-imidazol-4-yl)methyl)-2-(3-methoxy-1H-pyrazol-4-yl)-5-methylaniline;
   N-((1H-imidazol-4-yl)methyl)-5-fluoro-2-(3-methoxy-1H-pyrazol-4-yl)aniline;
   (+)-N-(1-(1H-imidazol-4-yl)ethyl)-2-(pyridin-3-yl)aniline;
   (+)-N-(1-(1H-imidazol-4-yl)ethyl)-2-(6-methoxypyridin-2-yl)aniline;
   (+)-N-(1-(1H-imidazol-4-yl)ethyl)-2-(thiazol-4-yl)aniline;
   N-((1H-imidazol-4-yl)methyl)-4-(furan-3-yl)thiophen-3-amine;
   N-((1H-imidazol-4-yl)methyl)-4-(pyrimidin-5-yl)thiophen-3-amine;
   N-((1H-imidazol-4-yl)methyl)-4-(2-methoxypyridin-3-yl)thiophen-3-amine;
   4-((2-(thiophen-3-yl)phenoxy)methyl)-1H-imidazole;
   4-((2-(furan-2-yl)phenoxy)methyl)-1H-imidazole;
   4-((2-(thiophen-2-yl)phenoxy)methyl)-1H-imidazole;
   4-(2-((1H-imidazol-4-yl)methoxy)phenyl)-1H-pyrazole;
   3-(2-((1H-imidazol-4-yl)methoxy)phenyl)pyridine;
   4-(2-((1H-imidazol-4-yl)methoxy)phenyl)-3-methyl-1H-pyrazole;
   4-(2-((IH-imidazol-4-yl)methoxy)phenyl)- IH-pyrazole-3 -carbonitrile;
   3-(2-((1H-imidazol-4-yl)methoxy)phenyl)-5-fluoropyridine;
   3-(2-((1H-imidazol-4-yl)methoxy)phenyl)-2-methoxypyridine;
   3-(2-((1H-imidazol-4-yl)methoxy)phenyl)-5-methoxypyridine;
   3-(2-((1H-imidazol-4-yl)methoxy)phenyl)-4-methoxypyridine;
   4-(2-((1H-imidazol-4-yl)methoxy)-4-methylphenyl)-1H-pyrazole;
   4-(2-((1H-imidazol-4-yl)methoxy)-4-methylphenyl)-1H-pyrazole-3-carbonitrile;
   3-(2-((1H-imidazol-4-yl)methoxy)-4-methylphenyl)-4-methylpyridine;
   4-(2-((1H-imidazol-4-yl)methoxy)-4-fluorophenyl)-3-methyl-1H-pyrazole;
   4-(2-((1H-imidazol-4-yl)methoxy)-4-methylphenyl)-3-methyl-1H-pyrazole;
   3-(2-((1H-imidazol-4-yl)methoxy)phenyl)-5-fluoro-4-methylpyridine;
   3-(2-((1H-imidazol-4-yl)methoxy)phenyl)-5-fluoro-2-methoxypyridine;
   3-(2-((1H-imidazol-4-yl)methoxy)-4-methylphenyl)-4-methoxypyridine;
   3-(2-((1H-imidazol-4-yl)methoxy)-4-fluorophenyl)-4-methoxypyridine;
   5-(2-((1H-imidazol-4-yl)methoxy)-4-fluorophenyl)-4-methylpyrimidine;
   5-(2-((1H-imidazol-4-yl)methoxy)-4-methylphenyl)-4-methylpyrimidine;
   2-(2-((1H-imidazol-4-yl)methoxy)-4-methylphenyl)pyrazine;
   4-(2-((1H-imidazol-4-yl)methoxy)phenyl)thiazole;
   2-(2-((1H-imidazol-4-yl)methoxy)phenyl)pyrazine;
   5-(2-((1H-imidazol-4-yl)methoxy)phenyl)pyrimidine;
   2-(2-((1H-imidazol-4-yl)methoxy)phenyl)thiazole;
   5-(2-((1H-imidazol-4-yl)methoxy)phenyl)thiazole;
   3-(2-((1H-imidazol-4-yl)methoxy)phenyl)-4-methylpyridine;
   3-(2-((1H-imidazol-4-yl)methoxy)phenyl)-4-cyclopropylpyridine;
   3-(2-((1H-imidazol-4-yl)methoxy)phenyl)-5-fluoro-4-methoxypyridine;
   5-(2-((1H-imidazol-4-yl)methoxy)phenyl)-4-methoxypyrimidine;
   5-(2-((1H-imidazol-4-yl)methoxy)phenyl)-4-methylpyrimidine;
   2-(2-((1H-imidazol-4-yl)methoxy)phenyl)-6-methoxypyrazine;
   (+)-4-(1-(2-(thiophen-2-yl)phenoxy)ethyl)-1H-imidazole;
   3-(2-(((1H-imidazol-4-yl)methyl)thio)phenyl)pyridine;
   4-(2-(((1H-imidazol-4-yl)methyl)thio)phenyl)-1H-pyrazole; and
   4-(((2-(thiophen-3-yl)phenyl)thio)methyl)-1H-imidazole.
(12) The compound or pharmacologically acceptable salt thereof according to (4), selected from the group consisting of the following compounds:
   N-((1H-imidazol-4-yl)methyl)-[2,3'-bithiophen]-4'-amine;
   N-((1H-imidazol-4-yl)methyl)-[2,2'-bithiophen]-3-amine;
   N-((1H-imidazol-4-yl)methyl)-4-(pyridin-3-yl)thiophen-3-amine;
   N-((1H-imidazol-4-yl)methyl)-4-(4-methoxypyridin-3-yl)thiophen-3-amine;
   N-((1H-imidazol-4-yl)methyl)-4-(5-methoxypyridin-3-yl)thiophen-3-amine;
   N-((1H-imidazol-4-yl)methyl)-4-(5-fluoropyridin-3-yl)thiophen-3-amine;
   N-((1H-imidazol-4-yl)methyl)-2-(pyridin-3-yl)thiophen-3-amine;
   N-((1H-imidazol-4-yl)methyl)-4-(1H-pyrazol-5-yl)thiophen-3-amine;
   N-((1H-imidazol-4-yl)methyl)-2-(pyridin-3-yl)aniline;
   N-((1H-imidazol-4-yl)methyl)-2-(pyrimidin-5-yl)aniline;
   N-((1H-imidazol-4-yl)methyl)-2-(thiazol-5-yl)aniline;
   N-((1H-imidazol-4-yl)methyl)-4-(thiazol-2-yl)thiophen-3-amine;
   N-((1H-imidazol-4-yl)methyl)-4-(pyrazin-2-yl)thiophen-3-amine;
   N-((1H-imidazol-4-yl)methyl)-4-(thiazol-4-yl)thiophen-3-amine;
   N-((1H-imidazol-4-yl)methyl)-2-(thiazol-4-yl)aniline;
   N-((1H-imidazol-4-yl)methyl)-4-(3-methoxypyridin-2-yl)thiophen-3-amine;
   N-((1H-imidazol-4-yl)methyl)-4-(4-methylthiazol-5-yl)thiophen-3-amine;
   N-((1H-imidazol-4-yl)methyl)-2-(4-methylpyrimidin-5-yl)aniline;
   N-((1H-imidazol-4-yl)methyl)-5-methyl-2-(4-methylpyrimidin-5-yl)aniline;
   N-((1H-imidazol-4-yl)methyl)-4-(4-methoxypyridin-2-yl)thiophen-3-amine;
   N-((1H-imidazol-4-yl)methyl)-4-(6-methoxypyridin-2-yl)thiophen-3-amine;
   N-((1H-imidazol-4-yl)methyl)-4-(4-methoxypyrimidin-5-yl)thiophen-3-amine;
   N-((1H-imidazol-4-yl)methyl)-4-(5-methylthiazol-4-yl)thiophen-3-amine;
   N-((1H-imidazol-4-yl)methyl)-5-fluoro-2-(4-methylpyrimidin-5-yl)aniline;
   N-((1H-imidazol-4-yl)methyl)-2-(pyrazin-2-yl)aniline;
   N-((1H-imidazol-4-yl)methyl)-2-(pyridin-2-yl)aniline;
   N-((1H-imidazol-4-yl)methyl)-4-(3-fluoropyridin-2-yl)thiophen-3-amine;
   N-((1H-imidazol-4-yl)methyl)-4-(pyridin-2-yl)thiophen-3-amine;
   N-((1H-imidazol-4-yl)methyl)-4-(6-methoxypyrazin-2-yl)thiophen-3-amine;
   N-((1H-imidazol-4-yl)methyl)-2-(6-methoxypyridin-2-yl)aniline;
   N-((1H-imidazol-4-yl)methyl)-2-(3-methoxy-1H-pyrazol-4-yl)-5-methylaniline;
   N-((1H-imidazol-4-yl)methyl)-5-fluoro-2-(3-methoxy-1H-pyrazol-4-yl)aniline;
   (+)-N-(1-(1H-imidazol-4-yl)ethyl)-2-(pyridin-3-yl)aniline;
   (+)-N-(1-(1H-imidazol-4-yl)ethyl)-2-(6-methoxypyridin-2-yl)aniline;
   (+)-N-(1-(1H-imidazol-4-yl)ethyl)-2-(thiazol-4-yl)aniline;
   N-((1H-imidazol-4-yl)methyl)-4-(furan-3-yl)thiophen-3-amine;
   N-((1H-imidazol-4-yl)methyl)-4-(2-methoxypyridin-3-yl)thiophen-3-amine;
   4-((2-(thiophen-3-yl)phenoxy)methyl)-1H-imidazole;
   4-((2-(thiophen-2-yl)phenoxy)methyl)-1H-imidazole;
   3-(2-((1H-imidazol-4-yl)methoxy)phenyl)pyridine;
   4-(2-((1H-imidazol-4-yl)methoxy)phenyl)-3-methyl-1H-pyrazole;
   4-(2-((IH-imidazol-4-yl)methoxy)phenyl)- IH-pyrazole-3 -carbonitrile;
   3-(2-((1H-imidazol-4-yl)methoxy)phenyl)-5-fluoropyridine;
   3-(2-((1H-imidazol-4-yl)methoxy)phenyl)-2-methoxypyridine;
   3-(2-((1H-imidazol-4-yl)methoxy)phenyl)-4-methoxypyridine;
   4-(2-((1H-imidazol-4-yl)methoxy)-4-methylphenyl)-1H-pyrazole;
   4-(2-((1H-imidazol-4-yl)methoxy)-4-methylphenyl)-1H-pyrazole-3-carbonitrile;
   4-(2-((1H-imidazol-4-yl)methoxy)-4-fluorophenyl)-3-methyl-1H-pyrazole;
   4-(2-((1H-imidazol-4-yl)methoxy)-4-methylphenyl)-3-methyl-1H-pyrazole;
   3-(2-((1H-imidazol-4-yl)methoxy)phenyl)-5-fluoro-4-methylpyridine;
   3-(2-((1H-imidazol-4-yl)methoxy)phenyl)-5-fluoro-2-methoxypyridine;
   3-(2-((1H-imidazol-4-yl)methoxy)-4-methylphenyl)-4-methoxypyridine;
   3-(2-((1H-imidazol-4-yl)methoxy)-4-fluorophenyl)-4-methoxypyridine;
   5-(2-((1H-imidazol-4-yl)methoxy)-4-fluorophenyl)-4-methylpyrimidine;
   2-(2-((1H-imidazol-4-yl)methoxy)-4-methylphenyl)pyrazine;
   4-(2-((1H-imidazol-4-yl)methoxy)phenyl)thiazole;
   2-(2-((1H-imidazol-4-yl)methoxy)phenyl)pyrazine;
   5-(2-((1H-imidazol-4-yl)methoxy)phenyl)pyrimidine;
   2-(2-((1H-imidazol-4-yl)methoxy)phenyl)thiazole;
   5-(2-((1H-imidazol-4-yl)methoxy)phenyl)thiazole;
   3-(2-((1H-imidazol-4-yl)methoxy)phenyl)-4-cyclopropylpyridine;
   3-(2-((1H-imidazol-4-yl)methoxy)phenyl)-5-fluoro-4-methoxypyridine;
   5-(2-((1H-imidazol-4-yl)methoxy)phenyl)-4-methoxypyrimidine;
   5-(2-((1H-imidazol-4-yl)methoxy)phenyl)-4-methylpyrimidine;
   2-(2-((1H-imidazol-4-yl)methoxy)phenyl)-6-methoxypyrazine;
   (+)-4-(1-(2-(thiophen-2-yl)phenoxy)ethyl)-1H-imidazole; and
   3-(2-(((1H-imidazol-4-yl)methyl)thio)phenyl)pyridine.
(13) The compound or pharmacologically acceptable salt thereof according to (5), selected from the group consisting of the following compounds:
   N-((1H-imidazol-4-yl)methyl)-[2,3'-bithiophen]-4'-amine;
   N-((1H-imidazol-4-yl)methyl)-[2,2'-bithiophen]-3-amine;
   N-((1H-imidazol-4-yl)methyl)-4-(pyridin-3-yl)thiophen-3-amine;
   N-((1H-imidazol-4-yl)methyl)-4-(4-methoxypyridin-3-yl)thiophen-3-amine;
   N-((1H-imidazol-4-yl)methyl)-4-(5-methoxypyridin-3-yl)thiophen-3-amine;
   N-((1H-imidazol-4-yl)methyl)-4-(5-fluoropyridin-3-yl)thiophen-3-amine;
   N-((1H-imidazol-4-yl)methyl)-4-(1H-pyrazol-5-yl)thiophen-3-amine;
   N-((1H-imidazol-4-yl)methyl)-2-(pyridin-3-yl)aniline;
   N-((1H-imidazol-4-yl)methyl)-2-(thiazol-5-yl)aniline;
   N-((1H-imidazol-4-yl)methyl)-4-(thiazol-2-yl)thiophen-3-amine;
   N-((1H-imidazol-4-yl)methyl)-4-(pyrazin-2-yl)thiophen-3-amine;
   N-((1H-imidazol-4-yl)methyl)-4-(thiazol-4-yl)thiophen-3-amine;
   N-((1H-imidazol-4-yl)methyl)-2-(thiazol-4-yl)aniline;
   N-((1H-imidazol-4-yl)methyl)-4-(3-methoxypyridin-2-yl)thiophen-3-amine;
   N-((1H-imidazol-4-yl)methyl)-5-methyl-2-(4-methylpyrimidin-5-yl)aniline;
   N-((1H-imidazol-4-yl)methyl)-4-(4-methoxypyridin-2-yl)thiophen-3-amine;
   N-((1H-imidazol-4-yl)methyl)-4-(6-methoxypyridin-2-yl)thiophen-3-amine;
   N-((1H-imidazol-4-yl)methyl)-4-(4-methoxypyrimidin-5-yl)thiophen-3-amine;
   N-((1H-imidazol-4-yl)methyl)-4-(5-methylthiazol-4-yl)thiophen-3-amine;
   N-((1H-imidazol-4-yl)methyl)-5-fluoro-2-(4-methylpyrimidin-5-yl)aniline;
   N-((1H-imidazol-4-yl)methyl)-2-(pyrazin-2-yl)aniline;
   N-((1H-imidazol-4-yl)methyl)-2-(pyridin-2-yl)aniline;
   N-((1H-imidazol-4-yl)methyl)-4-(3-fluoropyridin-2-yl)thiophen-3-amine;
   N-((1H-imidazol-4-yl)methyl)-4-(pyridin-2-yl)thiophen-3-amine;
   N-((1H-imidazol-4-yl)methyl)-4-(6-methoxypyrazin-2-yl)thiophen-3-amine;
   N-((1H-imidazol-4-yl)methyl)-2-(6-methoxypyridin-2-yl)aniline;
   N-((1H-imidazol-4-yl)methyl)-2-(3-methoxy-1H-pyrazol-4-yl)-5-methylaniline;
   N-((1H-imidazol-4-yl)methyl)-5-fluoro-2-(3-methoxy-1H-pyrazol-4-yl)aniline;
   (+)-N-(1-(1H-imidazol-4-yl)ethyl)-2-(pyridin-3-yl)aniline;
   (+)-N-(1-(1H-imidazol-4-yl)ethyl)-2-(6-methoxypyridin-2-yl)aniline;
   (+)-N-(1-(1H-imidazol-4-yl)ethyl)-2-(thiazol-4-yl)aniline;
   N-((1H-imidazol-4-yl)methyl)-4-(2-methoxypyridin-3-yl)thiophen-3-amine;
   4-((2-(thiophen-3-yl)phenoxy)methyl)-1H-imidazole;
   4-(2-((IH-imidazol-4-yl)methoxy)phenyl)- IH-pyrazole-3 -carbonitrile;
   3-(2-((1H-imidazol-4-yl)methoxy)phenyl)-5-fluoropyridine;
   3-(2-((1H-imidazol-4-yl)methoxy)phenyl)-4-methoxypyridine;
   4-(2-((1H-imidazol-4-yl)methoxy)-4-methylphenyl)-1H-pyrazole;
   4-(2-((1H-imidazol-4-yl)methoxy)-4-methylphenyl)-1H-pyrazole-3-carbonitrile;
   4-(2-((1H-imidazol-4-yl)methoxy)-4-fluorophenyl)-3-methyl-1H-pyrazole;
   4-(2-((1H-imidazol-4-yl)methoxy)-4-methylphenyl)-3-methyl-1H-pyrazole;
   3-(2-((1H-imidazol-4-yl)methoxy)phenyl)-5-fluoro-4-methylpyridine;
   3-(2-((1H-imidazol-4-yl)methoxy)-4-fluorophenyl)-4-methoxypyridine;
   2-(2-((1H-imidazol-4-yl)methoxy)-4-methylphenyl)pyrazine;
   4-(2-((1H-imidazol-4-yl)methoxy)phenyl)thiazole;
   2-(2-((1H-imidazol-4-yl)methoxy)phenyl)pyrazine;
   5-(2-((1H-imidazol-4-yl)methoxy)phenyl)pyrimidine;
   2-(2-((1H-imidazol-4-yl)methoxy)phenyl)thiazole;
   5-(2-((1H-imidazol-4-yl)methoxy)phenyl)thiazole;
   3-(2-((1H-imidazol-4-yl)methoxy)phenyl)-4-cyclopropylpyridine;
   3-(2-((1H-imidazol-4-yl)methoxy)phenyl)-5-fluoro-4-methoxypyridine;
   5-(2-((1H-imidazol-4-yl)methoxy)phenyl)-4-methoxypyrimidine;
   5-(2-((1H-imidazol-4-yl)methoxy)phenyl)-4-methylpyrimidine; and
   2-(2-((1H-imidazol-4-yl)methoxy)phenyl)-6-methoxypyrazine.
(14) A pharmaceutical composition comprising the compound or pharmacologically acceptable salt thereof according to any of (1) to (13) as an active ingredient.
(15) The pharmaceutical composition according to (14), for preventing or treating a disease selected from the group consisting of orthostatic hypotension, essential hypotension, acute hypotension associated with various diseases or conditions, and urinary incontinence.
(16) The compound or pharmacologically acceptable salt thereof according to any of (1) to (13), for use in preventing or treating a disease selected from the group consisting of orthostatic hypotension, essential hypotension, acute hypotension associated with various diseases or conditions, and urinary incontinence.
(17) Use of the compound or pharmacologically acceptable salt thereof according to any of (1) to (13), in manufacturing a medicament for preventing or treating a disease selected from the group consisting of orthostatic hypotension, essential hypotension, acute hypotension associated with various diseases or conditions, and urinary incontinence.
(18) A method for preventing or treating a disease selected from the group consisting of orthostatic hypotension, essential hypotension, acute hypotension associated with various diseases or conditions, and urinary incontinence, comprising administering a therapeutically effective amount of the compound or pharmacologically acceptable salt thereof according to any of (1) to (13) to a subject in need thereof.

### [Advantageous Effects of Invention]

The present compound has an excellent adrenergic α1A receptor agonistic action, and is useful as a preventive or therapeutic drug for orthostatic hypotension, essential hypotension, acute hypotension associated with various diseases or conditions, and urinary incontinence.

### [Description of Embodiments]

In the specification of the present application, each term is defined as below.

The term "C₁-C₃ alkyl group" represents a straight or branched alkyl group consisting of 1 to 3 carbon atoms, and examples thereof include a methyl group, an ethyl group, an n-propyl group, and an i-propyl group.

The term "C₁-C₃ alkoxy group" specifically represents -O- (C₁-C₃ alkyl) group, and examples thereof include a methoxy group, an ethoxy group, an n-propoxy group, and an i-propoxy group.

The term "C₁-C₆ alkyl group" represents a straight alkyl group consisting of 1 to 6 carbon atoms.

Hereinafter, the present compound will be described.

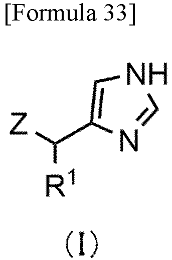

The present compound is a compound represented by the general formula (I) in which R¹ and Z are defined as below, or a pharmacologically acceptable salt thereof.

R⁵ represents a hydrogen atom or a C₁-C₃ alkyl group.

Z is selected from the group consisting of the following formulas: 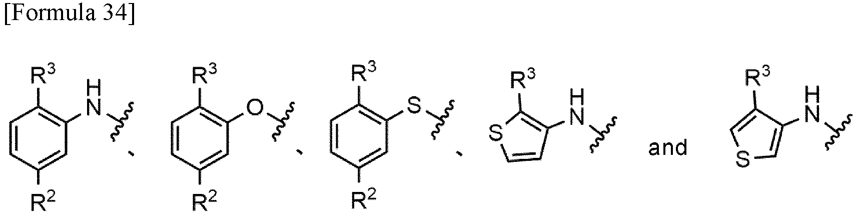
[wherein R² represents a hydrogen atom, a fluorine atom, or a C₁-C₃ alkyl group,
R³ is selected from the group consisting of the following formulas: 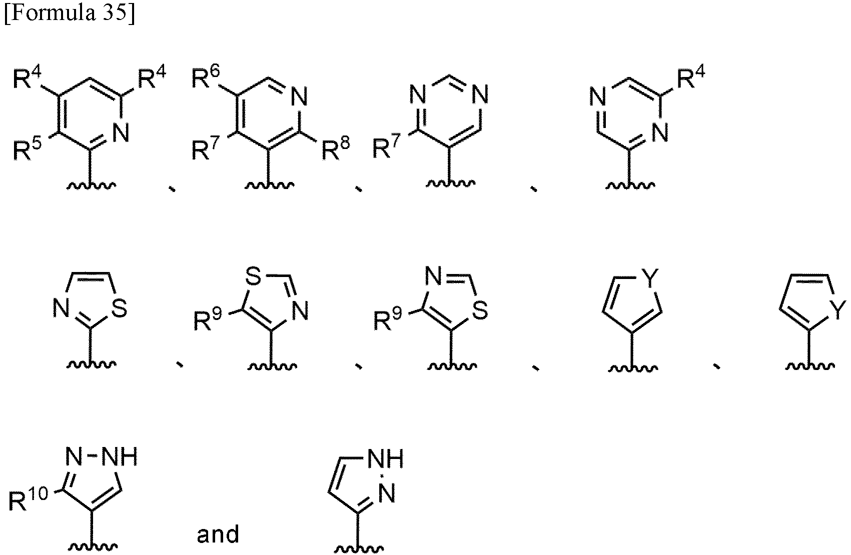
wherein Y represents an oxygen atom or a sulfur atom,
R⁴ each independently represents a hydrogen atom or a C₁-C₃ alkoxy group,
R⁵ represents a hydrogen atom, a fluorine atom, a C₁-C₃ alkyl group, or a C₁-C₃ alkoxy group,
R⁶ represents a hydrogen atom, a fluorine atom, or a C₁-C₃ alkoxy group,
R⁷ represents a hydrogen atom, a C₁-C₃ alkyl group, a C₁-C₃ alkoxy group, or a cyclopropyl group,
R⁸ represents a hydrogen atom or a C₁-C₃ alkoxy group,
R⁹ represents a hydrogen atom or a C₁-C₃ alkyl group,
R¹⁰ represents a hydrogen atom, a C₁-C₃ alkyl group, a C₁-C₃ alkoxy group, or a cyano group, and
when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom].

Preferably, when Z is: 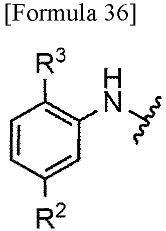 , and R² is a hydrogen atom, R³ is not 3-fluoropyridine bound at position 2;
when Z is: 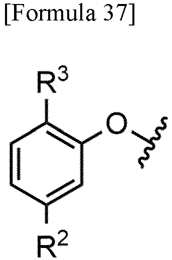 , and R² is a hydrogen atom, R³ is neither non-substituted pyridine bound at position 2, non-substituted pyrazole bound at position 3, nor 3-methoxypyrazole bound at position 4;
when Z is: 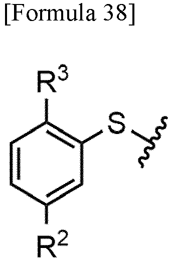 , and R² is a hydrogen atom, R³ is not non-substituted furan bound at position 2;
when Z is: 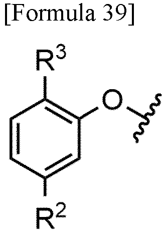 , R⁵ is a C₁-C₃ alkyl group, and R² is a hydrogen atom, R³ is not non-substituted pyrazole bound at position 4; and
when Z is: 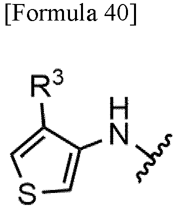 , R³ is not non-substituted thiazole bound at position 5.

In the present invention, a group selected from the group consisting of the following formulas is preferable among the above R³: 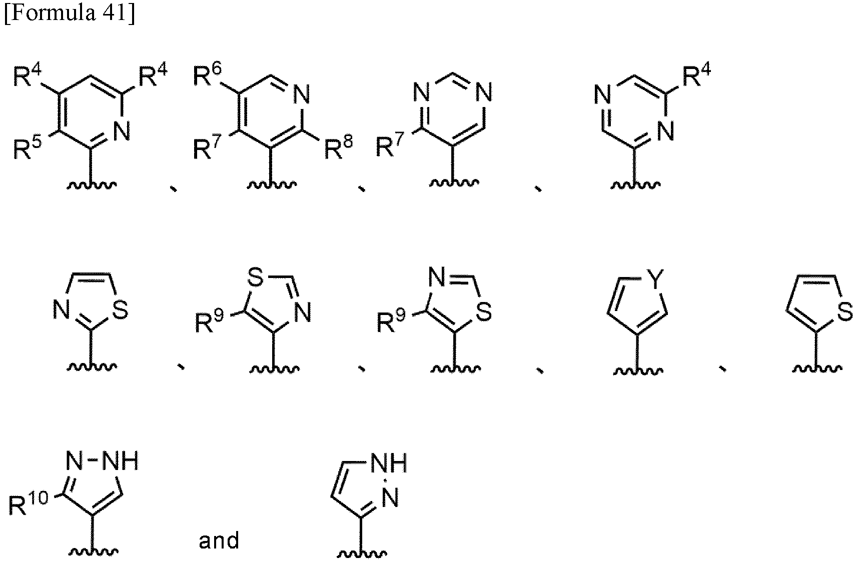
[wherein Y represents an oxygen atom or a sulfur atom,
R⁴ each independently represents a hydrogen atom or a C₁-C₃ alkoxy group,
R⁵ represents a hydrogen atom, a fluorine atom, or a C₁-C₃ alkoxy group,
R⁶ represents a hydrogen atom, a fluorine atom, or a C₁-C₃ alkoxy group,
R⁷ represents a hydrogen atom, a C₁-C₃ alkyl group, a C₁-C₃ alkoxy group, or a cyclopropyl group,
R⁸ represents a hydrogen atom or a C₁-C₃ alkoxy group,
R⁹ represents a hydrogen atom or a C₁-C₃ alkyl group,
R¹⁰ represents a hydrogen atom, a C₁-C₃ alkyl group, a C₁-C₃ alkoxy group, or a cyano group, and
when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom.
provided that when Z is: 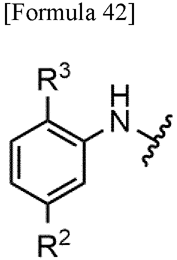 , R³ is neither thiophene bound at position 4 and substituted with R⁹ at position 3, pyridine bound at position 3 and substituted with C₁-C₃ alkyl group at position 4, nor non-substituted pyrazole bound at position 4;
when Z is: 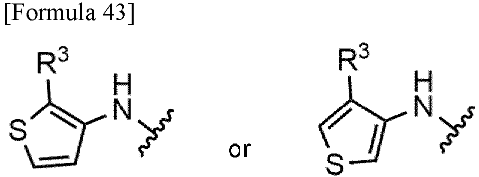 , R³ is neither thiophene bound at position 4 and substituted with R⁹ at position 3, pyridine bound at position 3 and substituted with C₁-C₃ alkyl group at position 4, non-substituted pyrazole bound at position 4, non-substituted pyrimidine bound at position 5, nor pyrimidine bound at position 5 and substituted with C₁-C₃ alkyl group at position 4;
when Z is: 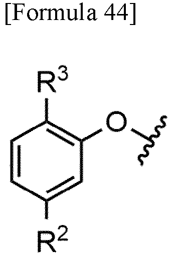 , and R² is a C₁-C₃ alkyl group, R³ is neither thiophene bound at position 4 and substituted with R⁹ at position 3, non-substituted pyrimidine bound at position 5, nor pyrimidine bound at position 5 and substituted with C₁-C₃ alkyl group at position 4;
when Z is the same as described above, and R² is a hydrogen atom, R³ is neither pyridine bound at position 3 and substituted with C₁-C₃ alkyl group at position 4 nor non-substituted pyrazole bound at position 4;
when Z is the same as described above, R¹ is a C₁-C₃ alkyl group, and R² is a hydrogen atom, R³ is neither 4-cyclopropylpyridine bound at position 3, nor non-substituted pyrimidine bound at position 5; and
when Z is: 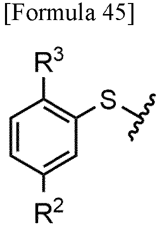 , R³ is neither thiophene bound at position 4 and substituted with R⁹ at position 3, pyridine bound at position 3 and substituted with C₁-C₃ alkyl group at position 4, non-substituted pyrazole bound at position 4, non-substituted pyrimidine bound at position 5, nor pyrimidine bound at position 5 and substituted with C₁-C₃ alkyl group at position 4].

In the present invention, a group selected from the group consisting of the following formulas is more preferable among the above R³: 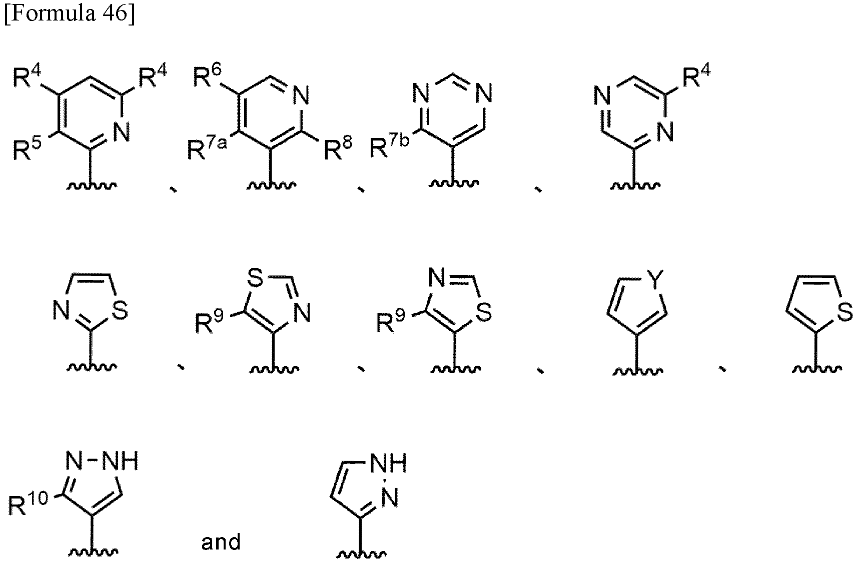
[wherein Y represents an oxygen atom or a sulfur atom,
R⁴ each independently represents a hydrogen atom or a C₁-C₃ alkoxy group,
R⁵ represents a hydrogen atom, a fluorine atom, or a C₁-C₃ alkoxy group,
R⁶ represents a hydrogen atom, a fluorine atom, or a C₁-C₃ alkoxy group,
R^{7a} represents a hydrogen atom, a C₁-C₃ alkoxy group, or a cyclopropyl group,
R^{7b} represents a hydrogen atom, a C₁-C₃ alkyl group, a C₁-C₃ alkoxy group, or a cyclopropyl group,
R⁸ represents a hydrogen atom or a C₁-C₃ alkoxy group,
R⁹ represents a hydrogen atom or a C₁-C₃ alkyl group,
R¹⁰ represents a hydrogen atom, a C₁-C₃ alkyl group, a C₁-C₃ alkoxy group, or a cyano group, and
when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom,
provided that when Z is: 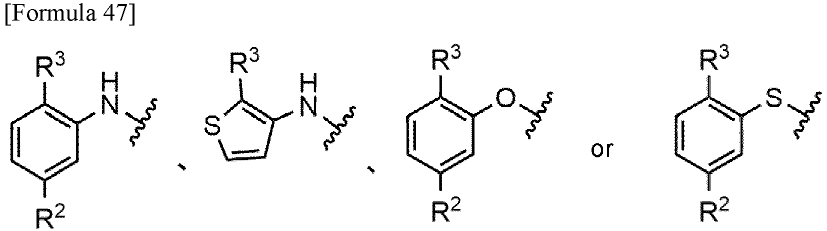 , R³ is neither pyridine bound at position 2 and substituted with a C₁-C₃ alkoxy group at position 3 nor pyridine bound at position 5 and substituted with a C₁-C₃ alkoxy group at position 3].

In the present invention, a group selected from the group consisting of the following formulas is further more preferable among the above R³: 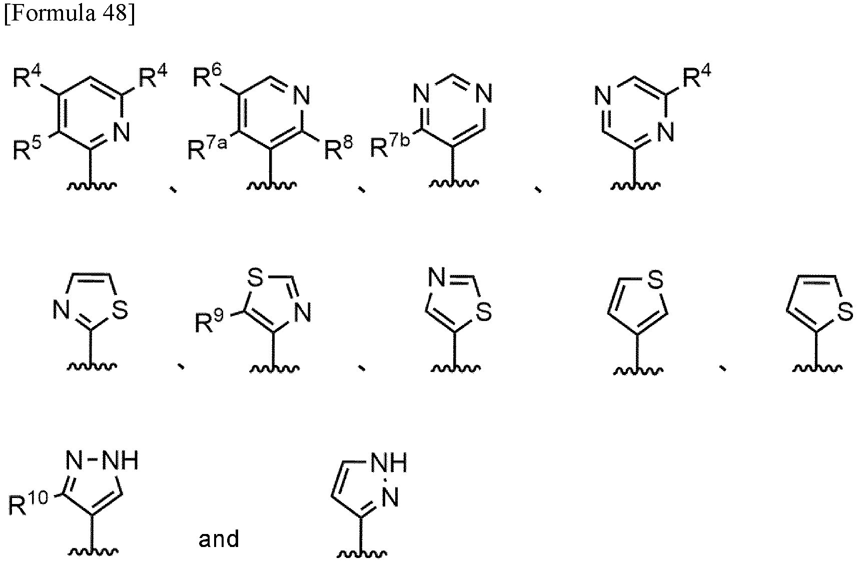
[wherein R⁴ each independently represents a hydrogen atom or a methoxy group,
R⁵ represents a hydrogen atom, a fluorine atom, or a methoxy group,
R⁶ represents a hydrogen atom, a fluorine atom, or a methoxy group,
R^{7a} represents a hydrogen atom, a methoxy group, or a cyclopropyl group,
R^{7b} represents a hydrogen atom, a methyl group, or a methoxy group,
R⁸ represents a hydrogen atom or a methoxy group,
R⁹ represents a hydrogen atom or a methyl group,
R¹⁰ represents a hydrogen atom, a methyl group, a methoxy group, or a cyano group, and
when R⁶ is a methoxy group, R^{7a} and R⁸ are hydrogen atoms; when R⁶ is a fluorine atom, R⁸ is a hydrogen atom; and when R⁸ is a methoxy group, R⁶ is a hydrogen atom or a fluorine atom, and R^{7a} is a hydrogen atom,
provided that when Z is: 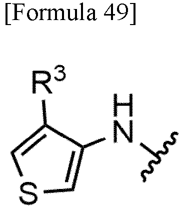 , R³ is not 3-methylpyrazole bound at position 4;
when Z is: 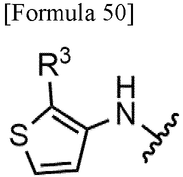 , R³ is neither non-substituted pyridine bound at position 3, 3-methylpyrazole bound at position 4, 2-methoxypyridine bound at position 3, 4-methoxypyridine bound at position 3, nor non-substituted thiophene bound at position 2;
when Z is: 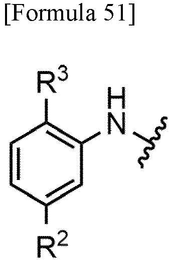 , R³ is neither non-substituted pyrimidine bound at position 5, 3-methylpyrazole bound at position 4, 2-methoxypyridine bound at position 3, 4-methoxypyridine bound at position 3, nor non-substituted thiophene bound at position 2;
when Z is: 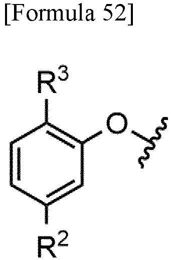 , and R² is a methyl group, R³ is neither non-substituted pyridine bound at position 3, 2-methoxypyridine bound at position 3, 4-methoxypyridine bound at position 3, nor non-substituted thiophene bound at position 2;
when Z is the same as described above, and R² is a hydrogen atom, R³ is neither non-substituted pyridine bound at position 3, 3-methylpyrazole bound at position 4, nor non-substituted thiophene bound at position 2;
when Z is the same as described above, and R² is a fluorine atom, R³ is neither non-substituted pyridine bound at position 3, 4-methylpyrimidine bound at position 5, 2-methoxypyridine bound at position 3, nor non-substituted thiophene bound at position 2; and
when Z is: 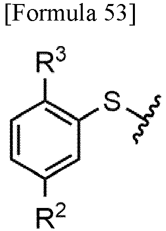 , R³ is neither non-substituted pyridine bound at position 3, 3-methylpyrazole bound at position 4, 2-methoxypyridine bound at position 3, 4-methoxypyridine bound at position 3, nor non-substituted thiophene bound at position 2].

In the present invention, a group selected from the group consisting of the following formulas is yet further more preferable among the above R³: 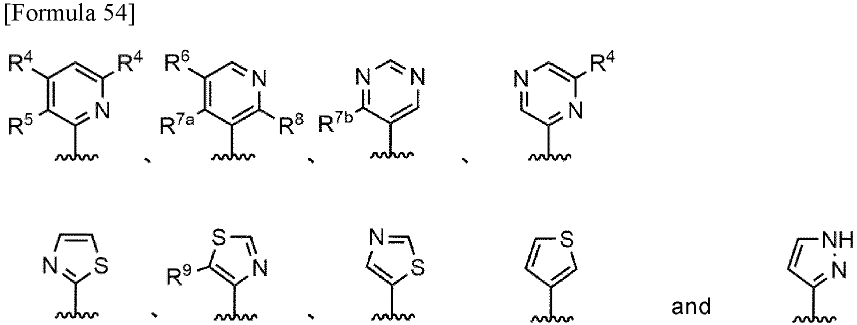
[wherein R⁴ each independently represents a hydrogen atom or a methoxy group,
R⁵ represents a hydrogen atom, a fluorine atom, or a methoxy group,
R⁶ represents a hydrogen atom, a fluorine atom, or a methoxy group,
R^{7a} represents a hydrogen atom, a methoxy group, or a cyclopropyl group,
R^{7b} represents a methoxy group,
R⁸ represents a hydrogen atom or a methoxy group,
R⁹ represents a hydrogen atom or a methyl group,
when R⁶ is a methoxy group, R^{7a} and R⁸ are hydrogen atoms; when R⁶ is a fluorine atom, R⁸ is a hydrogen atom; and when R^{7a} is a methoxy group, R⁶ is a fluorine atom, and R⁸ is a hydrogen atom,
provided that when Z is: 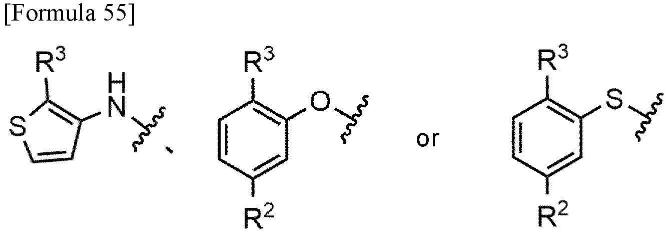 , R³ is not non-substituted pyridine bound at position 3].

In an aspect of the present invention, Z has a structure selected from the group consisting of the following formulas: 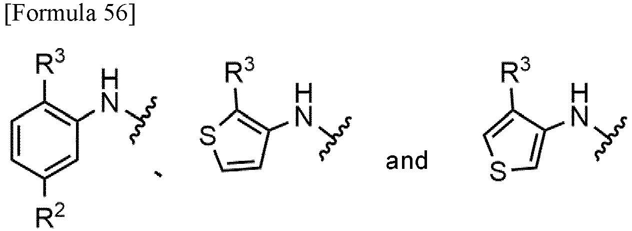 and, a structure represented by the following formula: 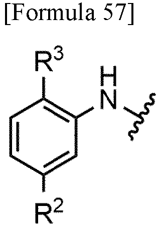 is preferable, among others.

Here, R² represents a hydrogen atom, a fluorine atom, or a C₁-C₃ alkyl group.

Also, R³ is preferably a group selected from the group consisting of the following formulas: 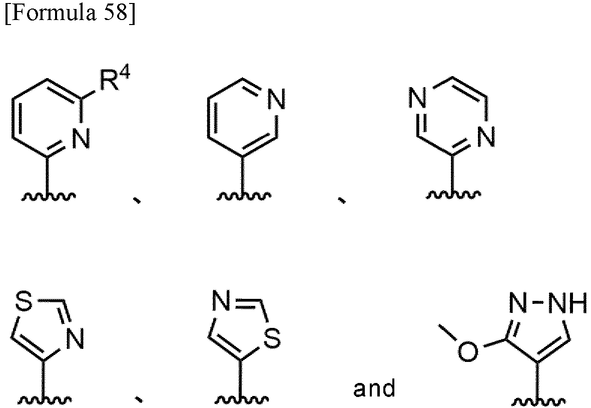 [wherein R⁴ represents a hydrogen atom or a methoxy group].

In another aspect of the present invention, Z is represented by the following formula:

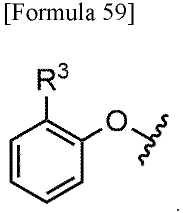

Here, R³ is preferably a group selected from the group consisting of the following formulas: 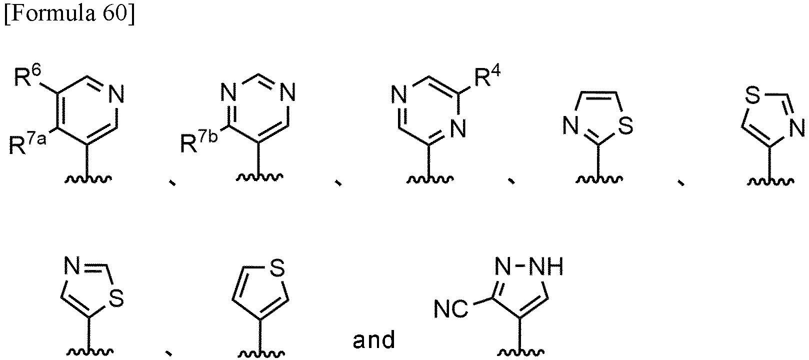
[wherein R⁴ represents a hydrogen atom or a methoxy group,
R⁶ represents a hydrogen atom or a fluorine atom,
R^{7a} represents a hydrogen atom, a methyl group, a methoxy group, or a cyclopropyl group,
R^{7b} represents a hydrogen atom, a methyl group, or a methoxy group,
when R⁶ is a hydrogen atom, R^{7a} represents a methoxy group or a cyclopropyl group; when R^{7a} is a methyl group, R⁶ is a fluorine atom; and when R^{7a} is a cyclopropyl group, R⁶ is a hydrogen atom.

In the present invention, a compound having a strong α1A agonistic action is preferable. As described above, selecting more preferable substituents Z or R³ allows a compound having a strong α1A agonistic action to be obtained.

Also, of two enantiomers that occur when R¹ is a C₁-C₃ alkyl group, the enantiomer having a weaker α1A agonistic action than 4-(2-((1H-imidazol-4-yl)methoxy)phenyl)-1H-pyrazole (Example 47) is excluded from the scope of the present invention. The enantiomer having a weaker α1A agonistic action than 4-(2-((1H-imidazol-4-yl)methoxy)phenyl)-1H-pyrazole (Example 47) is, for example, an enantiomer having an EC₅₀ value of 10 nM or more in the assay of pharmacological test examples in the present specification. In a preferred aspect of the present invention, of the two enantiomers generated when R⁵ is a C₁-C₃ alkyl group, the enantiomer having a weaker α1A agonistic action is excluded. Note that normally, enantiomers with (+) optical rotation have a stronger α1A agonistic action than (-) enantiomers. In other words, in a preferred aspect of the present invention, of the two enantiomers generated when R⁵ is a C₁-C₃ alkyl group, the enantiomer with (+) optical rotation is included in the present invention, but the (-) enantiomer is not included.

A pharmacologically acceptable salt of the present compound represented by the general formula (I) is included in the scope of the present invention. Examples of the pharmacologically acceptable salt include salts with amino acids (e.g., salts with aspartic acid and glutamic acid), salts with inorganic acids (e.g., salts with hydrochloride, hydrobromic acid, sulfuric acid, and nitric acid), and salts with organic acids (e.g., salts with formic acid, acetic acid, and trifluoroacetic acid). The reaction of forming these salts can be carried out according to a conventional method.

Compounds converted to the compound represented by the general formula (I) by reactions with enzymes and gastric acids in the physiological conditions in the living body are included in the scope of the present invention. Examples thereof include compounds in which the imidazole group of the compound represented by the general formula (I) was subjected to amidation (e.g., acetamidation).

The compound represented by the general formula (I), which is the present compound, can be produced by the methods shown in the following reaction schemes I to IX, the methods described in Examples, or a combination of known methods. Note that a compound that is used as a starting material or a reagent necessary for producing the present compound and for which the production method is not indicated is commercially available or can be produced by referring to a known method.

The reaction scheme I represents a method for producing a compound (4) in which Z is bound via a nitrogen atom, and R¹ is a hydrogen atom in the general formula (I). 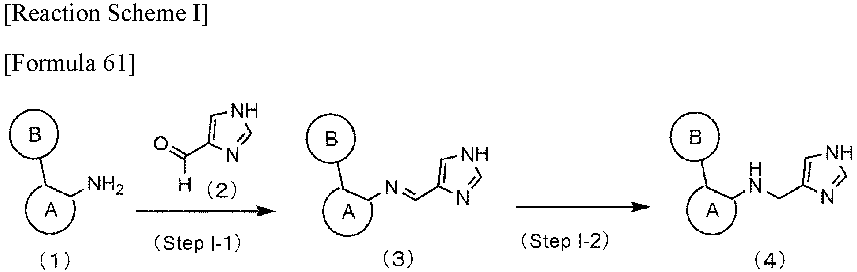
[wherein the ring A represents a ring selected from the group consisting of the following formulas:] 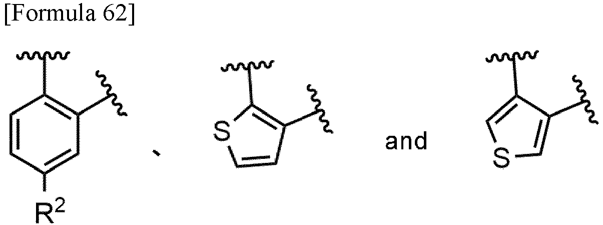
[wherein R² represents a hydrogen atom, a fluorine atom, or a C₁-C₃ alkyl group].

Also, the ring B in the above scheme represents a ring selected from the group consisting of the following formulas: 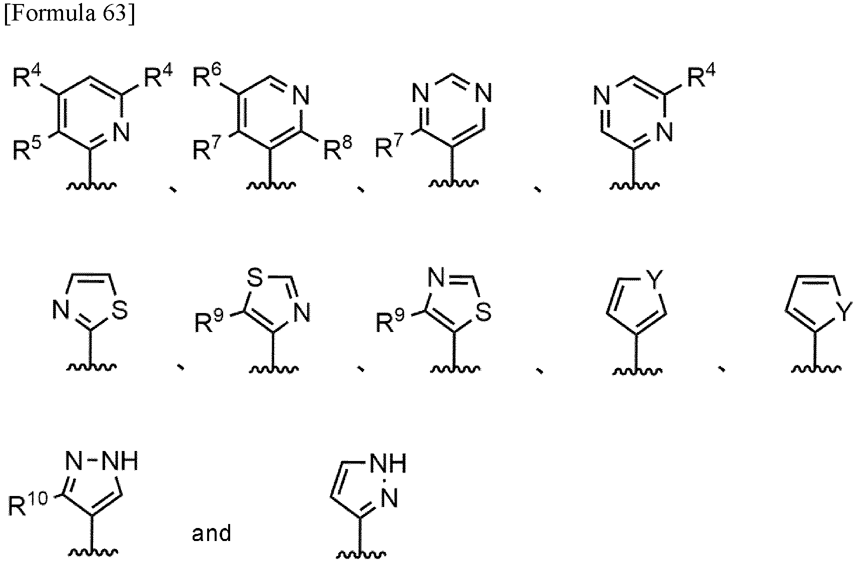
[wherein Y represents an oxygen atom or a sulfur atom,
R⁴ each independently represents a hydrogen atom or a C₁-C₃ alkoxy group,
R⁵ represents a hydrogen atom, a fluorine atom, a C₁-C₃ alkyl group, or a C₁-C₃ alkoxy group,
R⁶ represents a hydrogen atom, a fluorine atom, or a C₁-C₃ alkoxy group,
R⁷ represents a hydrogen atom, a C₁-C₃ alkyl group, a C₁-C₃ alkoxy group, or a cyclopropyl group,
R⁸ represents a hydrogen atom or a C₁-C₃ alkoxy group,
R⁹ represents a hydrogen atom or a C₁-C₃ alkyl group,
R¹⁰ represents a hydrogen atom, a C₁-C₃ alkyl group, a C₁-C₃ alkoxy group, or a cyano group, and
when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom].

### [Step I-1]

A compound represented by the general formula (1) and 4-formylimidazole (2) are reacted in a suitable solvent (e.g., N,N-dimethylacetamide, methanol, tetrahydrofuran, toluene, dichloromethane, or a mixed solvent thereof) with an additive (e.g., acetic acid, triethylamine, or titanium tetraisopropoxide) to obtain a compound represented by the general formula (3). The reaction temperature ranges from room temperature to the boiling point of the solvent, and the reaction time ranges from 30 minutes to 48 hours.

### [Step 1-2]

The compound represented by the general formula (3) is reacted in a suitable solvent (e.g., N,N-dimethylacetamide, methanol, tetrahydrofuran, toluene, dichloromethane, or a mixed solvent thereof) with a reductant (e.g., sodium borohydride, or sodium triacetoxyborohydride) to obtain the compound represented by the general formula (4). The reaction temperature ranges from room temperature to the boiling point of the solvent, and the reaction time ranges from 30 minutes to 48 hours. Note that the reactions in step I-1 and step I-2 may be conducted serially without isolating or purifying after step I-1 or in one pot process.

The reaction scheme II represents a method for producing a compound (9) in which Z is bound via a nitrogen atom, and R¹ is a C₁-C₃ alkyl group in the general formula (I). 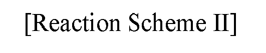 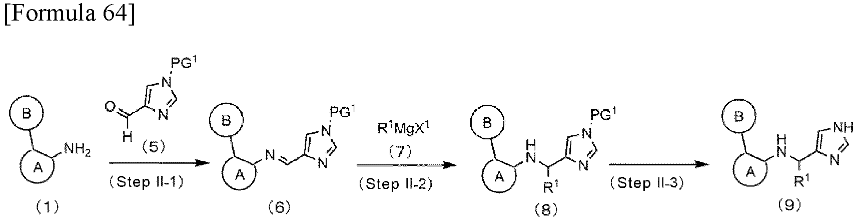
[wherein the rings A and B represent the same rings as in the reaction scheme I,
PG¹ represents a protecting group for an imidazolyl group,
R¹ represents a C₁-C₃ alkyl group,
X¹ represents a bromine atom or a chlorine atom].

### [Step II-1]

The compound represented by the general formula (1) and a compound represented by the general formula (5) are reacted in a suitable solvent (e.g., N,N-dimethylacetamide, methanol, tetrahydrofuran, toluene, dichloromethane, or a mixed solvent thereof) with an additive (e.g., acetic acid, p-toluenesulfonic acid, triethylamine, titanium tetraisopropoxide, or molecular sieves) to obtain a compound represented by the general formula (6). The reaction temperature ranges from room temperature to the boiling point of the solvent, and the reaction time ranges from 30 minutes to 48 hours.

### [Step II-2]

The compound represented by the general formula (6) is reacted in a suitable solvent (e.g., tetrahydrofuran) with a Grignard reagent (e.g., methylmagnesium bromide) (7) to obtain a compound represented by the general formula (8). The reaction temperature ranges from 0°C to the boiling point of the solvent, and the reaction time ranges from 30 minutes to 24 hours.

### [Step II-3]

By referring to the method described in "Protecting Groups in Organic Synthesis, 5th Edition, Wiley (2014)", the protecting group for the compound represented by the general formula (8) is removed to obtain the compound represented by the general formula (9).

The compound represented by the general formula (1) used as the starting material in the reaction schemes I and II can be produced by the method shown in the following reaction scheme III, the methods described in Reference Examples, or a combination of known methods. 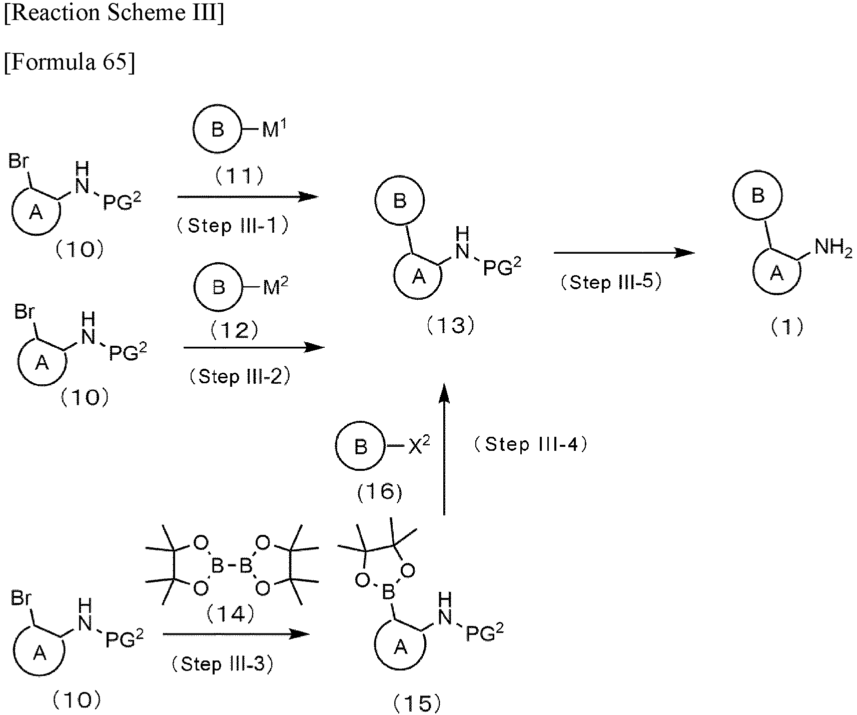
[In the scheme, PG² represents a protecting group for the amino group or a hydrogen atom,
the rings A and B represent the same rings as in the reaction scheme I,
M¹ represents -B(OH)₂ or 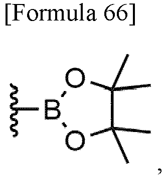
M² represents a C₁-C₆ trialkyltin group,
X² represents a bromine atom or an iodine atom].

Note that when PG² is a hydrogen atom, the compound (1) can be obtained without going through a compound represented by the general formula (13) and step III-5.

### [Step III-1]

A compound represented by the general formula (10) and a compound represented by the general formula (11) are reacted in a suitable solvent (e.g., dioxane, water, or a mixed solvent of dioxane and water), in the presence of a base (e.g., potassium carbonate, sodium carbonate, or potassium phosphate), with a palladium catalyst (e.g., palladium acetate, palladium chloride, [1,1'-bis(diphenylphosphino)ferrocene]palladium dichloride, tetrakis(triphenylphosphine)palladium, or [1,1'-bis(di-t-butylphosphino)ferrocene]palladium dichloride) to obtain the compound represented by the general formula (13). The reaction temperature ranges from room temperature to the boiling point of the solvent, and the reaction time ranges from 30 minutes to 48 hours.

### [Step III-2]

The compound represented by the general formula (10) and a compound represented by the general formula (12) are reacted in a suitable solvent (e.g., dioxane, or toluene), in the presence or absence of an additive (e.g., potassium fluoride), with a palladium catalyst (e.g., tetrakis(triphenylphosphine)palladium) to obtain the compound represented by the general formula (13). The reaction temperature ranges from room temperature to the boiling point of the solvent, and the reaction time ranges from 30 minutes to 48 hours.

### [Step III-3]

The compound represented by the general formula (10) and bis(pinacolato)diboron (14) are reacted in a suitable solvent (e.g., dioxane), in the presence of a base (e.g., potassium acetate), with a palladium catalyst (e.g., tetrakis(triphenylphosphine)palladium) to obtain a compound represented by the general formula (15). The reaction temperature ranges from room temperature to the boiling point of the solvent, and the reaction time ranges from 30 minutes to 48 hours.

### [Step III-4]

The compound represented by the general formula (15) and a compound represented by the general formula (16) are reacted in a suitable solvent (e.g., dioxane, water, or a mixed solvent of dioxane and water), in the presence of a base (e.g., potassium carbonate, sodium carbonate, or potassium phosphate), with a palladium catalyst (e.g., palladium acetate, palladium chloride, [1,1'-bis(diphenylphosphino)ferrocene]palladium dichloride, tetrakis(triphenylphosphine)palladium, or [1,1'-bis(di-t-butylphosphino)ferrocene]palladium dichloride) to obtain the compound represented by the general formula (13). The reaction temperature ranges from room temperature to the boiling point of the solvent, and the reaction time ranges from 30 minutes to 48 hours.

### [Step III-5]

When PG² of the compound represented by the general formula (13) is a protecting group for the amino group, the protecting group is removed by referring to the method described in "Protecting Groups in Organic Synthesis, 5th Edition, Wiley (2014)" to obtain the compound represented by the general formula (1).

The reaction scheme IV represents a method for producing the compound (4) in which Z is bound via a nitrogen atom, and R¹ is a hydrogen atom in the general formula (I), and a method alternative to the reaction scheme I. 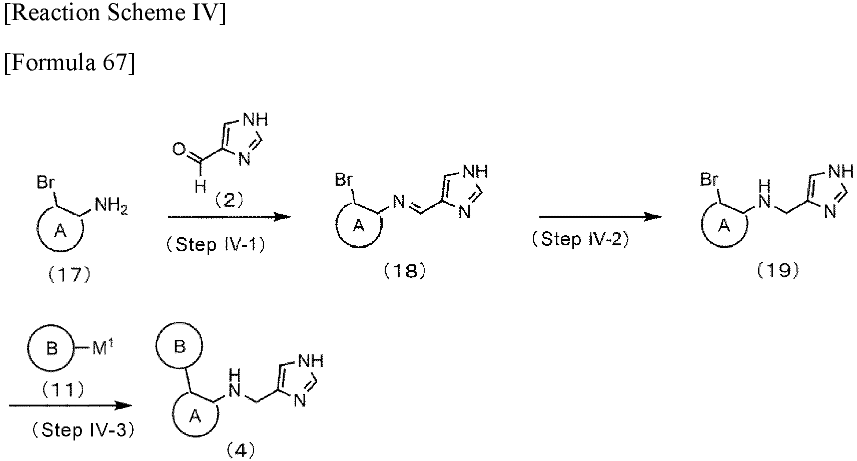
[In the scheme, the rings A and B represent the same rings as in the reaction scheme I,
M¹ represents the same group as in the reaction scheme III.]

### [Step IV-1]

A compound represented by the general formula (17) and 4-formylimidazole (2) are reacted in a suitable solvent (e.g., N,N-dimethylacetamide, methanol, tetrahydrofuran, toluene, dichloromethane, or a mixed solvent thereof) with an additive (e.g., acetic acid, triethylamine, or titanium tetraisopropoxide) to obtain a compound represented by the general formula (18). The reaction temperature ranges from room temperature to the boiling point of the solvent, and the reaction time ranges from 30 minutes to 48 hours.

### [Step IV-2]

The compound represented by the general formula (18) is reacted in a suitable solvent (e.g., N,N-dimethylacetamide, methanol, tetrahydrofuran, toluene, dichloromethane, or a mixed solvent thereof) with a reductant (e.g., sodium borohydride, or sodium triacetoxyborohydride) to obtain a compound represented by the general formula (19). The reaction temperature ranges from room temperature to the boiling point of the solvent, and the reaction time ranges from 30 minutes to 48 hours. Note that the reactions in step IV-1 and step IV-2 may be conducted serially without isolating or purifying after step IV-1 or in one pot process.

### [Step IV-3]

The compound represented by the general formula (19) and the compound represented by the general formula (11) are reacted in a suitable solvent (e.g., dioxane, water, or a mixed solvent of dioxane and water), in the presence of a base (e.g., potassium carbonate, sodium carbonate, or potassium phosphate), with a palladium catalyst (e.g., palladium acetate, palladium chloride, [1,1'-bis(diphenylphosphino)ferrocene]palladium dichloride, tetrakis(triphenylphosphine)palladium, or [1,1'-bis(di-t-butylphosphino)ferrocene]palladium dichloride) to obtain the compound represented by the general formula (4). The reaction temperature ranges from room temperature to the boiling point of the solvent, and the reaction time ranges from 30 minutes to 48 hours.

The reaction scheme V represents a method for producing a compound (25) in which Z is bound via an oxygen atom, and R¹ is a hydrogen atom in the general formula (I). 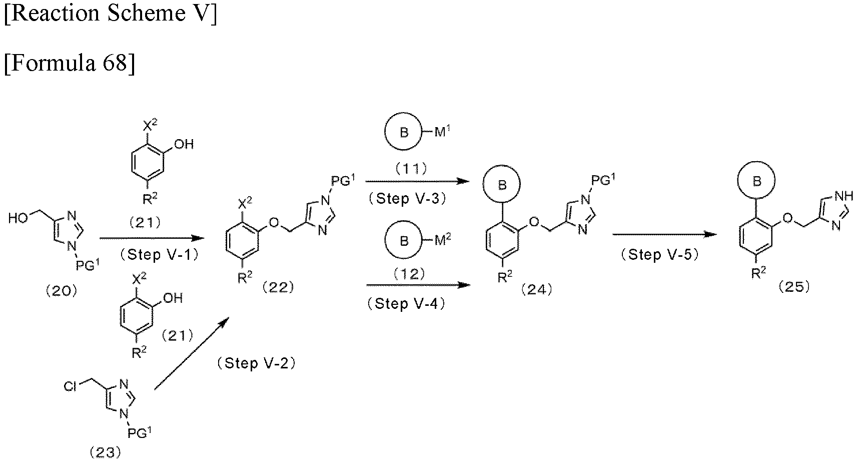
[In the scheme, PG¹ represents the same group as in the reaction scheme II,
X² represents the same group as in the reaction scheme III,
R² represents the same group as in the reaction scheme I,
the ring B represents the same ring as in the reaction scheme I,
M¹ and M² represent the same group as in the reaction scheme III].

### [Step V-1]

A compound represented by the general formula (20) and a compound represented by the general formula (21) are reacted in a suitable solvent (e.g., tetrahydrofuran), in the presence of an additive (e.g., triphenylphosphine), with a Mitsunobu reaction reagent (e.g., diisopropyl azodicarboxylate) to obtain a compound represented by the general formula (22). The reaction temperature ranges from 0°C to the boiling point of the solvent, and the reaction time ranges from 30 minutes to 48 hours.

### [Step V-2]

A compound represented by the general formula (23) and the compound represented by the general formula (21) are reacted in a suitable solvent (e.g., N,N-dimethylformamide, dimethylsulfoxide, or N-methyl-2-pyrrolidone), in the presence or absence of an additive (e.g., potassium iodide), with a base (e.g., potassium carbonate, or sodium carbonate) to obtain the compound represented by the general formula (22). The reaction temperature ranges from 0°C to the boiling point of the solvent, and the reaction time ranges from 30 minutes to 48 hours.

### [Step V-3]

The compound represented by the general formula (22) and the compound represented by the general formula (11) are reacted in a suitable solvent (e.g., dioxane, water, or a mixed solvent of dioxane and water), in the presence of a base (e.g., potassium carbonate, sodium carbonate, or potassium phosphate), with a palladium catalyst (e.g., palladium acetate, palladium chloride, [1,1'-bis(diphenylphosphino)ferrocene]palladium dichloride, tetrakis(triphenylphosphine)palladium, or [1,1'-bis(di-t-butylphosphino)ferrocene]palladium dichloride) to obtain a compound represented by the general formula (24). The reaction temperature ranges from room temperature to the boiling point of the solvent, and the reaction time ranges from 30 minutes to 48 hours.

### [Step V-4]

The compound represented by the general formula (22) and the compound represented by the general formula (12) are reacted in a suitable solvent (e.g., dioxane, or toluene), in the presence or absence of an additive (e.g., potassium fluoride), with a palladium catalyst (e.g., tetrakis(triphenylphosphine)palladium) to obtain the compound represented by the general formula (24). The reaction temperature ranges from room temperature to the boiling point of the solvent, and the reaction time ranges from 30 minutes to 48 hours.

### [Step V-5]

By referring to the method described in "Protecting Groups in Organic Synthesis, 5th Edition, Wiley (2014)", the protecting group for the compound represented by the general formula (24) is removed to obtain a compound represented by the general formula (25).

The reaction scheme VI represents a method for producing the compound (25) in which Z is bound via an oxygen atom, and R¹ is a hydrogen atom in the general formula (I), and a method alternative to the reaction scheme V. 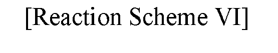 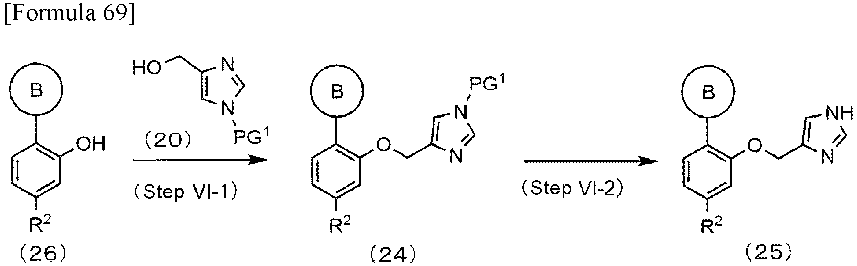
[In the scheme, the ring B represents the same ring as in the reaction scheme I,
R² represents the same group as in the reaction scheme I,
PG¹ represents the same group as in the reaction scheme II].

### [Step VI-1]

A compound represented by the general formula (26) and the compound represented by the general formula (20) are reacted in a suitable solvent (e.g., tetrahydrofuran), in the presence of an additive (e.g., triphenylphosphine), with a Mitsunobu reaction reagent (e.g., diisopropyl azodicarboxylate) to obtain the compound represented by the general formula (24). The reaction temperature ranges from 0°C to the boiling point of the solvent, and the reaction time ranges from 30 minutes to 48 hours.

### [Step VI-2]

By referring to the method described in "Protecting Groups in Organic Synthesis, 5th Edition, Wiley (2014)", the protecting group for the compound represented by the general formula (24) is removed to obtain a compound represented by the general formula (25).

The reaction scheme VII represents a method for producing a compound (30) in which Z is bound via an oxygen atom, and R¹ is a C₁-C₃ alkyl group in the general formula (I). 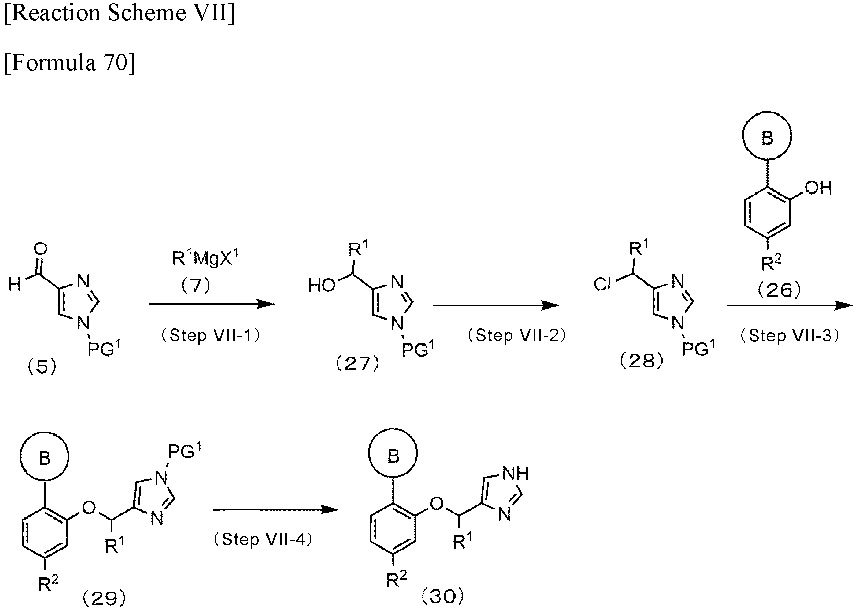
[In the scheme, PG¹, X¹, and R¹ represent the same groups as in the reaction scheme II,
R² represents the same group as in the reaction scheme I,
the ring B represents the same ring as in the reaction scheme I].

### [Step VII-1]

The compound represented by the general formula (5) is reacted in a suitable solvent (e.g., tetrahydrofuran) with a Grignard reagent (e.g., methylmagnesium bromide) (7) to obtain a compound represented by the general formula (27). The reaction temperature ranges from 0°C to the boiling point of the solvent, and the reaction time ranges from 30 minutes to 24 hours.

### [Step VII-2]

The compound represented by the general formula (27) is reacted in a suitable solvent (e.g., chloroform), in the presence or absence of an additive (e.g., triethylamine), with a chlorinating agent (e.g., methanesulfonyl chloride) to obtain a compound represented by the general formula (28). The reaction temperature ranges from 0°C to the boiling point of the solvent, and the reaction time ranges from 30 minutes to 24 hours.

### [Step VII-3]

The compound represented by the general formula (28) and the compound represented by the general formula (26) are reacted in a suitable solvent (e.g., N,N-dimethylformamide, dimethylsulfoxide, or N-methyl-2-pyrrolidone), in the presence or absence of an additive (e.g., potassium iodide), with a base (e.g., potassium carbonate, or sodium carbonate) to obtain a compound represented by the general formula (29). The reaction temperature ranges from 0°C to the boiling point of the solvent, and the reaction time ranges from 30 minutes to 48 hours.

### [Step VII-4]

By referring to the method described in "Protecting Groups in Organic Synthesis, 5th Edition, Wiley (2014)", the protecting group for the compound represented by the general formula (29) is removed to obtain a compound represented by the general formula (30).

The compound represented by the general formula (26) used as the starting material in the reaction schemes VI and VII can be produced by the method shown in the following reaction scheme VIII, the methods described in Reference Examples, or a combination of known methods. 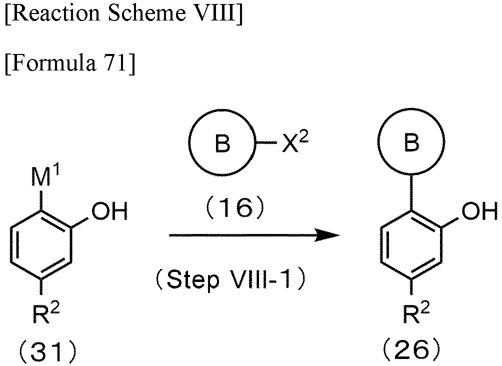
In the scheme, M¹ represents the same group as in the reaction scheme III,
the ring B represents the same ring as in the reaction scheme I,
X² represents the same group as in the reaction scheme III.

### [Step VIII-1]

A compound represented by the general formula (31) and the compound represented by the general formula (16) are reacted in a suitable solvent (e.g., dioxane, water, or a mixed solvent of dioxane and water), in the presence of a base (e.g., potassium carbonate, sodium carbonate, or potassium phosphate), with a palladium catalyst (e.g., palladium acetate, palladium chloride, [1,1'-bis(diphenylphosphino)ferrocene]palladium dichloride, tetrakis(triphenylphosphine)palladium, or [1,1'-bis(di-t-butylphosphino)ferrocene]palladium dichloride) to obtain the compound represented by the general formula (26). The reaction temperature ranges from room temperature to the boiling point of the solvent, and the reaction time ranges from 30 minutes to 48 hours.

The reaction scheme IX represents a method for producing a compound (36) in which Z is bound via a sulfur atom, and R¹ is a hydrogen atom in the general formula (I). 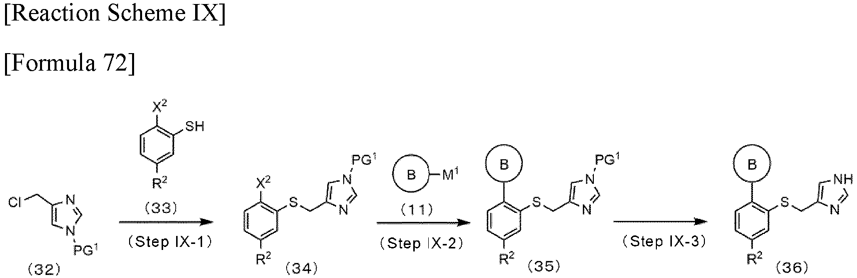
[In the scheme, PG¹ represents the same group as in the reaction scheme II,
R² represents the same group as in the reaction scheme I,
the ring B represents the same ring as in the reaction scheme I,
M¹ and X² represent the same groups as in the reaction scheme III].

### [Step IX-1]

A compound represented by the general formula (32) and a compound represented by the general formula (33) are reacted in a suitable solvent (e.g., N,N-dimethylformamide, dimethylsulfoxide, or N-methyl-2-pyrrolidone), in the presence or absence of an additive (e.g., potassium iodide), with a base (e.g., potassium carbonate, or sodium carbonate) to obtain a compound represented by the general formula (34). The reaction temperature ranges from 0°C to the boiling point of the solvent, and the reaction time ranges from 30 minutes to 48 hours.

### [Step IX-2]

The compound represented by the general formula (34) and the compound represented by the general formula (11) are reacted in a suitable solvent (e.g., dioxane, water, or a mixed solvent of dioxane and water), in the presence of a base (e.g., potassium carbonate, sodium carbonate, or potassium phosphate), with a palladium catalyst (e.g., palladium acetate, palladium chloride, [1,1'-bis(diphenylphosphino)ferrocene]palladium dichloride, tetrakis(triphenylphosphine)palladium, or [1,1'-bis(di-t-butylphosphino)ferrocene]palladium dichloride) to obtain a compound represented by the general formula (35). The reaction temperature ranges from room temperature to the boiling point of the solvent, and the reaction time ranges from 30 minutes to 48 hours.

### [Step IX-3]

By referring to the method described in "Protecting Groups in Organic Synthesis, 5th Edition, Wiley (2014)", the protecting group for the compound represented by the general formula (35) is removed to obtain the compound represented by the general formula (36).

For substituents (e.g., hydroxyl group, amino group, and carboxy group) contained in the present compound and compounds used for producing the compound, it may be effective in compound production that a suitable protecting group is introduced in the substituents when they are in the form of raw materials or intermediates, and the protecting groups described in the above-described "Protecting Groups in Organic Synthesis, 5th Edition, Wiley (2014)" can be appropriately selected as necessary.

In order to isolate and purify the present compound and compounds used for producing the compound from a reaction solution, commonly used methods can be employed. For example, solvent extraction, ion exchange resin, column chromatography using silica gel, alumina, or the like as a carrier, high performance liquid chromatography (HPLC) fractionation, thin-layer chromatography, scavenger resin, recrystallization, and the like can be employed, and methods for isolating and purifying them can be carried out alone or in combination thereof. Isolation and purification may be carried out for each reaction, or after completion of some reactions.

When the compound in this specification has an asymmetric carbon, and optical isomers are present, these optical isomers can be resolved by a general optical resolution method for a racemic compound, such as a fractional crystallization method in which the compound is recrystallized as a diastereomeric salt with a general optically active compound, or a method for subjecting the compound to chromatography as a diastereomer by a reaction with a general optically active compound, or the like. In addition, each optical isomer can also be resolved by high performance liquid chromatography (HPLC) fractionation using a column for separating an optically active substance.

Since the present compound thus produced serves as an adrenergic α1A receptor agonist, it can be used as a therapeutic drug for orthostatic hypotension, essential hypotension, acute hypotension associated with various diseases or conditions, and urinary incontinence.

In order to prevent or treat a disease selected from the group consisting of orthostatic hypotension, essential hypotension, acute hypotension associated with various diseases or conditions, and urinary incontinence, a therapeutically effective amount of the present compound can be administered to a subject in need thereof. Examples of the subject include humans and non-human mammals, and preferably humans.

The administration form of a pharmaceutical composition containing the present compound can be oral or parenteral, and various administration forms can be selected according to the purpose. Examples of oral administration forms include tablets, capsules, powders, and granules, while examples of parenteral administration forms include patches, liniments, and ointments. When forming into a convenient tablet form, orally ingestible components used in the art can be appropriately selected. Examples thereof include excipients, binders, disintegrants, lubricants, flavoring agents, and anti-aggregation agents.

The dosage of a medicament containing the present compound as an active ingredient is not particularly limited and can be appropriately selected. The dosage of a medicament containing the present compound is appropriately determined according to its administration form, usage, age, sex and other conditions of a patient, and the severity of a disease. In a case of oral administration, the daily dosage of the present compound ranges from about 0.1 µg to 100 mg per kg of body weight, which can be appropriately administered 1 to 4 times per day. However, the dosage and the number of administration are determined according to relevant circumstances including the severity of symptoms to be treated, the selection of the compound to be administered, and the selected administration route, and therefore, the range of the dosage and the number of administration do not limit the scope of the present invention.

### [Examples]

Hereinafter, the present invention will be explained in more detail with examples, reference examples, and pharmacological test examples; however, the technical scope of the present invention is not limited to the contents of the description.

### [Nuclear Magnetic Resonance Spectrum]

Nuclear magnetic resonance (¹H-NMR) spectra in the following Examples and Reference Examples were measured with 400 MR manufactured by Agilent Technologies, Inc. and AVANCE NEO 400 manufactured by Bruker Corporation, and their chemical shift values were recorded in δ values (ppm) using tetramethylsilane as an internal standard reference. The splitting patterns were designated as singlet "s", doublet "d", triplet "t", quartet "q", quintet "quint", multiplet "m", and broad line "br".

Mass spectrometry was performed by electrospray ionization (ESI) under any of the following measurement conditions of A to D.

### [Measurement Condition A]

Measurement equipment: ACQUITY UPLC/SQD system manufactured by Waters Corporation
Column: ACQUITY UPLC BEH C18 (1.7 µm, 2.1 mm × 50 mm)
Column temperature: 50°C
Flow rate: 1.5 mL/min
UV detection wavelength: 210-400 nm
Mobile phase: [A] water/acetonitrile/formic acid = 97/3/0.1; [B] water/acetonitrile/formic acid = 5/95/0.1
Gradient: [A]/[B] = 95/5 to 1/99 (linear gradient over 1 minute)

### [Measurement Condition B]

Measurement equipment: LC/MSD 1200 series manufactured by Agilent Technologies, Inc.
Column: Xbridge-C18 (1.7 µm, 2.1 mm × 50 mm)
Column temperature: 30°C
Flow rate: 1.5 mL/min
UV detection wavelength: 214 or 254 nm
Mobile phase: [A] 0.1% trifluoroacetic acid-containing aqueous solution; [B] acetonitrile
Gradient: [A]/[B] = 95/5 to 5/95 (linear gradient over 2.5 minutes)

### [Measurement Condition C]

Measurement equipment: LC/MSD 1200 series manufactured by Agilent Technologies, Inc.
Column: Xbridge-C18 (1.7 µm, 2.1 mm × 50 mm)
Column temperature: 30°C
Flow rate: 1.5 mL/min
UV detection wavelength: 214 or 254 nm
Mobile phase: [A] 0.1% ammonium bicarbonate aqueous solution; [B] acetonitrile
Gradient: [A]/[B] = 95/5 to 5/95 (linear gradient over 2.5 minutes)

In the Tables, "Me" represents a methyl group, "Et" represents an ethyl group, "Pr" represents a propyl group, "Bu" represents a butyl group, "Trt" represents a trityl group, "Boc" represents a t-butoxy carbonyl group, "Ac" represents an acetyl group, "DMA" represents N,N-dimethylacetamide, "THF" represents tetrahydrofuran, "TFA" represents trifluoroacetic acid, "dppf" represents 1,1'-bis(diphenylphosphino)ferrocene, and "dtbpf" represents 1,1'-bis(di-t-butylphosphino)ferrocene.

### <Production of Present Compound in Which Biaryl Moiety and Imidazole Moiety are Bound Via Nitrogen Atom>

### Production of Biarylamine 1, Step III-1 (Reference Examples 1 to 11)

### [Reference Example 1]

### t-Butyl [2,3'-bithiophen]-4'-ylcarbamate

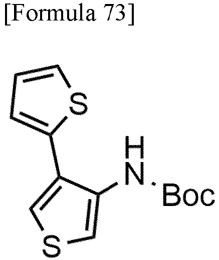

t-Butyl (4-bromothiophen-3-yl)carbamate (210 mg) was dissolved in a mixed solvent of 1,4-dioxane (5 mL) and water (2.5 mL), and to the mixture were added 2-thiopheneboronic acid (290 mg), tetrakis(triphenylphosphine)palladium (43.6 mg), and potassium carbonate (41.7 mg). The reaction vessel was then purged with argon gas, and the mixture was stirred at 90°C for 4 hours. The reaction solution was filtered through Celite, the residue was washed with ethyl acetate, and the filtrate and washing were combined and concentrated under reduced pressure. The resulting residue was purified with column chromatography on silica gel (hexane/ethyl acetate = 1/0 to 4/1) to yield the title compound (211 mg).
ESI/MS(m/z) 282 (M+H)⁺

Referring to the method of Reference Example 1, compounds were synthesized according to the following reaction scheme. The synthesized compounds and their data are shown in Table 1.

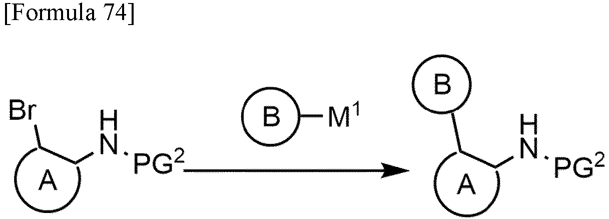

**[Table 1]**

| Reference Example | Ring A | M¹ | Ring B | PG² | Catalyst | Base | ESI/MS (m/z) |
|---|---|---|---|---|---|---|---|
| 2 | 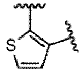 | - B(OH)₂ | 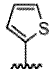 | Boc | Pd(PPh3)₄ | K₂CO₃ | 282 (M+H)⁺ |
| 3 | 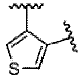 | - B(OH)₂ | 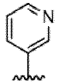 | H | Pd(PPh₃)₄ | K₂CO₃ | 277 (M+H)⁺ |
| 4 | 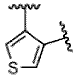 | 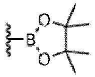 | 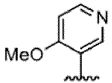 | Boc | Pd(dppf)Cl₂ | Na₂CO₃ | 307 (M+H)⁺ |
| 5 | 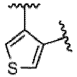 | 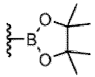 | 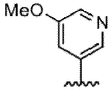 | Boc | Pd(dppf)Cl₂ | Na₂CO₃ | 307 (M+H)⁺ |
| 6 | 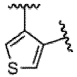 | 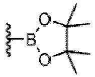 | 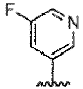 | Boc | Pd(dppt)Cl₂ | Na₂CO₃ | 295 (M+H)⁺ |
| 7 | 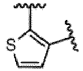 | 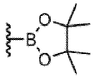 | 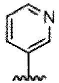 | Boc | Pd(PPh₃)₄ | K₂CO₃ | 277 (M+H)⁺ |
| 8 | 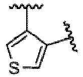 | 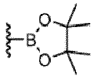 | 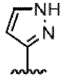 | Boc | Pd(dppf)Cl₂ | K₂CO₃ | 266 (M+H)⁺ |
| 9 | 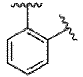 | 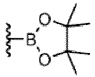 | 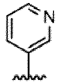 | H | Pd(dppf)Cl₂ | K₂CO₃ | 171 (M+H)⁺ |
| 10 | 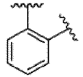 | 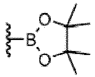 | 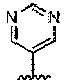 | H | Pd(dppf)Cl₂ | K₂CO₃ | 172 (M+H)⁺ |
| 11 | 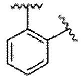 | 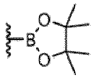 | 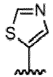 | H | Pd(dppf)Cl₂ | K₂CO₃ | 177 (M+H)⁺ |

### Production of Biarylamine 2, Step III-2 (Reference Examples 12 to 15)

### [Reference Example 12]

### t-Butyl (4-(thiazol-2-yl)thiophen-3-yl)carbamate

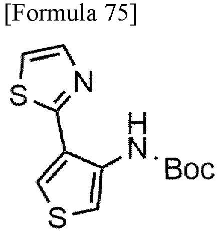

t-Butyl (4-bromothiophen-3-yl)carbamate (1.1 g) and 2-(tributylstannyl)thiazole (3.0 g) were dissolved in toluene (30 mL) and to the mixture was added tetrakis(triphenylphosphine)palladium (464 mg). The reaction vessel was then purged with nitrogen gas, and the mixture was stirred overnight at 110°C. The reaction solution was concentrated under reduced pressure, and the resulting residue was purified with column chromatography on silica gel (pentane/ethyl acetate = 20/1) to yield the title compound (451 mg).
ESI/MS(m/z) 283 (M+H)⁺.

Referring to the method of Reference Example 12, compounds were synthesized according to the following reaction scheme. The synthesized compounds and their data are shown in Table 2.

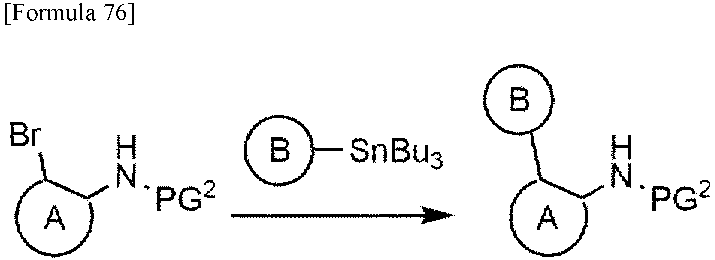

**[Table 2]**

| Reference Example | Ring A | Ring B | PG² | Additive | Solvent | ESI/MS (m/z) |
|---|---|---|---|---|---|---|
| 13 | 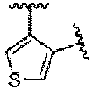 | 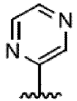 | Boc | KF | dioxane | 278 (M+H)⁺ |
| 14 | 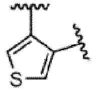 | 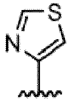 | Boc | none | toluene | 283 (M+H)⁺ |
| 15 | 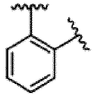 | 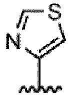 | H | none | toluene | 177 (M+H)⁺ |

### Production of Biarylamine 3, Steps III-3 and 4 (Reference Examples 16 to 36)

### [Reference Example 16]

### t-Butyl (4-(4,4,5,5-tetramethyl-1,3,2-dioxaborolan-2-yl)thiophen-3-yl)carbamate

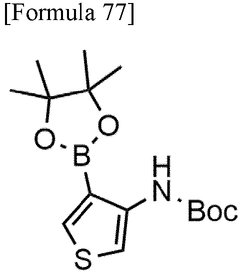

t-Butyl (4-bromothiophen-3-yl)carbamate (3 g) and potassium acetate (3.17 g) were dissolved in 1,4-dioxane (30 mL) and to the mixture were added bis(pinacolato)diboron (5.5 g) and [1,1'-bis(diphenylphosphino)ferrocene]palladium dichloride (395 mg). The reaction vessel was then purged with nitrogen gas, and the mixture was stirred overnight at 100°C. The reaction solution was concentrated under reduced pressure, and the resulting residue was purified with column chromatography on silica gel (pentane/ethyl acetate = 10/1) to yield the title compound (3.2 g).
ESI/MS(m/z) 326 (M+H)⁺.

### [Reference Example 17]

### t-Butyl (4-(3-methylpyridin-2-yl)thiophen-3-yl)carbamate

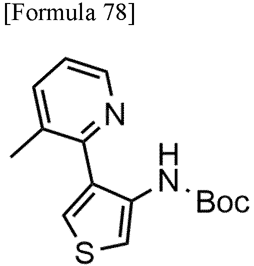

t-Butyl (4-(4,4,5,5-tetramethyl-1,3,2-dioxaborolan-2-yl)thiophen-3-yl)carbamate (Reference Example 16, 325 mg) was dissolved in a mixed solvent of 1,4-dioxane (30 mL) and water (3 mL) and to the mixture were added potassium carbonate (552 mg), 2-bromo-3-methylpyridine (345 mg), and [1,1'-bis(diphenylphosphino)ferrocene]palladium dichloride (395 mg). The reaction vessel was then purged with nitrogen gas, and the mixture was stirred overnight at 100°C. The reaction solution was concentrated under reduced pressure, and the resulting residue was purified with column chromatography on silica gel (pentane/ethyl acetate = 10/1) to yield the title compound (250 mg).
ESI/MS(m/z) 291 (M+H)⁺.

### [Reference Example 18]

### t-Butyl 4-iodo-5-methoxy-1H-pyrazole-1-carboxylate

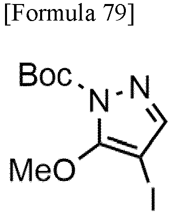

4-Iodo-3-methoxy-1H-pyrazole (1 g) and 4-dimethylaminopyridine (109 mg) were dissolved in dichloromethane (20 mL), and to the mixture was added di-t-butyl dicarbonate (1.46 g), and stirred at room temperature for 16 hours. The reaction solution was concentrated under reduced pressure, and the resulting residue was purified with column chromatography on silica gel (pentane/ethyl acetate = 20/1 to 10/1) to yield the title compound (1.2 g).
ESI/MS(m/z) 225 (M+H-boc)⁺.

Referring to the method of Reference Example 17, compounds were synthesized according to the following reaction scheme. The synthesized compounds and their data are shown in Table 3.

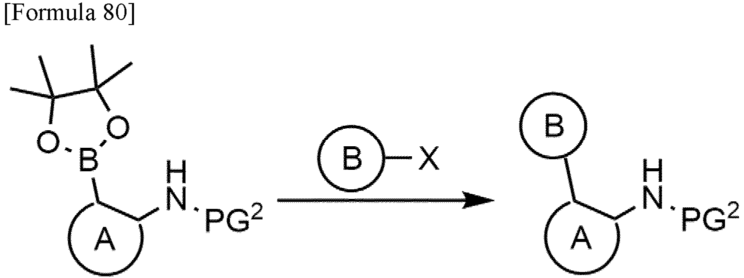

**[Table 3-1]**

| Reference Example | Ring A | Ring B | PG² | X | Catalyst | Base | ESI/MS (m/z) |
|---|---|---|---|---|---|---|---|
| 19 | 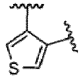 | 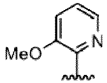 | Boc | Br | Pd(dppt)Cl₂ | K₂CO₃ | 307 (M+H)⁺ |
| 20 | 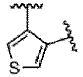 | 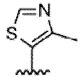 | Boc | Br | Pd(dppf)Cl₂ | K₂CO₃ | 297 (M+H)⁺ |
| 21 | 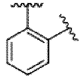 | 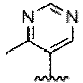 | H | Br | Pd(dppf)Cl₂ | K₂CO₃ | 186 (M+H)⁺ |
| 22 | 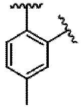 | 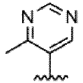 | H | Br | Pd(dppf)Cl₂ | K₃PO₄ • 3H₂O | 200 (M+H)⁺ |
| 23 | 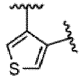 | 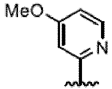 | Boc | Br | Pd(dppf)Cl₂ | K₂CO₃ | 307 (M+H)⁺ |
| 24 | 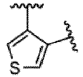 | 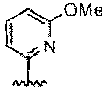 | Boc | Br | Pd(dppf)Cl₂ | K₂CO₃ | 307 (M+H)⁺ |
| 25 | 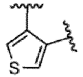 | 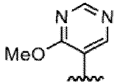 | Boc | Br | Pd(dppf)Cl₂ | K₂CO₃ | 308 (M+H)⁺ |
| 26 | 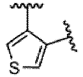 | 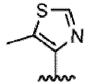 | Boc | Br | Pd(dppf)Cl₂ | K₂CO₃ | 297 (M+H)⁺ |
| 27 | 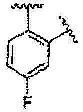 | 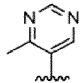 | H | Br | Pd(dppf)Cl₂ | K₃PO₄ · 3H₂O | 204 (M+H)⁺ |
| 28 | 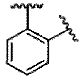 | 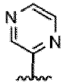 | H | Br | Pd(dppl)Cl₂ | K₂CO₃ | 172 (M+H)⁺ |
| 29 | 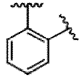 | 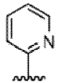 | H | Br | Pd(dppf)Cl₂ | K₂CO₃ | 171 (M+H)⁺ |

**[Table 3-2]**

| Reference Example | Ring A | Ring B | PG² | X | Catalyst | Base | ESI/MS (m/z) |
|---|---|---|---|---|---|---|---|
| 30 | 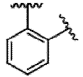 | 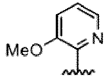 | H | Br | Pd(dppf)Cl₂ | K₂CO₃ | 201 (M+H)⁺ |
| 31 | 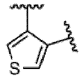 | 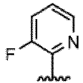 | Boc | Br | Pd(dppf)Cl₂ | K2CO3 | 295 (M+H)⁺ |
| 32 | 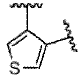 | 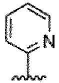 | Boc | Br | Pd(dppf)Cl₂ | K₂CO₃ | 277 (M+H)⁺ |
| 33 | 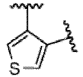 | 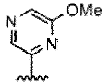 | Boc | Br | Pd(dppf)Cl₂ | K₂CO₃ | 308 (M+H)⁺ |
| 34 | 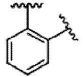 | 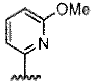 | H | Br | Pd(dppf)Cl₂ | K₂CO₃ | 201 (M+H)⁺ |
| 35 | 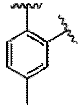 | 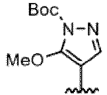 | H | I | Pd(dtbpf)Cl₂ | K₃PO₄ • 3H₂O | 304 (M+H)⁺ |
| 36 | 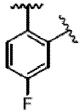 | 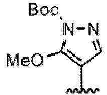 | H | I | Pd(dtbpf)Cl₂ | K₃PO₄ • 3H₂O | 308 (M+H)⁺ |

### Production of Biarylamine 4, Step III-5 (Reference Examples 37 to 56)

### [Reference Example 37]

### [2,3'-Bithiophen]-4'-amine

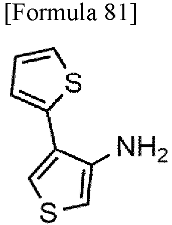

t-Butyl [2,3'-bithiophen]-4'-ylcarbamate (Reference Example 1, 211 mg) was dissolved in chloroform (4 mL), and to the mixture was added trifluoroacetic acid (4 mL), and stirred at room temperature for 1.5 hours. The reaction solution was concentrated under reduced pressure, basified with saturated sodium bicarbonate aqueous solution, and then extracted with ethyl acetate. The organic layer was washed with saturated saline, and then dried over anhydrous sodium sulfate. The anhydrous sodium sulfate was filtered, and the residue was then washed with ethyl acetate. The filtrate and washing were combined and concentrated under reduced pressure to yield the title compound (137 mg).

### ESI/MS(m/z) 182 (M+H)⁺.

Referring to the method of Reference Example 37, compounds were synthesized according to the following reaction scheme. The synthesized compounds and their data are shown in Table 4.

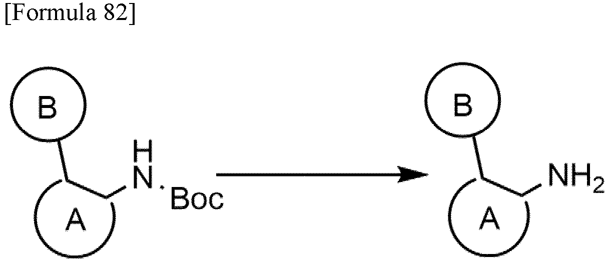

**[Table 4-1]**

| Reference Example | Starting material | Ring A | Ring B | Acid | Solvent | ESI/MS (m/z) |
|---|---|---|---|---|---|---|
| 38 | Reference Example 2 | 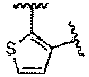 | 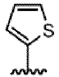 | TFA | CHCl₃ | 182 (M+H)⁺ |
| 39 | Reference Example 4 | 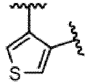 | 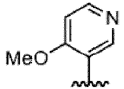 | HCl | AcOEt | 207 (M+H)⁺ |
| 40 | Reference Example 5 | 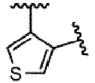 | 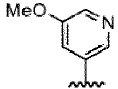 | HCl | AcOEt | 207 (M+H)⁺ |
| 41 | Reference Example 6 | 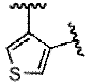 | 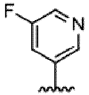 | HCl | AcOEt | 195 (M+H)⁺ |
| 42 | Reference Example 7 | 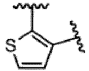 | 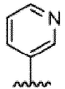 | TFA | CHCl₃ | 177 (M+H)⁺ |
| 43 | Reference Example 8 | 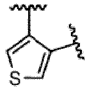 | 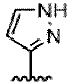 | TFA | CHCl₃ | 166 (M+H)⁺ |
| 44 | Reference Example 12 | 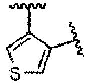 | 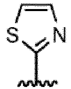 | TFA | CH₂Cl₂ | 183 (M+H)⁺ |
| 45 | Reference Example 13 | 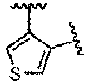 | 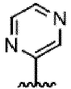 | HCl | AcOEt | 178 (M+H)⁺ |
| 46 | Reference Example 14 | 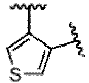 | 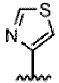 | TFA | CH₂Cl₂ | 183 (M+H)⁺ |
| 47 | Reference Example 17 | 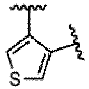 | 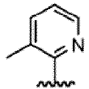 | TFA | CH₂Cl₂ | 191 (M+H)⁺ |
| 48 | Reference Example 19 | 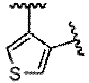 | 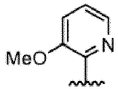 | TFA | CH₂Cl₂ | 207 (M+H)⁺ |
| 49 | Reference Example 20 | 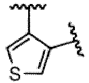 | 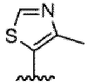 | TFA | CH₂Cl₂ | 197 (M+H)⁺ |

**[Table 4-2]**

| Reference Example | Starting material | Ring A | Ring B | Acid | Solvent | ESI/MS (m/z) |
|---|---|---|---|---|---|---|
| 50 | Reference Example 23 | 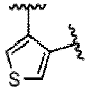 | 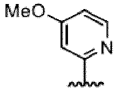 | TFA | CH₂Cl₂ | 207 (M+H)⁺ |
| 51 | Reference Example 24 | 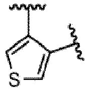 | 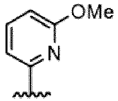 | TFA | CH₂Cl₂ | 207 (M+H)⁺ |
| 52 | Reference Example 25 | 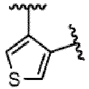 | 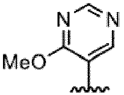 | TFA | CH₂Cl₂ | 208 (M+H)⁺ |
| 53 | Reference Example 26 | 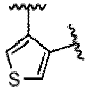 | 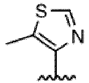 | TFA | CH₂Cl₂ | 197 (M+H)⁺ |
| 54 | Reference Example 31 | 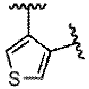 | 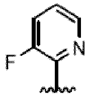 | TFA | CH₂Cl₂ | 195 (M+H)⁺ |
| 55 | Reference Example 32 | 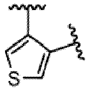 | 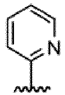 | TFA | CH₂Cl₂ | 177 (M+H)⁺ |
| 56 | Reference Example 33 | 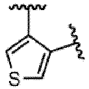 | 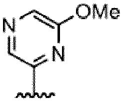 | TFA | CH₂Cl₂ | 208 (M+H)⁺ |

### Production of Present Compound, Steps I-1 and 2 (Examples 1 to 34)

### [Example 1]

### N-((1H-Imidazol-4-yl)methyl)[2,3'-bithiophen]-4'-amine

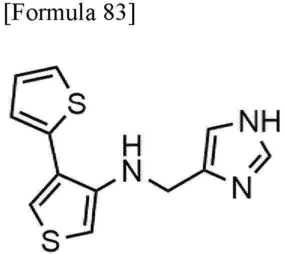

[2,3'-Bithiophen]-4'-amine (Reference Example 37, 20.0 mg) and 4-formylimidazole (21.2 mg) were dissolved in N,N-dimethylformamide (550 µL), and the mixture was stirred overnight at 70°C. After cooling to room temperature, to the mixture were added methanol (550 µL) and sodium borohydride (10.5 mg) and stirred at room temperature for 30 minutes. The reaction solution was added with water and after stirring for 30 minutes, the mixture was extracted with ethyl acetate. The organic layer was washed with saturated saline, and then dried over anhydrous sodium sulfate. The anhydrous sodium sulfate was filtered, and the residue was then washed with ethyl acetate. The filtrate and washing were combined and concentrated under reduced pressure. The resulting residue was purified with preparative thin-layer chromatography (NH silica; chloroform/methanol = 10/1) to yield the title compound (13.7 mg).
¹H-NMR (400 MHz, CD₃OD) δ 7.61 (d, J = 1.2 Hz, 1H), 7.35 (dd, J = 5.2, 1.1 Hz, 1H), 7.27 (d, J = 3.3 Hz, 1H), 7.22 (dd, J = 3.5, 1.1 Hz, 1H), 7.08 (dd, J = 5.2, 3.5 Hz, 1H), 7.01 (brs, 1H) 6.21 (d, J = 3.3 Hz, 1H), 4.24 (s, 2H).
ESI/MS(m/z) 262 (M+H)⁺.

Referring to the method of Example 1, compounds were synthesized according to the following reaction scheme. The synthesized compounds and their data are shown in Table 5 and Table 6, respectively.

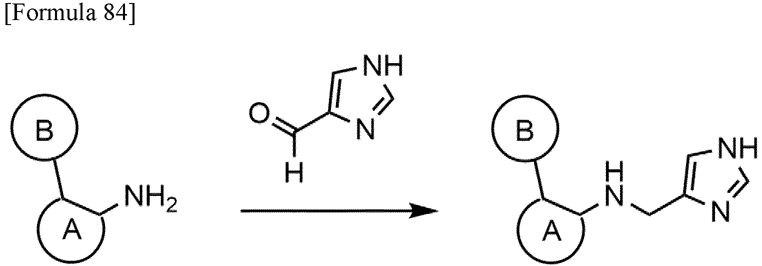

**[Table 5-1]**

| Example | Starting material | Ring A | Ring B | Reductant | Additive | Solvent |
|---|---|---|---|---|---|---|
| 2 | Reference Example 38 | 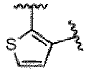 | 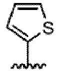 | NaBH₄ | none | DMA/MeOH |
| 3 | Reference Example 3 | 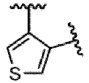 | 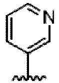 | NaBH₄ | none | THF/EtOH |
| 4 | Reference Example 39 | 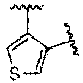 | 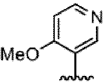 | NaBH₄ | none | THF/EtOH |
| 5 | Reference Example 40 | 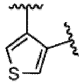 | 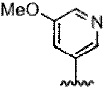 | NaBH₃CN | Et₃N | MeOH |
| 6 | Reference Example 41 | 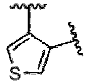 | 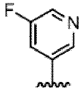 | NaBH₃CN | Et₃N | MeOH |
| 7 | Reference Example 42 | 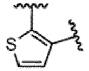 | 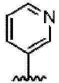 | NaBH(OAc)₃ | Ti(O*i*Pr)₄ | CH₂Cl₂ |
| 8 | Reference Example 43 | 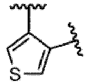 | 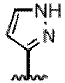 | NaBH₄ | Ti(O*i*Pr)₄ | CH₂Cl₂/MeOH |
| 9 | Reference Example 9 | 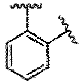 | 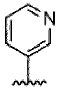 | NaBH₃CN | AcOH | MeOH |
| 10 | Reference Example 10 | 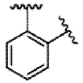 | 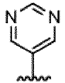 | NaBH₃CN | AcOH | MeOH |
| 11 | Reference Example 11 | 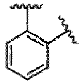 | 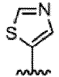 | NaBH₃CN | AcOH | MeOH |
| 12 | Reference Example 44 | 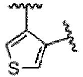 | 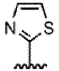 | NaBH₄ | Ti(O*i*Pr)₄ | CH₂Cl₂/MeOH |
| 13 | Reference Example 45 | 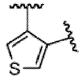 | 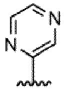 | NaBH₃CN | Et₃N | MeOH |

**[Table 5-2]**

| Example | Starting material | Ring A | Ring B | Reductant | Additive | Solvent |
|---|---|---|---|---|---|---|
| 14 | Reference Example 46 | 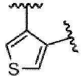 | 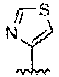 | NaBH₃CN | none | MeOH |
| 15 | Reference Example 15 | 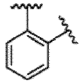 | 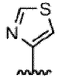 | NaBH₃CN | AcOH | MeOH |
| 16 | Reference Example 47 | 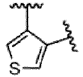 | 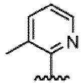 | NaBH₄ | TFA | MeOH |
| 17 | Reference Example 48 | 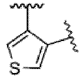 | 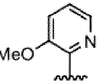 | NaBH₄ | TFA | MeOH |
| 18 | Reference Example 49 | 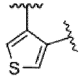 | 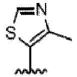 | NaBH₄ | Ti(O*i*Pr)₄ | CH₂Cl₂/MeOH |
| 19 | Reference Example 21 | 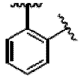 | 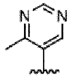 | NaBH₃CN | AcOH | MeOH |
| 20 | Reference Example 22 | 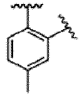 | 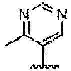 | NaBH₃CN | AcOH | MeOH |
| 21 | Reference Example 50 | 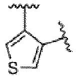 | 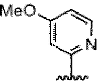 | NaBH₄ | Ti(O*i*Pr)₄ | CH₂Cl₂/MeOH |
| 22 | Reference Example 51 | 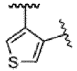 | 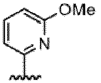 | NaBH₄ | TFA | MeOH |
| 23 | Reference Example 52 | 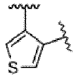 | 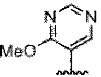 | NaBH₄ | Ti(O*i*Pr)₄ | CH₂Cl₂/MeOH |
| 24 | Reference Example 53 | 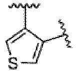 | 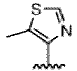 | NaBH₄ | Ti(O*i*Pr)₄ | CH₂Cl₂/MeOH |
| 2 5 | Reference Example 27 | 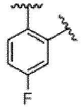 | 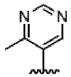 | NaBH₃CN | AcOH | MeOH |

**[Table 5-3]**

| Example | Starting material | Ring A | Ring B | Reductant | Additive | Solvent |
|---|---|---|---|---|---|---|
| 26 | Reference Example 28 | 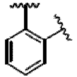 | 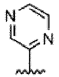 | NaBH₃CN | AcOH | MeOH |
| 27 | Reference Example 29 | 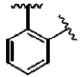 | 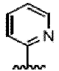 | NaBH₃CN | AcOH | MeOH |
| 28 | Reference Example 30 | 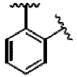 | 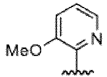 | NaBH₃CN | AcOH | MeOH |
| 29 | Reference Example 54 | 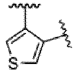 | 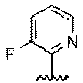 | NaBH₄ | TFA | CH₂Cl₂/MeOH |
| 30 | Reference Example 55 | 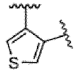 | 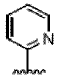 | NaBH₄ | TFA | CH₂Cl₂/MeOH |
| 31 | Reference Example 56 | 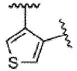 | 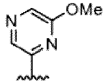 | NaBH₄ | TFA | CH₂Cl₂/MeOH |
| 32 | Reference Example 34 | 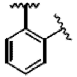 | 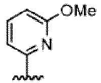 | NaBH₃CN | AcOH | MeOH |

**[Table 6-1]**

| Example | ¹H-NMR | ESI/MS (m/z) |
|---|---|---|
| 2 | ¹H-NMR (400MHz, CD₃OD) δ 7.59 (d. *J =* 1.2 Hz. 1H), 7.30 (dd, *J* = 4.8, 1.5 Hz, 1H), 7.18 (d, *J =* 5.5 Hz. 1H), 7.08-7,04 (m, 2H). 6.93 (brs. 1H), 6.84 (d, *J =* 5.5 Hz. 1H), 4.31 (s, 2H). | 262 (M+H)⁺ |
| 3 | ¹H-NMR (400MHz, CD₃OD) δ 8.69 (dd, *J* = 2.3. 0.9 Hz. 1H), 8.47 (dd, *J* = 4.9, 1.6 Hz, 1H), 8.02 (ddd, *J* = 7.9, 2.3, 1.6 Hz. 1H). 7.60 (d, *J =* 1.2 Hz. 1H), 7.47 (ddd, *J* = 7.9, 4.9, 0.9 Hz. 1H), 7.34 (d, *J =* 3.3 Hz. 1H). 6.99 (s, 1H). 6.25 (d. *J =* 3.3 Hz. 1H), 4.21 (s. 2H). | 257 (M+H)⁺ |
| 4 | ¹H-NMR (400 MHz, DMSO-*d*₆) δ 8.45 (d. *J =* 7.6 Hz. 1H), 8.29 (s. 1H), 7.73 (s. 1H), 7.33 (d. *J =* 4.0 Hz. 1H), 7.15 (d. *J* = 8.0 Hz. 1H), 7.02 (s. 1H), 6.20 (d, *J* = 4.4 Hz, 1H), 4.71 (t, *J* = 8.0 Hz, 1H), 4.08 (d, *J =* 6.8 Hz. 2H), 3.85 (s. 3H). | 287 (M+H)⁺ |
| 5 | ¹H-NMR (400 MHz, DMSO-*d*₆) δ 11.86 (brs, 1H). 8.40-8.20 (m, 2H). 7.60-7.45 (m, 3H), 7.00 (brs, 1H). 6.26 (d, *J* = 3.6 Hz. 1H). 5.00 (brs. 1H). 4.09 (brs, 2H). 3.87 (s, 3H). | 287 (M+H)⁺ |
| 6 | ¹H-NMR (400 MHz, CD₃OD) δ 8.62 (s, 1H), 8.42 (d. *J* = 2.8 Hz. 1H), 7.95-7.84 (m, 1H), 7.64 (s. 1H), 7.45 (d. *J =* 3.6 Hz. 1H), 7.03 (s. 1H), 6.30 (d, *J =* 3.2 Hz. 1H), 4.25 (s, 2H). | 275 (M+H)⁺ |
| 7 | ¹H-NMR (400 MHz, CD₃OD) δ 8.66 (d, *J* = 1.6 Hz. 1H). 8.45-8.30 (m, 1H), 7.96 (d, *J* = 8.4 Hz. 1H), 7.60 (s, 1H), 7.44 (dd. *J* = 7.8. 5.0 Hz. 1H), 7.32 (d, *J=* 5.6 Hz. 1H), 6.92 (s, 1H). 6.89 (d, *J =* 5.6 Hz, 1H). 4.28 (s, 2H). | 257 (M+H)⁺ |
| 8 | ¹H-NMR (400 MHz, DMSO-*d₆*) δ 12.83 (s. 1H). 7.74 (brs. 1H), 7.70-7.60 (m, 2H). 7.02 (s. 1H). 6.71 (brs. 1H). 6.63 (d. *J* = 2.4 Hz. 1H). 6.09 (brs. 1H), 4.16 (s. 2H). | 246 (M+H)⁺ |
| 9 | ¹H-NMR (400 MHz, CD₃OD) δ 8.58-8.55 (m, 1H). 8.52-8.47 (m, 1H). 7.92 (d, *J* = 7.6 Hz. 1H), 7.58 (s, 1H). 7.50 (dd, *J* = 7.8. 5.0 Hz, 1H), 7.24-7.18 (m, 1H), 7.03 (d. *J* = 7.2 Hz, 1H). 6.93 (s, 1H), 6.83-6.73 (m, 2H). 4.25 (s. 2H). | 251 (M+H)⁺ |
| 10 | ¹H-NMR (400 MHz, CD₃OD) δ 9.10 (s. 1H), 8.87 (s. 2H), 7.72 (s, 1H), 7.28-7.21 (m, 1H). 7.06 (dd. *J* = 7.4. 1.4 Hz. 1H), 7.00 (s. 1H). 6.84-6.75 (m, 2H). 4.28 (s. 2H). | 252 (M+H)⁺ |
| 11 | ¹H-NMR (400 MHz, CD₃OD) δ 9.02 (s. 1H), 7.96 (s, 1H), 7.62 (s. 1H). 7.24-7.14 (m, 2H). 6.96 (s. 1H), 6.82 (d. *J =* 8.4 Hz. 1H). 6.77-6.71 (m, 1H), 4.29 (s. 2H). | 257 (M+H)⁺ |
| 1 2 | ¹H-NMR (400 MHz, DMSO-*d*₆) & 12.01 (brs. 1H). 8.02 (d. *J* = 3.2 Hz. 1H), 7.84 (d. *J* = 3.2 Hz. 1H), 7.67 (d. *J* = 3.6 Hz. 1H). 7.61 (s, 1H). 7.03 (s. 1H). 6.95 (t, *J* = 5.2 Hz. 1H). 6.22 (d, *J* = 3.6 Hz, 1H), 4.18 (d, *J* = 5.2 Hz, 2H). | 263 (M+H)⁺ |

**[Table 6-2]**

| Example | ¹H-NMR | ESI/MS (m/z) |
|---|---|---|
| 13 | ¹H-NMR (400 MHz, DMSO-*d*₆) δ 9.21 (d. *J* = 1.6 Hz, 1H), 8.59-8.56 (m, 1H), 8.49 (d, *J* = 2.8 Hz, 1H). 8.31 (d, *J* = 3.2 Hz. 1H), 7.59 (d, *J* = 1.2 Hz, 1H), 7.17 (t, *J* = 5.0 Hz. 1H), 7.01 (brs, 1H), 6.23 (d, *J* = 3.2 Hz, 1H), 4.18 (d, *J =* 5.2 Hz. 2H). | 258 (M+H)⁺ |
| 14 | ¹H-NMR (400 MHz, DMSO-*d*₆) δ 9.21 (d, *J* = 2.0 Hz, 1H), 8.00 (d, *J* = 2.0 Hz, 1H), 7.82 (d, *J* = 3.6 Hz, 1H), 7.68 (brs, 1H), 7.04 (s, 1H), 6.65-6.50 (m, 1H), 6.18 (d, *J* = 3.6 Hz, 1H), 4.18 (brs ,2H). | 263 (M+H)⁺ |
| 1 5 | ¹H-NMR (400 MHz, CD₃OD) δ 9.04 (d, *J* = 1.6 Hz, 1H), 7.70 (d, *J* = 1.6 Hz, 1H), 7.62 (s, 1H), 7.51 (dd, *J* = 7.8, 1.4 Hz, 1H), 7.23-7.13 (m, 1H), 6.98 (s, 1H), 6.81 (d, *J* = 8.0 Hz, 1H), 6.73-6.64 (m, 1H), 4.35 (s. 2H). | 257 (M+H)⁺ |
| 16 | ¹H-NMR (400 MHz, DMSO-*d*₆) δ 8.45 (d, *J* = 4.4 Hz, 1H), 7.77 (d, *J* = 10.4 Hz. 1H), 7.65 (d, *J* = 4.0 Hz, 1H), 7.58 (s, 1H), 7.29-7.25 (m, 1H), 6.99 (brs, 1H), 6.24 (d, *J* = 4*.*0 Hz, 1H), 6.01 (brs, 1H), 4.10 (s, 2H), 2.42 (s, 3H). | 271 (M+H)⁺ |
| 17 | ¹H-NMR (400 MHz, DMSO-*d*₆) δ 11.96 (brs, 1H), 8.15 (dd, *J* = 4.8, 1.2 Hz, 1H), 8.08 (d, *J =* 3.6 Hz, 1H), 7.59 (d, *J* = 1.2 Hz, 1H), 7.56 (dd, *J* = 8.6, 1.0 Hz. 1H), 7.33 (brs, 1H), 7.30 (dd, *J* = 8.4, 4.8 Hz, 1H) 6.95 (s, 1H), 6.11 (d. *J* = 3.6 Hz, 1H), 4.14 (s, 2H), 3.89 (s, 3H). | 287 (M+H)⁺ |
| 18 | ¹H-NMR (400 MHz, DMSO-*d*₆) δ 9.02 (s, 1H), 7.56 (s, 1H), 7.45 (d, *J* = 3.2 Hz. 1H), 6.94 (s, 1H), 6.24 (d, *J =* 3.6 Hz, 1H), 4.72 (t. *J =* 5.6 Hz, 1H), 4.09 (d, *J* = 5.6 Hz, 2H), 2.32 (s, 3H). | 277 (M+H)⁺ |
| 19 | ¹H-NMR (400 MHz, DMSO-*d*₆) δ 11.87 (brs, 1H), 9.04 (s, 1H), 8.47 (s, 1H), 7.52 (brs, 14), 7.25-7.15 (m, 1H), 6.93 (dd, *J* = 7.6, 1.6 Hz, 1H), 6.85 (brs, 1H), 6.76 (d, *J* = 8.0 Hz. 1H), 6.70-6.60 (m, 1H), 4.98 (brs, 1H), 4.14 (brs, 2H), 2.28 (s, 3H). | 266 (M+H)⁺ |
| 20 | ¹H-NMR (400 MHz, CD₃OD) δ 8.97 (s. 1H). 8.47 (s, 1H), 7.58 (s. 1H), 6.89 (s, 1H), 6.82 (d, *J* = 7.6 Hz, 1H), 6.65 (s, 1H), 6.60 (d, *J* = 7.6 Hz, 1H), 4.24 (s. 2H), 2.36 (s, 3H), 2.30 (s, 3H). | 280 (M+H)⁺ |
| 21 | ¹H-NMR (400 MHz, DMSO-*d*₆) δ 11.94 (brs, 1H), 8.35 (d, *J =* 5.6 Hz, 1H), 8.11 (d, *J =* 3.2 Hz, 1H), 7.66 (brs, 1H), 7.58 (s, 1H), 7.42 (d, *J* = 2.4 Hz, 1H), 6.99 (s, 1H), 6.87 (dd, *J* = 5.8, 2.2 Hz, 1H), 6.12 (d, *J* = 3.2 Hz, 1H), 4.15 (s, 2H), 3.89 (s, 3H). | 287 (M+H)⁺ |
| 22 | ¹H-NMR (400 MHz, DMSO-*d*₆) δ 11.98 (brs, 1H), 8.03 (d, *J* = 3.2 Hz, 1H), 7.77-7.70 (m, 1H). 7.59 (s, 1H), 7.46 (d, *J* = 7.6 Hz, 1H), 7.32 (t, *J* = 5.2 Hz, 1H), 7.05 (s, 1H), 6.68 (d. *J* = 8.0 Hz, 1H), 6.17 (d, *J* = 3.2 Hz. 1H), 4.13 (d, *J =* 5.2 Hz, 2H), 3.66 (s. 3H). | 287 (M+H)⁺ |
| 23 | ¹H-NMR (400 MHz, DMSO-*d₆*) δ 11.88 (brs, 1H), 8.78 (s, 1H), 8.48 (s, 1H), 7.56 (s, 1H), 7.43 (d, *J =* 3.2 Hz, 1H), 6.93 (s, 1H), 6.20 (d, *J =* 3.2 Hz, 1H), 4,97 (t, *J =* 5.6 Hz. 1H), 4.06 (d, *J =* 5.6 Hz, 2H), 3.95 (s, 3H). | 288 (M+H)⁺ |

**[Table 6-3]**

| Example | ¹H-NMR | ESI/MS (m/z) |
|---|---|---|
| 24 | ¹H-NMR (400 MHz, DMSO-*d*₆) δ 8.98 (s, 1H), 7.60-7.53 (m, 2H), 6.99 (s, 1H), 6.20 (d, *J* = 3.6 Hz, 1H), 6.03 (t, *J =* 5.2 Hz. 1H), 4.10 (d, *J* = 4.8 Hz. 2H), 2.55 (s, 3H). | 277 (M+H)⁺ |
| 25 | ¹H-NMR (400 MHz, CD₃OD) δ 9.00 (s, 1H), 8.49 (s, 1H), 7.60 (d, *J* = 1.2 Hz, 1H), 6.97-6.90 (m, 2H), 6.51 (dd, *J* = 12.0, 2.6 Hz, 1H), 6,49-6.42 (m, 1H), 4.24 (s, 2H). 2.36 (s, 3H). | 284 (M+H)⁺ |
| 26 | ¹H-NMR (400 MHz, CD₃OD) δ 9.01 (d, *J =* 1.2 Hz, 1H), 8.58-8.53 (m, 1H), 8.41 (d, *J* = 2.8 Hz, 1H), 7.67 (dd, *J* = 7.8, 1.8 Hz, 1H), 7.66-7.58 (m, 1H), 7.31-7.23 (m, 1H), 7.00-6.95 (m, 1H), 6.85 (d, *J =* 8.0 Hz. 1H), 6.79-6.71 (m, 1H), 4.38 (s, 2H). | 252 (M+H)⁺ |
| 27 | ¹H-NMR (400 MHz, CD₃OD) δ 8.52 (d, *J* = 4.0 Hz, 1H), 7.88-7.80 (m, 1H), 7.71 (d, *J =* 8.0 Hz, 1H), 7.59 (s, 1H), 7.48 (d, *J* = 7.6 Hz, 1H), 7.28-7.16 (m, 2H), 6.96 (s, 1H), 6.80 (d, *J* = 8.0 Hz, 1H), 6.76-6.68 (m, 1H), 4.34 (s, 2H). | 251 (M+H)⁺ |
| 28 | ¹H-NMR (400 MHz, CD₃OD) δ 8.16 (dd, *J* = 4.8, 1.2 Hz, 1H), 7.58-7.52 (m, 2H), 7.38 (dd, *J* = 8.4, 4.8 Hz, 1H), 7.24-7.12 (m, 2H), 6.92-6.88 (m, 1H), 6.79 (d, *J =* 8.0 Hz, 1H), 6.74-6.68 (m, 1H), 4.27 (s, 2H), 3.80 (s, 3H). | 281 (M+H)⁺ |
| 29 | ¹H-NMR (400 MHz, DMSO-*d*₆) δ 11.99 (brs, 1H), 8.46-8.41 (m, 1H), 7.99-7.94 (m, 1H), 7.91-7.83 (m, 1H), 7.59 (s, 1H), 7.44-7.37 (m, 1H), 7.25 (t, *J* = 5.2 Hz, 1H), 7.00 (s, 1H), 6.23-6.19 (m, 1H), 4.16 (d, *J =* 5.2 Hz. 2H). | 275 (M+H)⁺ |
| 30 | ¹H-NMR (400 MHz, DMSO-*d*₆) δ 11.95 (brs, 1H), 8.52 (d, *J* = 4.4 Hz, 1H), 8.07 (d, *J =* 3.6 Hz. 1H), 7.92-7.81 (m, 2H), 7.63-7.56 (m, 2H), 7.29-7.22 (m. 1H), 7.00 (s, 1H), 6.15 (d, *J =* 3.2 Hz. 1H), 4.17 (d, *J* = 4.0 Hz, 2H). | 257 (M+H)⁺ |
| 31 | ¹H-NMR (400 MHz, DMSO-*d*₆) δ 11.91 (brs, 1H), 8.74 (s, 1H), 8.24 (d, *J =* 3.2 Hz, 1H), 8.15 (s, 1H), 7.59 (s, 1H), 7.15-6.85 (m, 2H), 6.24 (brs, 1H), 4.13 (brs, 2H), 3.82 (brs, 3H). | 288 (M+H)⁺ |
| 32 | ¹H-NMR (400 MHz, CD₃OD) δ 7,73-7.65 (m, 1H), 7.63 (s, 1H), 7.56 (d, *J =* 7.6 Hz, 1H), 7.28 (d, *J* = 7.6 Hz, 1H), 7.27-7.19 (m, 1H), 7.03 (s, 1H), 6.84 (d, *J =* 8.0 Hz, 1H), 6.75-6.67 (m, 1H), 6.62 (d, *J =* 8.0 Hz, 1H), 4.33 (s, 2H), 3.58 (s, 3H). | 281 (M+H)⁺ |

### [Example 33]

### N-((1H-Imidazol-4-yl)methyl)-2-(3-methoxy-1H-pyrazol-4-yl)-5-methylaniline

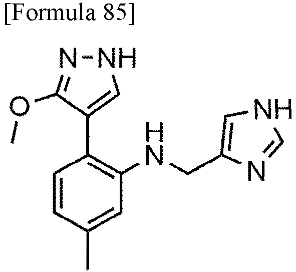

t-Butyl 4-(2-amino-4-methylphenyl)-5-methoxy-1H-pyrazole-1-carboxylate (Reference Example 35, 117 mg), 4-formylimidazole (111 mg), and acetic acid (0.1 mL) were dissolved in methanol (8 mL), and the mixture was stirred at 50°C for 48 hours. After cooling to room temperature, the mixture was added sodium cyanoborohydride (109 mg) and stirred at 50°C for 16 hours. The reaction solution was concentrated under reduced pressure, added with water, and extracted with ethyl acetate, and the organic layer was dried over anhydrous sodium sulfate. The anhydrous sodium sulfate was filtered, and the residue was then washed with ethyl acetate. The filtrate and washing were combined and concentrated under reduced pressure, the resulting residue was dissolved in dichloromethane (10 mL), added with trifluoroacetic acid (2 mL), and the mixture was then stirred at room temperature for 2 hours. The reaction solution was concentrated under reduced pressure, and the resulting residue was purified with preparative high performance liquid chromatography (ammonium bicarbonate water/acetonitrile = 80/20 to 30/70) to yield the title compound (16 mg).
¹H-NMR (400 MHz, CD₃OD) δ 7.60 (s, 1H), 7.53 (s, 1H), 6.99 (d, J = 7.2 Hz, 1H), 6.95 (s, 1H), 6.59 (s, 1H), 6.51 (d, J = 7.6 Hz, 1H), 4.25 (s, 2H), 3.85 (s, 3H), 2.26 (s, 3H).
ESI/MS(m/z) 284 (M+H)⁺.

Referring to the method of Example 33, a compound was synthesized according to the following reaction scheme. The data is shown in Table 7.

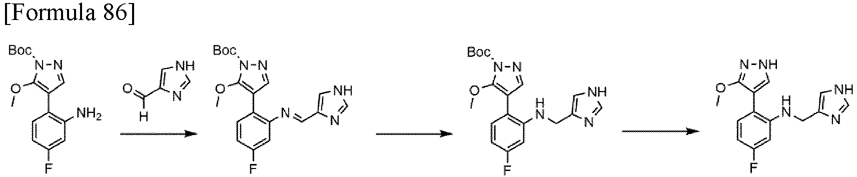

**[Table 7]**

| Example | ¹H-NMR | ESI/MS (m/z) |
|---|---|---|
| 34 | ¹H-NMR (400 MHz, CD₃OD) δ 7.63 (s, 1H), 7.56 (s, 1H), 7.09-7.01 (m, 1H), 6.98 (s, 1H), 6.42 (dd, *J* = 12.0, 2.4 Hz, 1H), 6.38-6.30 (m, 1H), 4.25 (s, 2H), 3.86 (s, 3H). | 288 (M+H)⁺ |

### Production of Present Compound, Steps II-1 to 3 (Examples 35 to 40, Reference Examples 57 to 59)

### [Example 35]

### N-(1-(1H-Imidazol-4-yl)ethyl)-2-(pyridin-3-yl)aniline

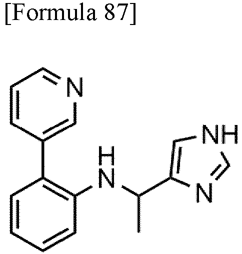

2-(Pyridin-3-yl)aniline (Reference Example 9, 34 mg), 1-trityl-1H-imidazole-4-carbaldehyde (81 mg), and molecular sieves 3A (90 mg) were suspended in toluene (2 mL), and the mixture was heated to reflux for 1 day. The reaction solution was hot filtered, and the residue was washed with toluene. The filtrate and washing were combined and concentrated under reduced pressure, and the resulting residue was dissolved in tetrahydrofuran (2 mL). After cooling to -78°C, the mixture was added dropwise with 1M solution of methylmagnesium bromide in tetrahydrofuran (500 µL), and warmed to room temperature. After stirring for 1 hour, the mixture was added with saturated ammonium chloride aqueous solution to extract with ethyl acetate. The organic layer was washed with water and saturated saline, and then dried over anhydrous sodium sulfate. The anhydrous sodium sulfate was filtered, and the residue was then washed with ethyl acetate. The filtrate and washing were combined and concentrated under reduced pressure, and the resulting residue was purified with preparative thin-layer chromatography (hexane/ethyl acetate = 1/1) to yield 2-(pyridin-3-yl)-N-(1-(1-trityl-1H-imidazol-4-yl)ethyl)aniline (52 mg).
ESI/MS(m/z) 507 (M+H)⁺.

Thus obtained 2-(pyridin-3-yl)-N-(1-(1-trityl-1H-imidazol-4-yl)ethyl)aniline (52 mg) was dissolved in formic acid (2 mL), and the mixture was stirred overnight at room temperature. The reaction solution was washed with hexane, concentrated under reduced pressure, and then azeotroped with chloroform. The resulting residue was dissolved in tetrahydrofuran (2 mL), added with potassium carbonate (200 mg), and stirred at room temperature for 30 minutes. Potassium carbonate was filtered, and the residue was then washed with tetrahydrofuran. The filtrate and washing were combined and concentrated under reduced pressure, and the resulting residue was purified with preparative thin-layer chromatography (methanol/chloroform = 1/9) to yield the title compound (20 mg).
¹H-NMR (400 MHz, DMSO-d₆) δ 11.82 (s, 1H), 8.58 (s, 1H), 8.55 (dd, J = 4.8, 1.6 Hz, 1H), 7.86 (dt, J = 7.9, 1.2 Hz, 1H), 7.51 (s, 1H), 7.49 (ddd, J = 7.9, 4.8, 1.2 Hz, 1H), 7.16 (t, J = 7.1 Hz, 1H), 7.00 (dd, J = 7.1, 1.5 Hz, 1H), 6.91 (s, 1H), 6.77 (d, J = 8.1 Hz, 1H), 6.69 (t, J = 7.1 Hz, 1H), 4.56-4.52 (m, 2H), 1.34 (d, J = 5.0 Hz, 3H).
ESI/MS(m/z) 265 (M+H)⁺.

### [Example 36]

### (+)-N-(1-(1H-Imidazol-4-yl)ethyl)-2-(pyridin-3-yl)aniline, and

### [Reference Example 57]

### (-)-N-(1-(1H-Imidazol-4-yl)ethyl)-2-(pyridin-3-yl)aniline

(N-(1-(1H-imidazol-4-yl)ethyl)-2-(pyridin-3-yl)aniline), the compound produced in Example 35 was subjected to optical resolution using high performance liquid chromatography (CHIRALPAK IC; hexane/ethanol/diethylamine = 900/100/1) to yield the title compounds.
Example 36: ¹H-NMR (400 MHz, CD₃OD) δ 8.57 (dd, J = 2.2, 0.9 Hz, 1H), 8.51 (dd, J = 5.0, 1.6 Hz, 1H), 7.93 (ddd, J = 7.9, 2.2, 1.6 Hz, 1H), 7.56 (d, J = 1.2 Hz, 1H), 7.51 (ddd, J = 7.9, 5.0, 0.9 Hz, 1H), 7.17 (ddd, J = 8.2, 7.4, 1.6 Hz, 1H), 7.02 (dd, J = 7.4, 1.6 Hz, 1H), 6.87 (s, 1H), 6.80-6.72 (m, 2H), 4.61 (q, J = 6.7 Hz, 1H), 1.43 (d, J = 6.7 Hz, 3H).
ESI/MS(m/z) 265 (M+H)⁺.
Reference Example 57: ¹H-NMR (400 MHz, CD₃OD) δ 8.57 (dd, J = 2.3, 0.9 Hz, 1H), 8.51 (dd, J = 4.9, 1.7 Hz, 1H), 7.93 (ddd, J = 7.9, 2.3, 1.7 Hz, 1H), 7.56 (d, J = 1.2 Hz, 1H), 7.51 (ddd, J = 7.9, 4.9, 0.9 Hz, 1H), 7.17 (ddd, J = 8.3, 7.4, 1.6 Hz, 1H), 7.02 (dd, J = 7.4, 1.6 Hz, 1H), 6.87 (s, 1H), 6.81-6.71 (m, 2H), 4.61 (q, J = 6.7 Hz, 1H), 1.43 (d, J = 6.7 Hz, 3H).
ESI/MS(m/z) 265 (M+H)⁺.

Referring to the method of Example 35, compounds were synthesized according to the following reaction scheme. The synthesized compounds and their data are shown in Table 8 and Table 9, respectively.

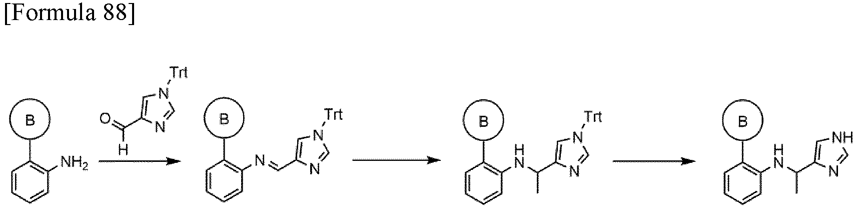

**[Table 8]**

| Example | Ring B |
|---|---|
| 37 | 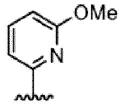 |
| 38 | 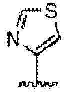 |

**[Table 9]**

| Example | ¹H-NMR | ESI/MS (m/z) |
|---|---|---|
| 37 | ¹H-NMR (400 MHz, CDCl₃) δ 9.55 (brs, 1H), 7.98 (d, *J =* 6.4 Hz, 1H), 7.66 (dd. *J =* 8.3, 7.6 Hz, 1H), 7.51-7.47 (m, 2H), 7.26 (s, 1H), 7.23 (dd, *J* = 7.6, 0.7 Hz, 1H), 7.17 (ddd, *J =* 8.3, 7.3, 1.6 Hz, 1H), 6.86 (s, 1H), 6.73-6.68 (m, 2H), 6.65 (dd, *J =* 8.3, 0.7 Hz, 1H), 4.76 (quint, *J =* 6.5 Hz, 1H), 3.65 (s, 3H), 1.60 (d, *J* = 6.6 Hz, 3H). | 295 (M+H)⁺ |
| 38 | ¹H-NMR (400 MHz. CD₃OD) δ 9.11 (d, *J* = 2.0 Hz, 1H), 7.74 (d, *J =* 2.0 Hz, 1H), 7.63-7.56 (m, 1H), 7.53 (dd, *J* = 7.7, 1.5 Hz, 1H), 7.17-7.08 (m, 1H), 6.89 (s, 1H), 6.73 (d, *J* = 8.2 Hz, 1H), 6.71 - 6.64 (m, 1H), 4.71 (q, *J =* 6.7 Hz, 1H), 1.57 (d, *J =* 6.7 Hz, 3H). | 271 (M+H)⁺ |

### [Example 39]

### (+)-N-(1-(1H-Imidazol-4-yl)ethyl)-2-(6-methoxypyridin-2-yl)aniline, and

### [Reference Example 58]

### (-)-N-(1-(1H-Imidazol-4-yl)ethyl)-2-(6-methoxypyridin-2-yl)aniline

(N-(1-(1H-imidazol-4-yl)ethyl)-2-(6-methoxypyridin-2-yl)aniline), the compound produced in Example 37 was subjected to optical resolution using high performance liquid chromatography (CHIRALPAK IA; hexane/ethanol/diethylamine = 950/50/1) to yield the title compounds.
Example 39: ¹H-NMR (400 MHz, CDCl₃) δ 9.55 (brs, 1H), 7.98 (d, J = 6.4 Hz, 1H), 7.66 (dd, J = 8.3, 7.6 Hz, 1H), 7.51-7.47 (m, 2H), 7.26 (s, 1H), 7.23 (dd, J = 7.6, 0.7 Hz, 1H), 7.17 (ddd, J = 8.3, 7.3, 1.6 Hz, 1H), 6.86 (s, 1H), 6.73-6.68 (m, 2H), 6.65 (dd, J = 8.3, 0.7 Hz, 1H), 4.76 (quint, J = 6.5 Hz, 1H), 3.65 (s, 3H), 1.60 (d, J = 6.6 Hz, 3H). ESI/MS(m/z) 295 (M+H)⁺.
Reference Example 58: ¹H-NMR (400 MHz, CDCl₃) δ 9.55 (brs, 1H), 7.98 (d, J = 6.4 Hz, 1H), 7.66 (dd, J = 8.3, 7.6 Hz, 1H), 7.51-7.47 (m, 2H), 7.26 (s, 1H), 7.23 (dd, J = 7.6, 0.7 Hz, 1H), 7.17 (ddd, J = 8.3, 7.3, 1.6 Hz, 1H), 6.86 (s, 1H), 6.73-6.68 (m, 2H), 6.65 (dd, J = 8.3, 0.7 Hz, 1H), 4.76 (quint, J = 6.5 Hz, 1H), 3.65 (s, 3H), 1.60 (d, J = 6.6 Hz, 3H).
ESI/MS(m/z) 295 (M+H)⁺.

### [Example 40]

### (+)-N-(1-(1H-Imidazol-4-yl)ethyl)-2-(thiazol-4-yl)aniline, and

### [Reference Example 59]

### (-)-N-(1-(1H-Imidazol-4-yl)ethyl)-2-(thiazol-4-yl)aniline

(N-(1-(1H-imidazol-4-yl)ethyl)-2-(thiazol-4-yl)aniline), the compound produced in Example 38 was subjected to optical resolution using high performance liquid chromatography (CHIRALPAK IA; hexane/ethanol/diethylamine = 950/50/1) to yield the title compounds.
Example 40: ¹H-NMR (400 MHz, CDCl₃) δ 8.89 (d, J = 2.0 Hz, 1H), 7.50 (d, J = 1.1 Hz, 1H), 7.48-7.45 (m, 2H), 7.26 (s, 1H), 7.19-7.11 (m, 1H), 6.88 (s, 1H), 6.75-6.67 (m, 2H), 4.75 (quint, J = 6.7 Hz, 1H), 1.60 (d, J = 6.7 Hz, 3H).
ESI/MS(m/z) 271 (M+H)⁺.
Reference Example 59: ¹H-NMR (400 MHz, CDCl₃) δ 8.89 (d, J = 2.0 Hz, 1H), 7.50 (d, J = 1.1 Hz, 1H), 7.48-7.45 (m, 2H), 7.26 (s, 1H), 7.19-7.11 (m, 1H), 6.88 (s, 1H), 6.75-6.67 (m, 2H), 4.75 (quint, J = 6.7 Hz, 1H), 1.60 (d, J = 6.7 Hz, 3H). ESI/MS(m/z) 271 (M+H)⁺.

### Production of Imidazolylmethylaminoaryl, Steps IV-1 and 2 (Reference Example 60)

### [Reference Example 60]

### N-((1H-Imidazol-4-yl)methyl)-4-bromothiophen-3-amine

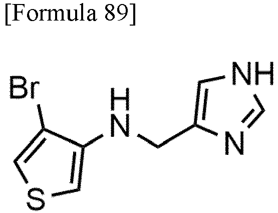

4-Bromothiophen-3-amine hydrochloride (50.0 mg) and 4-formylimidazole (44.8 mg) were dissolved in N,N-dimethylformamide (500 µL), to the mixture was added sodium triacetoxyborohydride (49.4 mg), and stirred at 70°C for 11 hours. To the mixture was additionally added sodium triacetoxyborohydride (49.4 mg) and stirred at 70°C for 5 hours. The reaction solution was added with water and saturated sodium bicarbonate, and extracted with ethyl acetate. The organic layer was washed with saturated saline, and then dried over anhydrous sodium sulfate. The anhydrous sodium sulfate was filtered, and the residue was then washed with ethyl acetate. The filtrate and washing were combined and concentrated under reduced pressure, and the resulting residue was purified with preparative thin-layer chromatography (NH silica; chloroform/methanol = 10/1) to yield the title compound (3.9 mg).
ESI/MS(m/z) 258, 260 (M+H)⁺.

### Production of Present Compound, Step IV-3 (Examples 41 to 43)

### [Example 41]

### N-((1H-Imidazol-4-yl)methyl)-4-(furan-3-yl)thiophen-3-amine

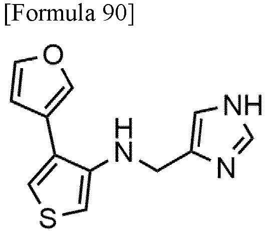

N-((1H-Imidazol-4-yl)methyl)-4-bromothiophen-3-amine (Reference Example 60, 3.9 mg) was dissolved in a mixed solvent of 1,4-dioxane (100 µL) and water (50 µL), and to the mixture were added 2-furanboronic acid (5.1 mg), tetrakis(triphenylphosphine)palladium (0.9 mg), and potassium carbonate (8.3 mg). The reaction vessel was then purged with argon gas, and the mixture was stirred overnight at 100°C. The reaction solution was filtered through Celite, and the residue was washed with ethyl acetate. The filtrate and washing were combined and concentrated under reduced pressure, and the resulting residue was purified with preparative thin-layer chromatography (NH silica; chloroform/methanol = 20/1) to yield the title compound (0.3 mg).
¹H-NMR (400 MHz, CD₃OD) δ 7.78 (dd, J = 1.7, 0.8 Hz, 1H), 7.62 (s, 1H), 7.53 (t, J = 1.7 Hz, 1H), 7.22 (d, J = 3.3 Hz, 1H), 7.01 (brs, 1H), 6.68 (dd, J = 1.9, 0.8 Hz, 1H), 6.19 (d, J = 3.3 Hz, 1H), 4.23 (s, 2H).
ESI/MS(m/z) 246 (M+H)⁺.

Referring to the method of Example 41, compounds were synthesized according to the following reaction scheme. The synthesized compounds and their data are shown in Table 10 and Table 11, respectively.

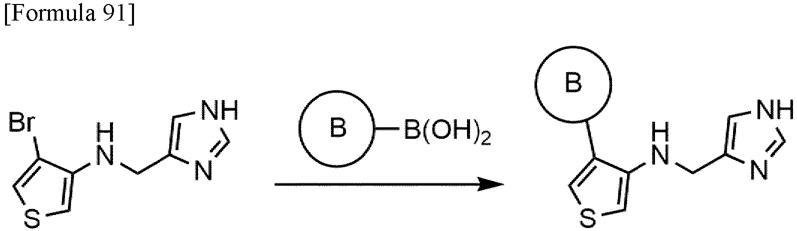

**[Table 10]**

| Example | Ring B |
|---|---|
| 42 | 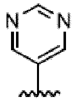 |
| 43 | 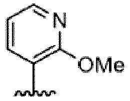 |

**[Table 11]**

| Example | ¹H-NMR | ESI/MS (m/z) |
|---|---|---|
| 42 | ¹H-NMR (400 MHz, CD₃OD) δ 9.09 (s, 1H), 9.01 (s, 2H), 7.65 (s, 1H), 7.50 (d. *J =* 3.3 Hz, 1H), 7.03 (s, 1H), 6.32 (d, *J =* 3.3 Hz, 1H), 4.24 (s, 2H). | 258 (M+H)⁺ |
| 43 | ¹H-NMR (400 MHz, CD₃OD) δ 8.14 (dd. *J* = 5.0, 1.9 Hz, 1H), 7.70 (dd, *J* = 7.3, 1.9 Hz, 1H), 7.62 (d, *J* = 1.0 Hz, 1H), 7.22 (d, *J =* 3.3 Hz. 1H), 7.03 (dd, *J =* 7.3, 5.0 Hz, 1H), 6.99 (s, 1H), 6.25 (d, *J =* 3.3 Hz, 1H), 4.22 (s, 2H), 3.93 (s, 3H). | 287 (M+H)⁺ |

### <Production of Present Compound in Which Biaryl Moiety and Imidazole Moiety are Bound Via Oxygen Atom>

### Production of Imidazolyl Methoxyaryl 1, Step V-1 (Reference Example 61)

### [Reference Example 61]

### 4-((2-Iodophenoxy)methyl)-N,N-dimethyl-1H-imidazole-1-sulfonamide

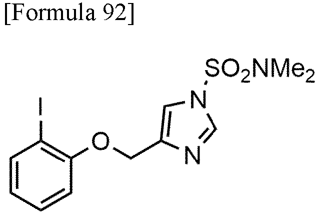

4-(Hydroxymethyl)-N,N-dimethyl-1H-imidazole-1-sulfonamide (307 mg), 2-iodophenol (363 mg), and triphenylphosphine (471 mg) were suspended in tetrahydrofuran (4 mL), added with diisopropyl azodicarboxylate (441 µL), and the mixture was stirred at room temperature for 3 hours. The reaction solution was concentrated under reduced pressure, and the resulting residue was purified with column chromatography on aminosilica gel (hexane/ethyl acetate = 2/3) and column chromatography on silica gel (hexane/ethyl acetate = 1/2) in this order to yield the title compound (350 mg).
ESI/MS(m/z) 408 (M+H)⁺.

### Production of Imidazolyl Methoxyaryl 2, Step V-2 (Reference Examples 62 to 64)

### [Reference Example 62]

### 4-((2-Bromophenoxy)methyl)-N,N-dimethyl-1H-imidazole-1-sulfonamide

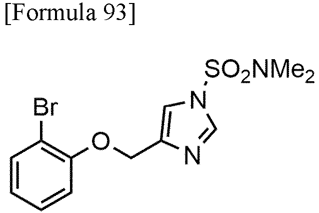

2-Bromophenol (8.7 g) and potassium carbonate (13.8 g) were suspended in dimethylsulfoxide (100 mL), added with 4-(chloromethyl)-N,N-dimethyl-1H-imidazole-1-sulfonamide (11.2 g), and the mixture was stirred at 100°C for 12 hours. The reaction solution was added with water, extracted with ethyl acetate, and the organic layer was dried over anhydrous sodium sulfate. The anhydrous sodium sulfate was filtered, and the residue was then washed with ethyl acetate. The filtrate and washing were combined and concentrated under reduced pressure, and the resulting residue was purified with column chromatography on silica gel (pentane/ethyl acetate = 5/1 to 2/1) to yield the title compound (15.1 g).
ESI/MS(m/z) 360, 362 (M+H)⁺.

Referring to the method of Reference Example 62, compounds were synthesized according to the following reaction scheme. The synthesized compounds and their data are shown in Table 12.

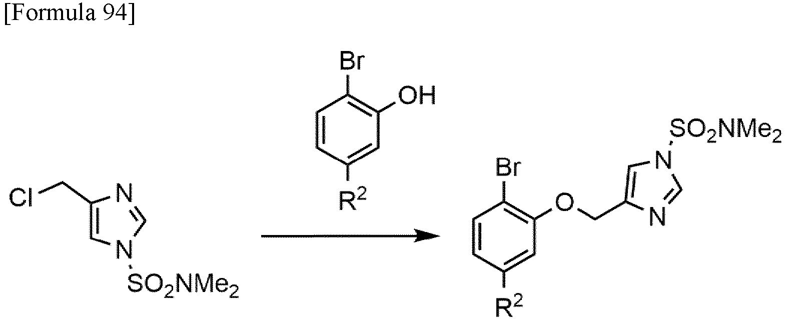

**[Table 12]**

| Reference Example | R² | ESI/MS (m/z) |
|---|---|---|
| 63 | Me | 359, 361 (M+H)⁺ |
| 64 | F | 363. 365 (M+H)⁺ |

### Production of Present Compound, Steps V-3 and 5 (Examples 44 to 65)

### [Example 44]

### 4-((2-(Thiophen-3-yl)phenoxy)methyl)-1H-imidazole

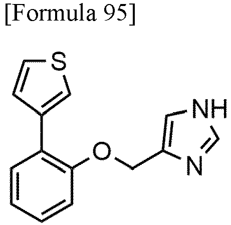

4-((2-Iodophenoxy)methyl)-N,N-dimethyl-1H-imidazole-1-sulfonamide (Reference Example 61, 61 mg) was dissolved in a mixed solvent of 1,4-dioxane (1 mL) and water (0.5 mL), to the solution were added 3-thiopheneboronic acid (21 mg), tetrakis(triphenylphosphine)palladium (17 mg), and potassium carbonate (62 mg), and the mixture was stirred at 90°C for 2.5 hours. The reaction solution was filtered through Celite, and the residue was washed with ethyl acetate. The filtrate and washing were combined and concentrated under reduced pressure, and the resulting residue was purified with column chromatography on silica gel (hexane/ethyl acetate = 1/2) to yield N,N-dimethyl-4-((2-(thiophen-3-yl)phenoxy)methyl)-1H-imidazole-1-sulfonamide (42 mg).
ESI/MS(m/z) 364 (M+H)⁺.

Thus obtained N,N-dimethyl-4-((2-(thiophen-3-yl)phenoxy)methyl)-1H-imidazole-1-sulfonamide (42 mg) was dissolved in methanol (1 mL), to the solution was added 2M hydrochloric acid methanol solution (1 mL), and the mixture was stirred at 70°C for 4 hours. The reaction solution was concentrated under reduced pressure, and the resulting residue was then purified with thin-layer chromatography (NH silica; chloroform/methanol = 10/1) to yield the title compound (27 mg).
¹H-NMR (400 MHz, CD₃OD) δ 7.70 (d, J = 0.9 Hz, 1H), 7.66 (dd, J = 3.0, 1.3 Hz, 1H), 7.53 (dd, J = 7.5, 1.7 Hz, 1H), 7.46 (dd, J = 5.1, 1.3 Hz, 1H), 7.36 (dd, J = 5.1, 3.0 Hz, 1H), 7.28 (ddd, J = 8.3, 7.5, 1.7 Hz, 1H), 7.20 (dd, J = 8.3, 1.3 Hz, 1H), 7.13 (s, 1H), 7.02 (td, J = 7.5, 1.3 Hz, 1H), 5.09 (s, 2H).
ESI/MS(m/z) 257 (M+H)⁺.

Referring to the method of Example 44, compounds were synthesized according to the following reaction scheme. The synthesized compounds and their data are shown in Table 13 and Table 14, respectively.

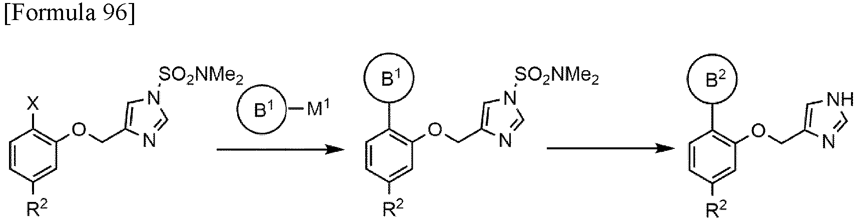

**[Table 13-1]**

| Example | Starting material | X | M¹ | R² | Ring B¹ | Ring B² |
|---|---|---|---|---|---|---|
| 45 | Reference Example 61 | I | -B(OH)₂ | H | 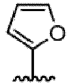 | 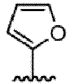 |
| 46 | Reference Example 61 | I | -B(OH)₂ | H | 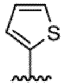 | 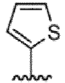 |
| 47 | Reference Example 61 | I | 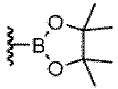 | H | 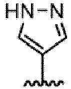 | 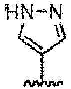 |
| 48 | Reference Example 61 | I | 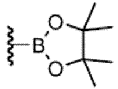 | H | 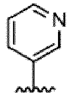 | 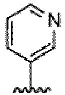 |
| 49 | Reference Example 61 | I | -B(OH)₂ | H | 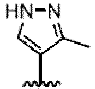 | 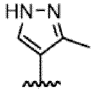 |
| 50 | Reference Example 61 | I | 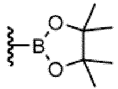 | H | 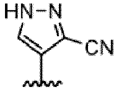 | 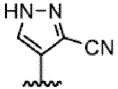 |
| 51 | Reference Example 62 | Br | -B(OH)₂ | H | 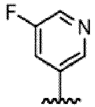 | 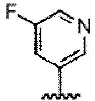 |
| 52 | Reference Example 62 | Br | -B(OH)₂ | H | 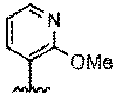 | 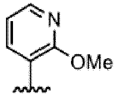 |
| 53 | Reference Example 62 | Br | -B(OH)₂ | H | 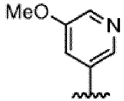 | 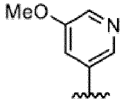 |
| 54 | Reference Example 62 | Br | -B(OH)₂ | H | 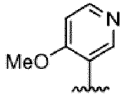 | 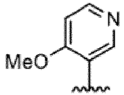 |
| 55 | Reference Example 63 | Br | 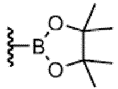 | Me | 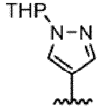 | 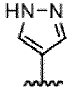 |
| 56 | Reference Example 63 | Br | 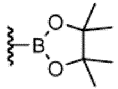 | Me | 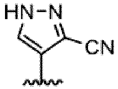 | 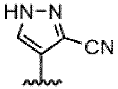 |

**[Table 13-2]**

| Example | Starting | X | M¹ | R² | Ring B¹ | Ring B² |
|---|---|---|---|---|---|---|
| 57 | Reference Example 63 | Br | -B(OH)₂ | Me | 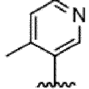 | 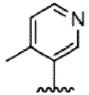 |
| 58 | Reference Example 64 | Br | 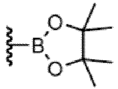 | F | 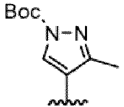 | 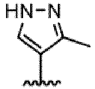 |
| 59 | Reference Example 63 | Br | 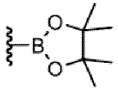 | Me | 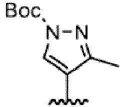 | 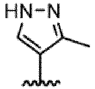 |
| 60 | Reference Example 62 | Br | 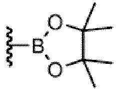 | H | 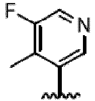 | 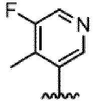 |
| 61 | Reference Example 62 | Br | -B(OH)₂ | H | 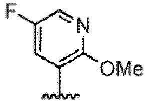 | 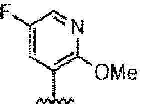 |
| 62 | Reference Example 63 | Br | -B(OH)₂ | Me | 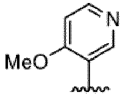 | 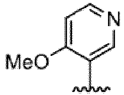 |
| 63 | Reference Example 64 | Br | -B(OH)₂ | F | 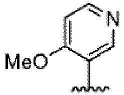 | 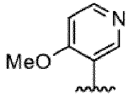 |
| 64 | Reference Example 64 | Br | 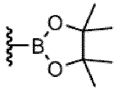 | F | 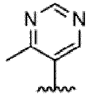 | 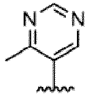 |
| 65 | Reference Example 63 | Br | 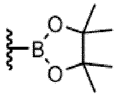 | Me | 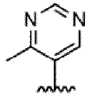 | 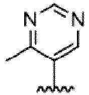 |

**[Table 14-1]**

| Example | ¹H-NMR | ESI/MS (m/z) |
|---|---|---|
| 45 | ¹H-NMR (400 MHz, CD₃OD) δ 7.82 (dd, *J* = 7.8, 1.6 Hz, 1H), 7.75 (d, *J* = 0.7 Hz. 1H), 7.50 (dd, *J =* 1.6, 0.7 Hz, 1H), 7.29-7.18 (m, 3H), 7.02 (td, *J* = 7.8, 1.6 Hz, 1H), 6.87 (d, *J =* 3.3 Hz, 1H), 6.43 (dd. *J =* 3.3, 1.8 Hz, 1H), 5.16 (s, 2H). | 241 (M+H)⁺ |
| 46 | ¹H-NMR (400 MHz, CD₃OD) δ 7.69 (br, 1H), 7.65 (dd, 1H), 7.50 (dd, 1H), 7.35-7.15 (m, 4H), 7.05-6.96 (m, 2H) 5.13 (s, 2H). | 257 (M+H)⁺ |
| 47 | ¹H-NMR (400 MHz, CD₃OD) δ 8.02 (s, 2H), 7.74 (d, *J* = 1.5 Hz, 1H), 7.62 (dd. *J* = 7.6, 1.0 Hz, 1H), 7.25-7.16 (m, 3H), 6.99 (ddd, *J* = 7.6, 7.0, 1.5 Hz, 1H), 5.13 (s, 1H), | 241 (M+H)⁺ |
| 48 | ¹H-NMR (400 MHz, CD₃OD) δ 8.68 (dd, *J =* 1.8, 0.7 Hz, 1H), 8.43 (dd, *J* = 4.9, 1.8 Hz. 1H), 8.00 (dt, *J* = 7.9. 1.8 Hz. 1H), 7.66 (d, *J = 0.7* Hz. 1H), 7.46-7.35 (m, 3H), 7.30-7.26 (m, 1H), 7.13-7.05 (m, 2H), 5.08 (s, 1H), | 252 (M+H)⁺ |
| 49 | ¹H-NMR (400 MHz, CD₃OD) δ 7.64 (d, *J* = 1.2 Hz, 1H), 7.62-7.55 (m, 1H), 7.29-7.21 (m, 2H), 7.15 (dd, *J* = 8.7, 1.2 Hz. 1H), 7.08-7.03 (m, 1H), 6.98 (td, *J* = 7.4, 1.2 Hz,1H), 5.00 (s, 2H). 2.22 (s. 3H). | 255 (M+H)⁺ |
| 50 | ¹H-NMR (400 MHz, CD₃OD) δ 8.06 (s, 1H), 7.68 (d, *J =* 1.1 Hz, 1H), 7.62 (dd. *J* = 7.6, 1.7 Hz, 1H), 7.34 (ddd, *J* = 8.3, 7.4, 1.7 Hz, 1H), 7.23 (dd, *J* = 8.4, 1.1 Hz. 1H), 7.17 (s, 1H), 7.04 (td, *J* = 7.5, 1.1 Hz, 1H), 5.11 (s. 2H). | 266 (M+H)⁺ |
| 51 | ¹H-NMR (400 MHz, CDCl₃) δ 9.28 (brs, 1H), 8.61 (s, 1H), 8.39 (d, *J = 2.8* Hz, 1H), 7.72-7.64 (m, 1H), 7.62 (d, *J =* 1.2 Hz, 1H), 7.43-7.37 (m, 1H), 7.35 (dd, *J* = 7.6, 1.7 Hz, 1H), 7.19 (d, *J =* 8.3 Hz, 1H), 7.09 (t, *J =* 7.5 Hz, 1H), 6.97 (s, 1H), 5.14 (s. 2H). | 270 (M+H)⁺ |
| 52 | ¹H-NMR (400 MHz, CDCl₃) δ 10.19 (brs, 1H), 8.13 (dd, *J =* 5.0, 2.0 Hz, 1H), 7.54 (dd, *J* = 7.2, 2.0 Hz, 1H), 7.50 (d, *J =* 1.2 Hz, 1H), 7.32 (ddd, *J =* 8.3*,* 7.4, 1.8 Hz, 1H), 7.26 (dd, *J* = 7.4, 1.8 Hz. 1H), 7.07 (d, *J* = 8.3 Hz, 1H), 7.03 (ddd, *J* = 7*.*4, 7.4, 1.1 Hz. 1H), 6.91 (dd, J = 7.2, 5.0 Hz, 1H), 6.82 (s, 1H), 5.07 (s. 2H), 3.87 (s, 3H). | 282 (M+H)⁺ |
| 53 | ¹H-NMR (400 MHz, CDCl₃) δ 9.51 (brs, 1H), 8.40 (s, 1H), 8.23 (d, *J* = 2.8 Hz, 1H), 7.59 (d, *J* = 1.2 Hz, 1H), 7.46 (brs, 1H), 7.41-7.31 (m, 2H), 7.17 (d, *J* = 8.1 Hz, 1H), 7.08 (t, *J =* 7.5 Hz, 1H), 6.96 (brs, 1H), 5.11 (s, 2H), 3.84 (s, 3H). | 282 (M+H)⁺ |
| 54 | ¹H-NMR (400 MHz, CDCl₃) δ 8.38 (d, *J =* 5.8 Hz, 1H). 8.33 (s, 1H), 7.47 (d, *J =* 1.2 Hz, 1H), 7.35 (ddd, *J* = 8.3, 7.4, 1.8 Hz. 1H), 7.28-7.23 (m, 1H), 7.13 (dd, *J =* 8.3*,* 1.1 Hz. 1H), 7.03 (td, *J* = 7.4, 1.1 Hz. 1H), 6.84 - 6.79 (m, 2H), 5.06 (s, 2H), 3.75 (s, 3H). | 282 (M+H)⁺ |
| 55 | ¹H-NMR (400 MHz, CD₃OD) δ 8.03-7.85 (m, 2H), 7.73-7.68 (m, 1H), 7.49-7.42 (m, 1H), 7.21-7.15 (m, 1H), 7.01-6.97 (m, 1H), 6.83-6.77 (m, 1H), 5.08 (s, 2H), 2.35 (s, 3H). | 255 (M+H)⁺ |

**[Table 14-2]**

| Example | ¹H-NMR | ESI/MS (m/z) |
|---|---|---|
| 56 | ¹H-NMR (400 MHz, CD₃OD) δ 8.04 (s, 1H), 7.72-7.68 (m, 1H), 7.56-7.50 (m, 1H), 7.20-1.16 (m, 1H), 7.11-7.06 (m, 1H), 6.92-6.86 (m, 1H), 5.11 (s, 2H). 3.33 (s. 3H). | 280 (M+H)⁺ |
| 57 | ¹H-NMR (400 MHz. CD₃OD) δ 8.30-8.24 (m, 1H), 8.18-8.12 (m, 1H). 7.62-7.56 (m, 1H), 7.28-7.22 (m, 1H), 7.10-7.05 (m, 1H) 7.04-6.98 (m, 1H), 6.95-6.86 (m, 2H), 4.99 (s. 2H), 2.42 (s, 3H), 2.15 (s. 3H). | 280 (M+H)⁺ |
| 58 | ¹H-NMR (400 MHz, CD₃OD) δ 7.69-7.44 (m, 2H), 7.28-7.17 (m, 1H), 7.15-7.05 (m, 1H), 7.03-6.92 (m, 1H), 6.77-6.66 (m, 1H), 5.01 (s, 2H), 2.20 (s, 3H). | 273 (M+H)⁺ |
| 59 | ¹H-NMR (400 MHz. CD₃OD) δ 7.68-7.45 (m, 2H), 7.17-6.95 (m, 3H), 6.85-6.76 (m, 1H), 4.98 (s, 2H), 2.36 (s. 3H), 2.21 (s, 3H). | 269 (M+H)⁺ |
| 60 | ¹H-NMR (400 MHz, CDCl₃) δ 9.81 (brs. 1H), 8.31 (s. 1H), 8.19 (s, 1H), 7.55 (d. *J =* 1.2 Hz, 1H), 7.40 (ddd, *J* = 8.3. 7.4. 1.8 Hz. 1H), 7.20-7.12 (m, 2H). 7.05 (ddd, J= 7.4, 7.4, 1.0 Hz. 1H), 6.82 (s. 1H), 5.06 (s. 2H). 2.10 (d. *J* = 2.1 Hz. 3H). | 284 (M+H)⁺ |
| 61 | ¹H-NMR (400 MHz. CDCl₃) δ 9.57 (brs, 1H), 7.97 (d, *J =* 3.0 Hz. 1H), 7.57 (d, *J =* 1.2 Hz, 1H), 7.44-7.33 (m, 2H), 7.31-7.25 (m, 1H), 7.17-7.07 (m, 1H), 7.04 (t, *J =* 7.5 Hz, 1H), 6.85 (s, 1H), 5.10 (s, 2H). 3.86 (s, 3H). | 300 (M+H)⁺ |
| 62 | ¹H-NMR (400 MHz, CD₃OD) δ 8.31 (d. 1H), 8.13 (s, 1H), 7.59 (d, 1H), 7.09-7.00 (m, 3H), 6.91 (br, 1H), 6.87-6.82 (m, 1H), 4.98 (s, 2H), 3.78 (s, 3H), 2.39 (s, 3H). | 296 (M+H)⁺ |
| 63 | ¹H-NMR (400 MHz, CD₃OD) δ 8.33(d, 1H), 8.14 (s, 1H), 7.61 (d, 1H), 7.19 (dd, 1H), 7.06 (d, 1H), 7.04-6.97 (m, 2H), 6.79-6.72 (m, 1H), 5.00 (s, 2H), 3.79 (s, 3H). | 300 (M+H)⁺ |
| 64 | ¹H-NMR (400 MHz, CD₃OD) δ 8.91 (s, 1H), 8.43 (s, 1H), 7.62 (d, 1H), 7.23 (dd, 1H), 7.13 (dd, 1H), 7.06 (br, 1H), 6.88-6.80 (m, 1H), 5.04 (s, 2H), 2.32 (s, 3H). | 285 (M+H)⁺ |
| 65 | ¹H-NMR (400 MHz, CD₃OD) δ 8.89 (s, 1H), 8.40 (s, 1H), 7.60 (d, 1H), 7.12 (br, 1H), 7.07 (d, 1H), 6.99 (br, 1H), 6.93 (m, 1H), 5.02 (s, 2H), 2.43 (s, 3H), 2.33 (s, 3H). | 281 (M+H)⁺ |

### Production of Present Compound, Steps V-4 and 5 (Example 66)

### [Example 66]

### 2-(2-((1H-Imidazol-4-yl)methoxy)-4-methylphenyl)pyrazine

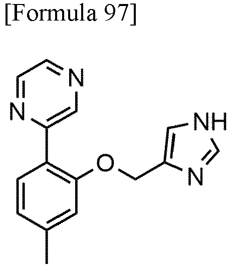

4-((2-Bromo-5-methylphenoxy)methyl)-N,N-dimethyl-1H-imidazole-1-sulfonamide (Reference Example 63, 38 mg), 2-(tributylstannyl)pyrazine (56 mg), and tetrakis(triphenylphosphine)palladium (17 mg) were suspended in toluene (0.7 mL), and the mixture was stirred overnight at 100°C. The reaction solution was concentrated under reduced pressure, and the resulting residue was purified with column chromatography on silica gel (ethyl acetate, using silica pre-column with potassium carbonate added). The resulting compound was dissolved in methanol (1 mL), to the solution was added 2M hydrochloric acid methanol solution (1 mL), and the mixture was stirred at 70°C for 4 hours. The reaction solution was concentrated under reduced pressure, and the resulting residue was then purified with thin-layer chromatography (NH silica; chloroform/methanol = 10/1) to yield the title compound (9 mg).
¹H-NMR (400 MHz, CD₃OD) δ 9.15-9.05 (m, 1H), 8.64-8.56 (m, 1H), 8.41-8.35 (m, 1H), 7.74-7.62 (m, 2H), 7.19-7.09 (m, 2H) 6.98-6.91 (m, 1H), 5.13 (s, 2H), 2.42 (s, 3H).
ESI/MS(m/z) 267 (M+H)⁺.

### Production of Biarylphenol, Step VIII-1 (Reference Examples 65 to 75)

### [Reference Example 65]

### 2-(Thiazol-4-yl)phenol

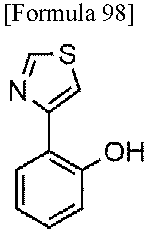

2-Hydroxyphenylboronic acid (200 mg), 4-bromothiazole (250 mg), tetrakis(triphenylphosphine)palladium (81 mg), and potassium carbonate (606 mg) were suspended in a mixed solvent of 1,4-dioxane (10 mL) and water (5 mL), and the mixture was stirred at 90°C for 4.5 hours. The reaction solution was added with water to extract with ethyl acetate. The organic layer was washed with saturated saline, and then dried over anhydrous sodium sulfate. The anhydrous sodium sulfate was filtered, and the residue was then washed with ethyl acetate. The filtrate and washing were combined and concentrated under reduced pressure, and the resulting residue was purified with column chromatography on silica gel (hexane/ethyl acetate = 4/1) to yield the title compound (195 mg).
ESI/MS(m/z) 178 (M+H)⁺.

Referring to the method of Reference Example 65, compounds were synthesized according to the following reaction scheme. The synthesized compounds and their data are shown in Table 15.

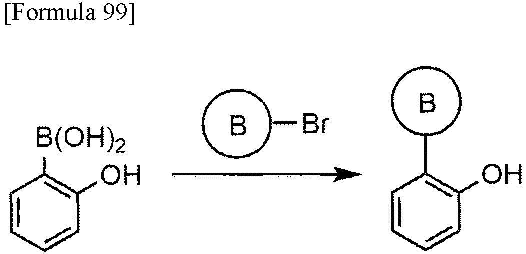

**[Table 15]**

| Reference Example | Ring B | ESI/MS (m/z) |
|---|---|---|
| 66 | 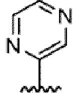 | 173 (M+H)⁺ |
| 67 | 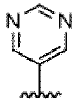 | 173 (M+H)⁺ |
| 68 | 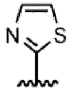 | 178 (M+H)⁺ |
| 69 | 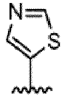 | 178 (M+H)⁺ |
| 70 | 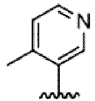 | 186 (M+H)⁺ |
| 71 | 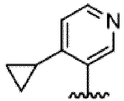 | 212 (M+H)⁺ |
| 72 | 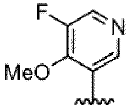 | 220 (M+H)⁺ |
| 73 | 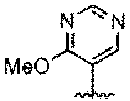 | 203 (M+H)⁺ |
| 74 | 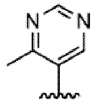 | 187 (M+H)⁺ |
| 75 | 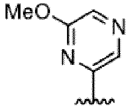 | 203 (M+H)⁺ |

### Production of Present Compound, Steps VI-1 and 2 (Examples 67 to 77)

### [Example 67]

### 4-(2-((1H-Imidazol-4-yl)methoxy)phenyl)thiazole

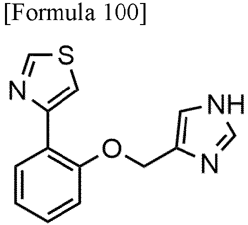

2-(Thiazol-4-yl)phenol (Reference Example 65, 24.3 mg), 4-(hydroxymethyl)-N,N-dimethyl-1H-imidazole-1-sulfonamide (33.7 mg), and triphenylphosphine (46 mg) were dissolved in tetrahydrofuran (2 mL), to the solution was added diisopropyl azodicarboxylate (35 µL), and the mixture was stirred at room temperature for 19 hours. The reaction solution was concentrated under reduced pressure, and the resulting residue was purified with column chromatography on aminosilica gel (hexane/ethyl acetate = 1/1) to yield N,N-dimethyl-4-((2-(thiazol-4-yl)phenoxy)methyl)-1H-imidazole-1-sulfonamide (24.1 mg).
ESI/MS(m/z) 365 (M+H)⁺.

Thus obtained N,N-dimethyl-4-((2-(thiazol-4-yl)phenoxy)methyl)-1H-imidazole-1-sulfonamide (18.2 mg) was dissolved in methanol (1 mL), to the solution was added 2M hydrochloric acid methanol solution (1 mL), and the mixture was stirred at 70°C for 3 hours. The reaction solution was concentrated under reduced pressure, and then diluted with chloroform. The mixture was washed with saturated sodium bicarbonate aqueous solution and saturated saline, and then dried over anhydrous sodium sulfate. The anhydrous sodium sulfate was filtered, and the residue was then washed with chloroform. The filtrate and washing were combined and concentrated under reduced pressure, and the resulting residue was purified with thin-layer chromatography (NH silica; chloroform/methanol = 20/1) to yield the title compound (12.9 mg).
¹H-NMR (400 MHz, CDCl₃) δ 9.44 (brs, 1H), 8.84 (s, 1H), 8.18 (brs, 1H), 8.01 (brs, 1H), 7.68-7.65 (m, 1H), 7.31 (ddd, J = 8.3, 7.3, 1.8 Hz, 1H), 7.18-7.01 (m, 3H), 5.23 (s, 2H).
ESI/MS(m/z) 258 (M+H)⁺.

Referring to the method of Example 67, compounds were synthesized according to the following reaction scheme. The synthesized compounds and their data are shown in Table 16 and Table 17, respectively.

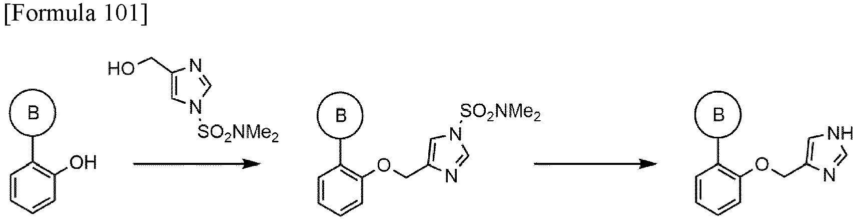

**[Table 16]**

| Example | Starting material | Ring B |
|---|---|---|
| 68 | Reference Example 66 | 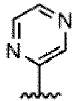 |
| 69 | Reference Example 67 | 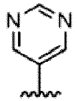 |
| 70 | Reference Example 68 | 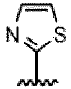 |
| 71 | Reference Example 69 | 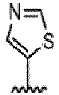 |
| 72 | Reference Example 70 | 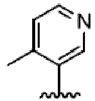 |
| 73 | Reference Example 71 | 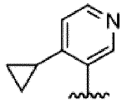 |
| 74 | Reference Example 72 | 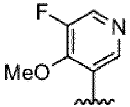 |
| 75 | Reference Example 73 | 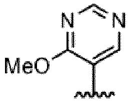 |
| 76 | Reference Example 74 | 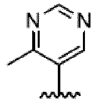 |
| 77 | Reference Example 75 | 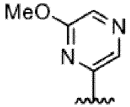 |

**[Table 17]**

| Example | ¹H-NMR | ESI/MS (m/z) |
|---|---|---|
| 68 | ¹H-NMR (400 MHz, CD₃OD) δ 9.12 (brs, 1H), 8.64 (dd, *J =* 2.6, 1.6 Hz, 1H), 8.42 (d, *J* = 2.6 Hz, 1H), 7.80 (dd, *J =* 7.7, 1.8 Hz, 1H), 7.67 (d, *J* = 1.2 Hz, 1H), 7.50-7.44(m, 1H), 7.31 (dd, *J =* 8.4, 1.0 Hz, 1H), 7.18-7.08 (m, 2H), 5.17 (s, 2H). | 253 (M+H)⁺ |
| 69 | ¹H-NMR (400 MHz, CD₃OD) δ 9.02 (brs, 1H), 8.93 (brs, 2H), 7.78 (br, 1H), 7.50-7.40 (m, 2H), 7.30 (d, *J* = 8.3 Hz, 1H), 7.19-7.09 (m, 2H) 5.11 (s, 2H). | 253 (M+H)⁺ |
| 70 | ¹H-NMR (400 MHz, CDCl₃) δ 9.45 (brs, 1H), 8.38-8.32 (m, 1H), 7.89 (d, *J* = 3.3 Hz, 1H), 7.68 (d, *J* = 1.2 Hz, 1H), 7.42-7.30 (m, 2H), 7.21-7.14 (m, 2H), 7.09 (ddd, *J =* 8.2, 7.3, 1.2 Hz, 1H), 5.32 (s, 2H). | 258 (M+H)⁺ |
| 71 | ¹H-NMR (400 MHz, CDCl₃) δ 9.65 (brs. 1H), 8.74 (s, 1H), 8.28 (brs, 1H), 7.68-7.60 (m, 2H), 7.35-7.29 (m, 1H), 7.20-7.11 (m, 2H), 7.07-7.01 (m, 1H), 5.21 (s, 2H). | 258 (M+H)⁺ |
| 72 | ¹H-NMR (400 MHz. CDCl₃) δ 9.46 (brs, 1H), 8.41 (d, *J* = 5.0 Hz, 1H), 8.36 (s. 1H), 7.55 (d, *J =* 1.2 Hz. 1H), 7.39 (ddd, *J* = 8.3, 7.4, 1.8 Hz. 1H), 7.20-7.12 (m, 3H), 7.06 (td, *J =* 7.4, 1.1 Hz. 1H), 6.79 (s, 1H). 5.06 (s, 2H), 2.17 (s, 3H). | 266 (M+H)⁺ |
| 73 | ¹H-NMR (400 MHz, CDCl₃) δ 9.99 (brs, 1H), 8.37 (d, *J =* 5.3 Hz, 1H), 8.33 (s, 1H), 7.52 (d, *J* = 1.2 Hz, 1H), 7.38 (ddd, *J* = 8.2, 7.4, 1.8 Hz, 1H), 7.29-7.23 (m, 1H), 7.22-7.12 (m, 1H), 7.07 (t, *J =* 7.4 Hz, 1H), 6.75 (s, 1H), 6.70 (d, *J =* 5.3 Hz, 1H), 5.07 (s, 2H), 1.82-1.70 (m, 1H), 0.96-0.82 (m, 2H), 0.78-0.62 (m, 2H). | 292 (M+H)⁺ |
| 74 | ¹H-NMR (400 MHz, CD₃OD) δ 8.30 (d, *J =* 4*.*2 Hz, 1H), 8.06 (s, 1H), 7.60 (d, *J* = 1.2 Hz, 1H), 7.40 (ddd, *J* = 8.4, 7.5, 1.8 Hz, 1H), 7.23 (dd, *J* = 8.4, 1.0 Hz, 1H), 7.20 (dd, *J* = 7.5, 1.8 Hz, 1H), 7.03 (td, *J* = 7.5, 1.0 Hz, 1H), 7.00 (brs. 1H), 5.03 (s, 2H), 3.87 (d, *J =* 3.6 Hz. 3H). | 300 (M+H)⁺ |
| 75 | ¹H-NMR (400 MHz. CD₃OD) δ 8.67 (s, 1H), 8.33 (s, 1H), 7.64 (s, 1H), 7.42 (ddd, *J =* 8.3, 7.5, 1.8 Hz, 1H), 7.28 (dd, *J* = 7.5, 1.8 Hz, 1H), 7.26-7.22 (m, 1H), 7.06 (td, *J =* 7.5. 1.0 Hz, 1H), 7.01 (s, 1H), 5.05 (s, 2H), 3.93 (s, 3H). | 283 (M+H)⁺ |
| 76 | ¹H-NMR (400 MHz, CD₃OD) δ 8.93 (s, 1H), 8.45 (s, 1H), 7.63 (s, 1H), 7.49 (ddd. *J =* 8.3, 7.5, 1.8 Hz, 1H), 7.32 (d, *J =* 8.3 Hz, 1H), 7.23 (dd, *J =* 7.5, 1.8 Hz. 1H), 7.12 (td, *J =* 7.5. 0.9 Hz, 1H), 7,03 (s, 1H), 5.06 (s. 2H), 2.35 (s. 3H). | 267 (M+H)⁺ |
| 77 | ¹H-NMR (400 MHz. CD₃OD) δ 8.76 (s. 1H), 8.00-7.98 (m, 2H). 7.69 (d, *J =* 1.2 Hz, 1H), 7.45-7.41 (m, 1H), 7.27 (d, *J =* 7.6 Hz, 1H), 7.17 (d, *J =* 0.4 Hz. 1H), 7.10 (ddd, *J* = 7.6, 7.6, 0.8 Hz, 1H), 5.14, (s, 2H), 4.01 (s, 3H). | 283 (M+H)⁺ |

### Production of Imidazolyl Methoxyaryl 3, Steps VII-1 and 2 (Reference Examples 76 to 77)

### [Reference Example 76]

### 4-(1-Hydroxyethyl)-N,N-dimethyl-1H-imidazole-1-sulfonamide

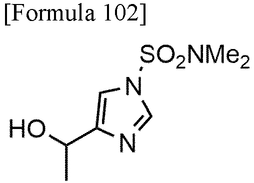

4-Formyl-N,N-dimethyl-1H-imidazole-1-sulfonamide (500 mg) was dissolved in tetrahydrofuran (25 mL), to the mixture was added 1M solution of methylmagnesium bromide in tetrahydrofuran (2.5 mL) under ice-cooling, and the mixture was stirred at room temperature for 20 hours. Under ice-cooling, the reaction solution was added with saturated ammonium chloride aqueous solution and water to extract with ethyl acetate. The organic layer was washed with saturated saline, and then dried over anhydrous sodium sulfate. The anhydrous sodium sulfate was filtered, and the residue was then washed with ethyl acetate. The filtrate and washing were combined and concentrated under reduced pressure, and the resulting residue was purified with column chromatography on silica gel (chloroform/methanol = 12/1) to yield the title compound (540 mg).
ESI/MS(m/z) 220 (M+H)⁺

### [Reference Example 77]

### 4-(1-Chloroethyl)-N,N-dimethyl-1H-imidazole-1-sulfonamide

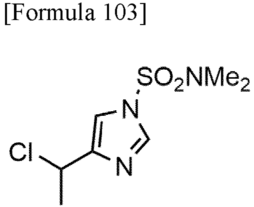

4-(1-Hydroxyethyl)-N,N-dimethyl-1H-imidazole-1-sulfonamide (Reference Example 76, 45 mg) was dissolved in chloroform (2.5 mL), to the solution were added triethylamine (47 µL) and methanesulfonyl chloride (21 µL) under ice-cooling, and the mixture was stirred under ice-cooling for 1.5 hours. The reaction solution was added with water to extract with chloroform. The organic layer was washed with saturated saline, and then dried over anhydrous sodium sulfate. The anhydrous sodium sulfate was filtered, and the residue was then washed with chloroform. The filtrate and washing were combined and concentrated under reduced pressure to yield the title compound (38 mg).
ESI/MS(m/z) 238 (M+H)⁺.

### [Reference Example 78]

### 2-(Thiophen-2-yl)phenol

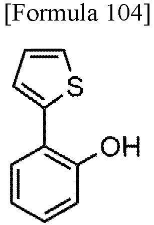

2-Hydroxyphenylboronic acid (1.00 g), tetrakis(triphenylphosphine)palladium (418 mg), and potassium carbonate (3.00 g) were suspended in a mixed solvent of 1,4-dioxane (24 mL) and water (12 mL), to the suspension was added 2-bromothiophene (930 µL), and the mixture was stirred at 90°C for 4 hours. The reaction solution was added with water to extract with ethyl acetate. The organic layer was washed with saturated saline, and then dried over anhydrous sodium sulfate. The anhydrous sodium sulfate was filtered, and the residue was then washed with ethyl acetate. The filtrate and washing were combined and concentrated under reduced pressure, and the resulting residue was purified with column chromatography on silica gel (hexane/ethyl acetate = 6/1) to yield the title compound (909 mg).
ESI/MS(m/z) 177 (M+H)⁺.

### Production of Present Compound, Steps VII-3 and 4 (Examples 78 to 79, Reference Example 79)

### [Example 78]

### 4-(1-(2-(Thiophen-2-yl)phenoxy)ethyl)-1H-imidazole

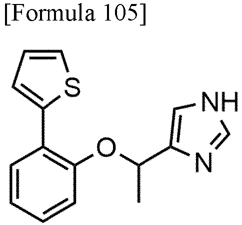

2-(Thiophen-2-yl)phenol (Reference Example 78, 50 mg) was dissolved in N,N-dimethylformamide (2 mL), to the solution was added potassium carbonate (65 mg) and 4-(1-chloroethyl)-N,N-dimethyl-1H-imidazole-1-sulfonamide (Reference Example 77, 36 mg), and the mixture was stirred at room temperature for 1 hour. To the reaction solution was added potassium iodide (56 mg) and stirred at room temperature for 1 hour. The solution was then heated to 50°C and stirred for 21 hours. The reaction solution was added with water to extract with ethyl acetate. The organic layer was washed with saturated saline, and then dried over anhydrous sodium sulfate. The anhydrous sodium sulfate was filtered, and the residue was then washed with ethyl acetate. The filtrate and washing were combined and concentrated under reduced pressure, and the resulting residue was purified with column chromatography on silica gel (hexane/ethyl acetate = 3/2) to yield N,N-dimethyl-4-(1-(2-(thiophen-2-yl)phenoxy)ethyl)-1H-imidazole-1-sulfonamide (24 mg).
ESI/MS(m/z) 378 (M+H)⁺.

Thus obtained N,N-dimethyl-4-(1-(2-(thiophen-2-yl)phenoxy)ethyl)-1H-imidazole-1-sulfonamide (23 mg) was dissolved in methanol (600 µL), to the solution was added 2M hydrochloric acid methanol solution (600 µL), and the mixture was stirred at 70°C for 19 hours. The reaction solution was concentrated under reduced pressure, and the resulting residue was then purified with thin-layer chromatography (NH silica; chloroform/methanol = 50/1) and thin-layer chromatography (chloroform/methanol = 12/1) in this order to yield the title compound (7.2 mg).
¹H-NMR (400 MHz, CD₃OD) δ 7.64-7.60 (m, 2H), 7.52 (dd, J = 3.7, 1.2 Hz, 1H), 7.36 (dd, J = 5.2, 1.2 Hz, 1H), 7.14 (ddd, J = 8.8, 7.2, 1.7 Hz, 1H), 7.09-7.01 (m, 2H), 6.98-6.91 (m, 2H), 5.56 (q, J = 6.4 Hz, 1H), 1.72 (d, J = 6.4 Hz, 3H).
ESI/MS(m/z) 271 (M+H)⁺.

### [Example 79]

### (+)-4-(1-(2-(Thiophen-2-yl)phenoxy)ethyl)-1H-imidazole, and

### [Reference Example 79]

### (-)-4-(1-(2-(Thiophen-2-yl)phenoxy)ethyl)-1H-imidazole

(4-(1-(2-(thiophen-2-yl)phenoxy)ethyl)-1H-imidazole), The compound produced in Example 78 was subjected to optical resolution using high performance liquid chromatography (CHIRALPAK IC; hexane/isopropanol/diethylamine = 950/50/1) to yield the title compounds.
Example 79: ¹H-NMR (400 MHz, CDCl₃) δ 7.67 (dd, J = 7.7, 1.7 Hz, 1H), 7.61 (d, J = 1.1 Hz, 1H), 7.55 (dd, J = 3.7, 1.1 Hz, 1H), 7.35 (dd, J = 5.1, 1.1 Hz, 1H), 7.18 (ddd, J = 8.4, 7.7, 1.7 Hz, 1H), 7.12 (dd, J = 5.1, 3.7 Hz, 1H), 7.05 (d, J = 7.7 Hz, 1H), 7.01-6.95 (m, 2H), 5.63 (q, J = 6.4 Hz, 1H), 1.80 (d, J = 6.4 Hz, 3H).
ESI/MS(m/z) 271 (M+H)⁺.
Reference Example 79: ¹H-NMR (400 MHz, CDCl₃) δ 9.07 (brs, 1H), 7.67 (dd, J = 7.7, 1.6 Hz, 1H), 7.60 (s, 1H), 7.54 (s, 1H), 7.38-7.34 (m, 1H), 7.18 (t, J = 7.2 Hz, 1H), 7.12 (dd, J = 5.1, 3.7 Hz, 1H), 7.06 (d, J = 7.7 Hz, 1H), 7.01-6.95 (m, 2H), 5.63 (q, J = 6.4 Hz, 1H), 1.80 (d, J = 6.4 Hz, 3H).
ESI/MS(m/z) 271 (M+H)⁺.

### <Production of Present Compound in Which Biaryl Moiety and Imidazole Moiety are Bound Via Sulfur Atom>

### Production of Imidazolyl Methylthioaryl, Step IX-1 (Reference Example 80)

### [Reference Example 80]

### 4-(((2-Bromophenyl)thio)methyl)-N,N-dimethyl-1H-imidazole-1-sulfonamide

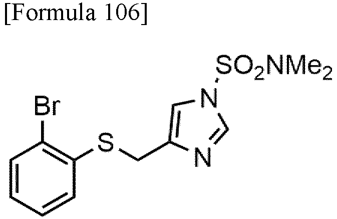

To the mixture of 4-(Chloromethyl)-N,N-dimethyl-1H-imidazole-1-sulfonamide hydrochloride (100 mg), potassium iodide (4.2 mg), and potassium carbonate (106.1 mg) were added N,N-dimethylformamide (2.5 mL) and 2-bromobenzenethiol (31 µL), and the mixture was stirred at room temperature for 15 hours, and at 60°C for 2 hours. The reaction solution was added with water, extracted with ethyl acetate, and the organic layer was dried over anhydrous sodium sulfate. The anhydrous sodium sulfate was filtered, and the residue was then washed with ethyl acetate. The filtrate and washing were combined and concentrated under reduced pressure, and the resulting residue was purified with thin-layer chromatography (hexane/ethyl acetate = 1/1), and further purified with thin-layer chromatography (chloroform/ethyl acetate = 9/1) to yield the title compound (82.1 mg).
ESI/MS(m/z) 377 (M+H)⁺.

### Production of Present Compound, Steps IX-2 and 3 (Examples 80 to 82)

### [Example 80]

### 3-(2-(((1H-Imidazol-4-yl)methyl)thio)phenyl)pyridine

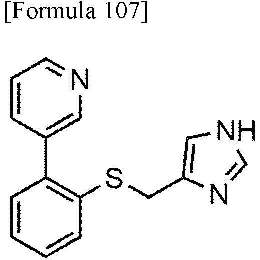

The mixture of 4-(((2-Bromophenyl)thio)methyl)-N,N-dimethyl-1H-imidazole-1-sulfonamide (Reference Example 80, 20 mg), 3-(4,4,5,5-tetramethyl-1,3,2-dioxaborolan-2-yl)pyridine (16.4 mg), tetrakis(triphenylphosphine)palladium (6.1 mg), and potassium carbonate (22.1 mg) was added with 1,4-dioxane (1.5 mL) and water (0.3 mL), and the mixture was stirred at 90°C for 15 hours. The reaction solution was added with water, extracted with ethyl acetate, and the organic layer was dried over anhydrous sodium sulfate. The anhydrous sodium sulfate was filtered, and the residue was then washed with ethyl acetate. The filtrate and washing were combined and concentrated under reduced pressure, and the resulting residue was purified with thin-layer chromatography (hexane/ethyl acetate = 1/2) to yield N,N-dimethyl-4-(((2-(pyridin-3-yl)phenyl)thio)methyl)-1H-imidazole-1-sulfonamide (25.2 mg).
ESI/MS(m/z) 375 (M+H)⁺.

Thus obtained N,N-dimethyl-4-(((2-(pyridin-3-yl)phenyl)thio)methyl)-1H-imidazole-1-sulfonamide (25.2 mg) was added with methanol (0.5 mL) and 2M hydrochloric acid methanol solution (0.5 mL), and the mixture was stirred at 70°C for 10 hours. The reaction solution was concentrated under reduced pressure, and the resulting residue was then purified with thin-layer chromatography (chloroform/methanol = 20/1) to yield the title compound (6.3 mg).
¹H-NMR (400 MHz, CD₃OD) δ 8.49 (dd, J = 4.8, 1.6 Hz, 1H), 8.42 (dd, J = 2.4, 0.8 Hz, 1H), 7.77 (ddd, J = 8.0, 2.4, 1.6 Hz, 1H), 7.56 (dd, J = 7.6, 1.2 Hz, 1H), 7.52 (d, J = 1.2 Hz, 1H), 7.46 (ddd, J = 8.0, 4.8, 0.8 Hz, 1H), 7.38 (ddd, J = 7.6, 7.6, 1.6 Hz, 1H), 7.32 (ddd, J = 7.6, 7.6, 1.2 Hz, 1H), 7.25 (m, 1H), 6.69 (s, 1H), 3.96 (d, J = 0.8 Hz, 2H).
ESI/MS(m/z) 268 (M+H)⁺

Referring to the method of Example 80, compounds were synthesized according to the following reaction scheme. The synthesized compounds and their data are shown in Table 18 and Table 19, respectively.

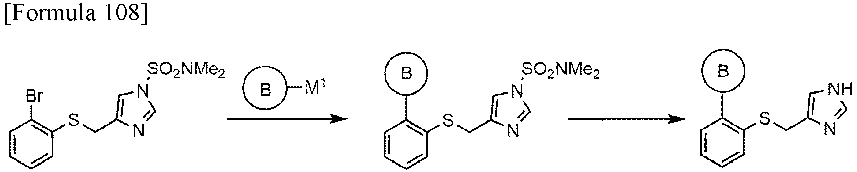

**[Table 18]**

| Example | Ring B | M¹ |
|---|---|---|
| 81 | 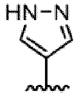 | 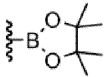 |
| 82 | 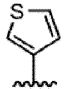 | -B(OH)₂ |

**[Table 19]**

| Example | ¹H-NMR | ESI/MS (m/z) |
|---|---|---|
| 81 | ¹H-NMR (400 MHz, CD₃OD) δ 7.85 (s, 2H), 7.54 (d, *J* = 1.2 Hz, 1H), 7.46-7.41 (m, 1H), 7.38-7.33 (m, 1H), 7.23-7.17 (m, 2H), 6.75-6.74 (m, 1H), 4.01 (d, *J =* 0.8 Hz, 2H). | 257 (M+H)⁺ |
| 82 | ¹H-NMR (400 MHz, CD₃OD) δ 7.53 (d, *J* = 0.8 Hz, 1H), 7.44-7.39 (m, 3H), 7.30-7.18 (m, 4H), 6.74 (d, *J =* 1.2 Hz, 1H), 3.95 (d, *J* = 0.8 Hz. 2H). | 273 (M+H)⁺ |

### <Pharmacological Test Examples>

### (1) Culture of Human α1A Adrenergic Receptor-Expressing Cells

CHO-K1 cells that highly express a human α1A adrenergic receptor (GeneBLAzer^{™} ADRA1A-NFAT-bla CHO-K1 Cells) were purchased from Thermo Fisher Scientific, Inc., and cultured according to the instructions.

### (2) Ca²⁺ Mobilization Assay in Human α1A Adrenergic Receptor-Expressing Cells

The cultured human α1A adrenergic receptor-expressing cells were washed with D-PBS, then replaced with a loading buffer (2 µmol/L Fluo-4/AM, 1x PowerLoad, 2.5 mmol/L Probenecid-containing DMEM (high-glucose)) and incubated at 37°C for 60 minutes in a 5% CO₂ incubator. The cells were detached using 0.05% trypsin-EDTA solution, and suspended in an assay buffer (1.15 mol/L NaCl, 0.054 mol/L KCl, 1.8 mmol/L CaCl₂, 1 mmol/L MgSO₄, 0.11 mol/L glucose, 0.01 mol/L NaH₂PO₄·2H₂O, 0.25 mol/L HEPES, pH 7.3) to be 500,000 cells/mL.

Test compounds were dissolved in dimethylsulfoxide, and diluted with an assay buffer containing 0.3% BSA. The final concentrations of the test compounds were set to fall within the range of 0.1 nmol/L to 10 µmol/L. As a reference compound, norepinephrine was set to have a final concentration within the range of 0.01 nmol/L to 100 µmol/L.

On a 384-well clear bottom black plate coated with poly-D-lysine (Corning Inc.), the above-described cell-suspended solution was dispersed in 40 µL each (20,000 cells/well) and allowed to stand for 10 minutes. The plate was transferred to a fluorescent imaging plate reader (FDSS7000, Hamamatsu Photonics K.K.), and measurements were started. After 1 minute from the start of the measurements, 20 µL of the test compound solutions or norepinephrine solution (total amount: 60 µL/well) was added to each well of the plate (final concentration: 0.1% BSA, 0.1% dimethylsulfoxide), and the temporal change in fluorescence intensity of Fluo-4 was further measured for 5 minutes (Ex 480 nm/Em 540 nm).

### (3) Calculation of Emax Value and EC₅₀ Value

The maximum fluorescence values in the changes in fluorescence intensity of the test compounds and norepinephrine were obtained and normalized by setting the maximum fluorescence value of a well that does not contain the test compound to 0% and the maximum fluorescence value of 100 µmol/L norepinephrine to 100%. The normalized values (% activation) were used for subsequent analyses.

Emax values and EC₅₀ values were calculated by creating a concentration-response curve of the test compounds using a 4-parameter logistic regression with XLFit, Emax value being defined as % activation of test compound at the concentration of 10 µmol/L, and EC₅₀ value being defined as the concentration in which 50% response of the Emax values is shown. The results thereof are shown in Table 20.

The results revealed that all the compounds of Examples tested in this study have high α1A receptor agonistic action.

**[Table 20-1]**

| Example | EC₅₀(nM) | Emax(%) | Example | EC₅₀(nM) | Eₘₐₓ(%) |
|---|---|---|---|---|---|
| 1 | 0.8 | 103 | 39 | 1.9 | 94 |
| 2 | 0.9 | 105 | 40 | 0.6 | 111 |
| 3 | 0.3 | 92 | 41 | 3.4 | 92 |
| 4 | 0.6 | 93 | 42 | 7.3 | 97 |
| 5 | 0.3 | 98 | 43 | 0.6 | *90* |
| 6 | 1.1 | 92 | 44 | 2.2 | 108 |
| 7 | 4.5 | 92 | 45 | 9.4 | 107 |
| 8 | 1.2 | 96 | 46 | 4.8 | 104 |
| 9 | 2.1 | 101 | 47 | 9.9 | 95 |
| 10 | 3.8 | 103 | 48 | 3.0 | 104 |
| 11 | 1.8 | 104 | 49 | 4.3 | 104 |
| 12 | 0.4 | 101 | 50 | 0.9 | 110 |
| 13 | 1.1 | 101 | 51 | 1.8 | 102 |
| 14 | 2.2 | 88 | 52 | 2.7 | 90 |
| 15 | 0.1 | 98 | 53 | 5.1 | 93 |
| 16 | 9.6 | 88 | 54 | 1.6 | 98 |
| 17 | 2.0 | 90 | 55 | 0.6 | 101 |
| 18 | 5.3 | 88 | 56 | 0.8 | 105 |
| 19 | 3.1 | 102 | 57 | 6.1 | 96 |
| 20 | 2.4 | 91 | 58 | 1.0 | 97 |
| 21 | 0.2 | 92 | 59 | 0.4 | 101 |
| 22 | 1.2 | 88 | 60 | 3.2 | 90 |
| 23 | 0.1 | 95 | 61 | 4.5 | 90 |
| 24 | 0.2 | 101 | 62 | 2.4 | 91 |
| 25 | 1.7 | 100 | 63 | 0.7 | 92 |
| 26 | 1.2 | 105 | 64 | 5.3 | 99 |
| 27 | 2.3 | 100 | 65 | 6.7 | 98 |
| 28 | 4.9 | 96 | 66 | 1.3 | 96 |
| 29 | 2.3 | 96 | 67 | 2.1 | 103 |
| 30 | 0.7 | 97 | 68 | 2.3 | 100 |
| 31 | 0.8 | 103 | 69 | 0.9 | 104 |
| 32 | 1.1 | 100 | 70 | 2.4 | 93 |
| 33 | 3.9 | 97 | 71 | 1.9 | 103 |
| 34 | 1.3 | 91 | 72 | 7.4 | 106 |
| 35 | 5.1 | 90 | 73 | 0.7 | 89 |
| 36 | 3.7 | 86 | 74 | 0.5 | 96 |
| 37 | 2.1 | 109 | 75 | 0.2 | 95 |
| 38 | 0.8 | 107 | 76 | 3.3 | 98 |

**[Table 20-2]**

| Example | EC₅₀(nM) | Emax(%) |
|---|---|---|
| 77 | 0.6 | 92 |
| 78 | 6.4 | 99 |
| 79 | 4.5 | 108 |
| 80 | 3.4 | 101 |
| 81 | 9.0 | 106 |
| 82 | 6.9 | 104 |
| Comparative compound 1 | 20 | 91 |

The comparative compound 1 represents the compound 14 described in Non Patent Literature 3 (Bioorg Med Chem Lett 12 3449-3452 2002), (N-((1H-imidazol-4-yl)methyl)-2-(oxazol-5-yl)aniline).

The present compound has an excellent adrenergic α1A receptor agonistic action, compared to the comparative compound 1.

### [Industrial Applicability]

The present compound has an excellent adrenergic α1A receptor agonistic action, and is therefore useful as a preventive or therapeutic drug for orthostatic hypotension, essential hypotension, acute hypotension associated with various diseases or conditions, and urinary incontinence.

## Claims

1. A compound represented by the following general formula (I) or a pharmacologically acceptable salt thereof: 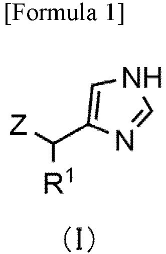
[wherein R¹ represents a hydrogen atom or a C₁-C₃ alkyl group,
Z is selected from the group consisting of the following formulas: 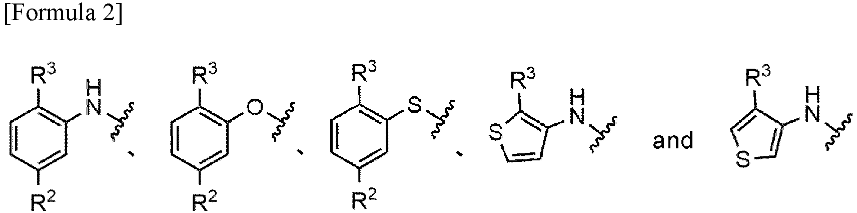
wherein R² represents a hydrogen atom, a fluorine atom, or a C₁-C₃ alkyl group,
R³ is selected from the group consisting of the following formulas: 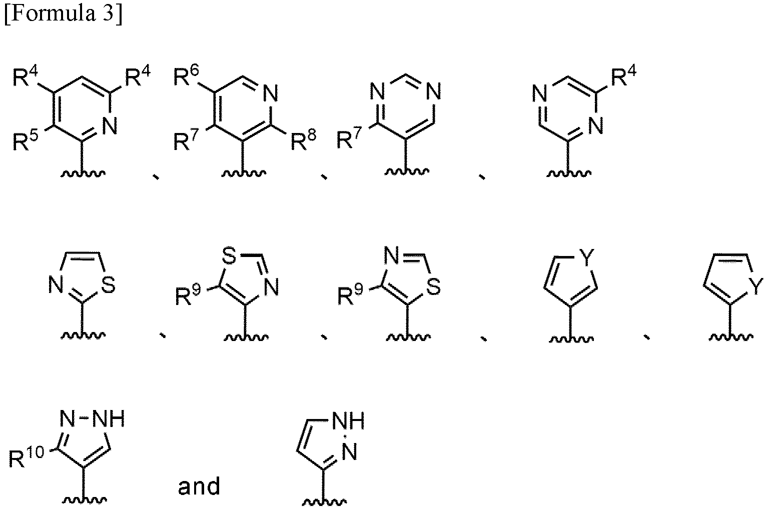
wherein Y represents an oxygen atom or a sulfur atom,
R⁴ each independently represents a hydrogen atom or a C₁-C₃ alkoxy group,
R⁵ represents a hydrogen atom, a fluorine atom, a C₁-C₃ alkyl group, or a C₁-C₃ alkoxy group,
R⁶ represents a hydrogen atom, a fluorine atom, or a C₁-C₃ alkoxy group,
R⁷ represents a hydrogen atom, a C₁-C₃ alkyl group, a C₁-C₃ alkoxy group, or a cyclopropyl group,
R⁸ represents a hydrogen atom or a C₁-C₃ alkoxy group,
R⁹ represents a hydrogen atom or a C₁-C₃ alkyl group,
R¹⁰ represents a hydrogen atom, a C₁-C₃ alkyl group, a C₁-C₃ alkoxy group, or a cyano group, and
when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom].

2. A compound represented by the following general formula (I) or a pharmacologically acceptable salt thereof: 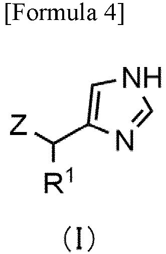
[wherein R¹ represents a hydrogen atom or a C₁-C₃ alkyl group,
Z is selected from the group consisting of the following formulas: 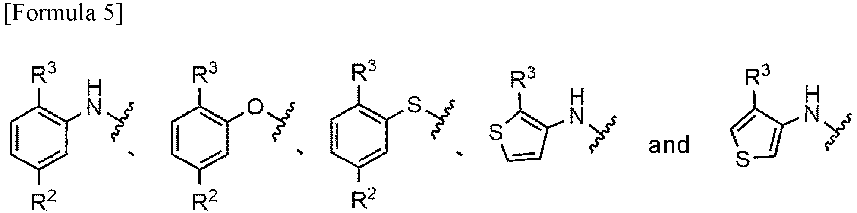
wherein R² represents a hydrogen atom, a fluorine atom, or a C₁-C₃ alkyl group,
R³ is selected from the group consisting of the following formulas: 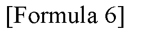 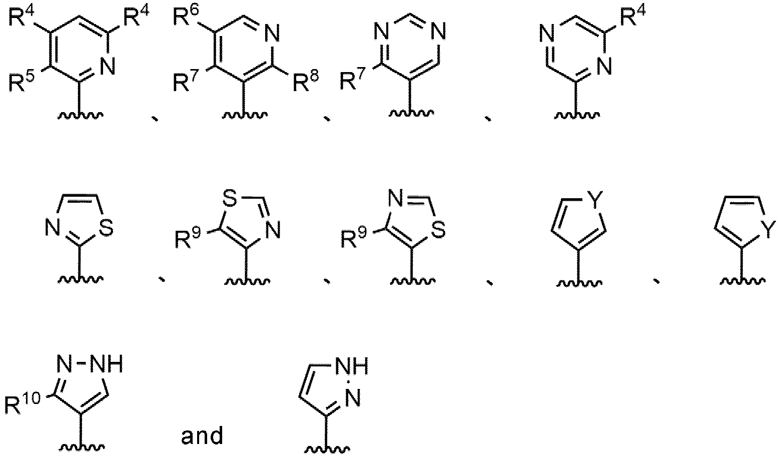
wherein Y represents an oxygen atom or a sulfur atom,
R⁴ each independently represents a hydrogen atom or a C₁-C₃ alkoxy group,
R⁵ represents a hydrogen atom, a fluorine atom, a C₁-C₃ alkyl group, or a C₁-C₃ alkoxy group,
R⁶ represents a hydrogen atom, a fluorine atom, or a C₁-C₃ alkoxy group,
R⁷ represents a hydrogen atom, a C₁-C₃ alkyl group, a C₁-C₃ alkoxy group, or a cyclopropyl group,
R⁸ represents a hydrogen atom or a C₁-C₃ alkoxy group,
R⁹ represents a hydrogen atom or a C₁-C₃ alkyl group,
R¹⁰ represents a hydrogen atom, a C₁-C₃ alkyl group, a C₁-C₃ alkoxy group, or a cyano group, and
when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom,
provided that when Z is: 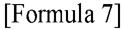 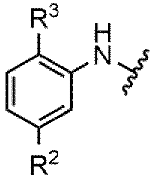 , and R² is a hydrogen atom, R³ is not 3-fluoropyridine bound at position 2;
when Z is: 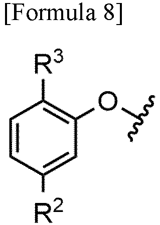 , and R² is a hydrogen atom, R³ is neither non-substituted pyridine bound at position 2, non-substituted pyrazole bound at position 3, nor 3-methoxypyrazole bound at position 4;
when Z is: 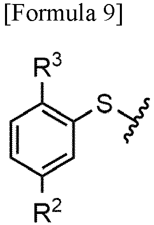 , and R² is a hydrogen atom, R³ is not non-substituted furan bound at position 2;
when Z is: 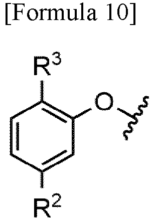 , R¹ is a C₁-C₃ alkyl group and R² is a hydrogen atom, R³ is not non-substituted pyrazole bound at position 4; and
when Z is: 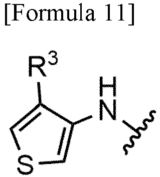 , R³ is not non-substituted thiazole bound at position 5, and
of two enantiomers that occur when R¹ is a C₁-C₃ alkyl group, the enantiomer having a weaker α1A agonistic action than 4-(2-((1H-imidazol-4-yl)methoxy)phenyl)-1H-pyrazole is excluded].

3. The compound or pharmacologically acceptable salt thereof according to claim 2, wherein R³ is selected from the group consisting of the following formulas: 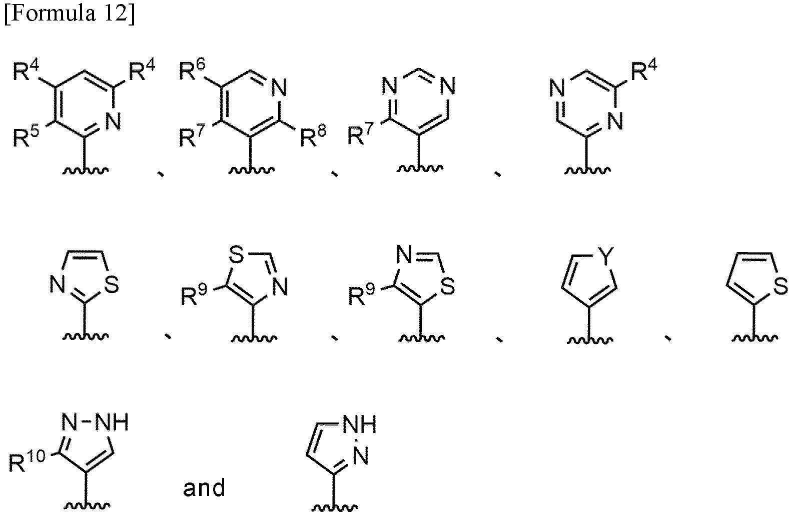
[wherein Y represents an oxygen atom or a sulfur atom,
R⁴ each independently represents a hydrogen atom or a C₁-C₃ alkoxy group,
R⁵ represents a hydrogen atom, a fluorine atom, or a C₁-C₃ alkoxy group,
R⁶ represents a hydrogen atom, a fluorine atom, or a C₁-C₃ alkoxy group,
R⁷ represents a hydrogen atom, a C₁-C₃ alkyl group, a C₁-C₃ alkoxy group, or a cyclopropyl group,
R⁸ represents a hydrogen atom or a C₁-C₃ alkoxy group,
R⁹ represents a hydrogen atom or a C₁-C₃ alkyl group,
R¹⁰ represents a hydrogen atom, a C₁-C₃ alkyl group, a C₁-C₃ alkoxy group, or a cyano group, and
when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom,
provided that when Z is: 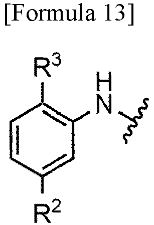
, R³ is neither thiophene bound at position 4 and substituted with R⁹ at position 3, pyridine bound at position 3 and substituted with a C₁-C₃ alkyl group at position 4, nor non-substituted pyrazole bound at position 4;
when Z is: 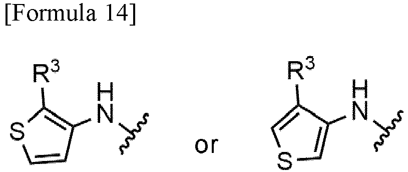
, R³ is neither thiophene bound at position 4 and substituted with R⁹ at position 3, pyridine bound at position 3 and substituted with a C₁-C₃ alkyl group at position 4, non-substituted pyrazole bound at position 4, non-substituted pyrimidine bound at position 5, nor pyrimidine bound at position 5 and substituted with a C₁-C₃ alkyl group at position 4;
when Z is: 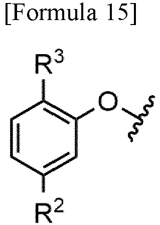 , and R² is a C₁-C₃ alkyl group, R³ is neither thiophene bound at position 4 and substituted with R⁹ at position 3, non-substituted pyrimidine bound at position 5, nor pyrimidine bound at position 5 and substituted with a C₁-C₃ alkyl group at position 4;
when Z is the same as described above, and R² is a hydrogen atom, R³ is neither pyridine bound at position 3 and substituted with a C₁-C₃ alkyl group at position 4, nor non-substituted pyrazole bound at position 4;
when Z is the same as described above, R¹ is a C₁-C₃ alkyl group and R² is a hydrogen atom, R³ is neither 4-cyclopropylpyridine bound at position 3, nor non-substituted pyrimidine bound at position 5; and
when Z is: 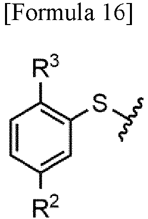 , R³ is neither thiophene bound at position 4 and substituted with R⁹ at position 3, pyridine bound at position 3 and substituted with a C₁-C₃ alkyl group at position 4, non-substituted pyrazole bound at position 4, non-substituted pyrimidine bound at position 5, nor pyrimidine bound at position 5 and substituted with a C₁-C₃ alkyl group at position 4].

4. The compound or pharmacologically acceptable salt thereof according to claim 3, wherein R³ is selected from the group consisting of the following formulas: 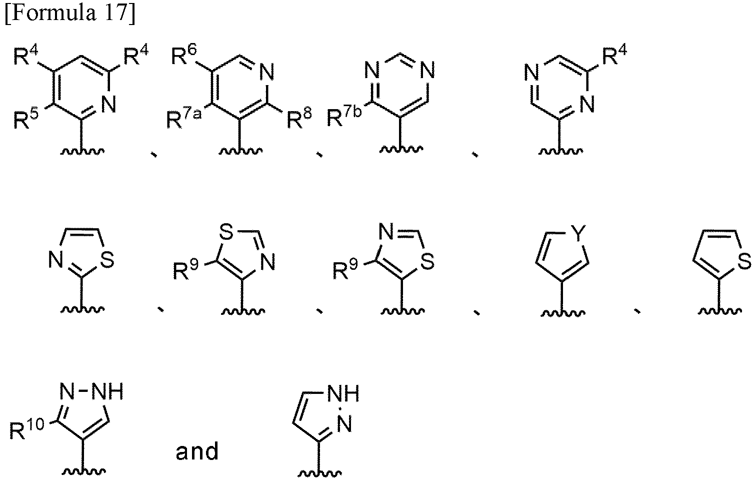
[wherein Y represents an oxygen atom or a sulfur atom,
R⁴ each independently represents a hydrogen atom or a C₁-C₃ alkoxy group,
R⁵ represents a hydrogen atom, a fluorine atom, or a C₁-C₃ alkoxy group,
R⁶ represents a hydrogen atom, a fluorine atom, or a C₁-C₃ alkoxy group,
R^{7a} represents a hydrogen atom, a C₁-C₃ alkoxy group, or a cyclopropyl group,
R^{7b} represents a hydrogen atom, a C₁-C₃ alkyl group, a C₁-C₃ alkoxy group, or a cyclopropyl group,
R⁸ represents a hydrogen atom or a C₁-C₃ alkoxy group,
R⁹ represents a hydrogen atom or a C₁-C₃ alkyl group,
R¹⁰ represents a hydrogen atom, a C₁-C₃ alkyl group, a C₁-C₃ alkoxy group, or a cyano group, and
when R⁶ is a C₁-C₃ alkoxy group, R⁷ and R⁸ are hydrogen atoms; and when R⁸ is a C₁-C₃ alkoxy group, R⁶ is a hydrogen atom or a fluorine atom, and R⁷ is a hydrogen atom,
provided that when Z is: 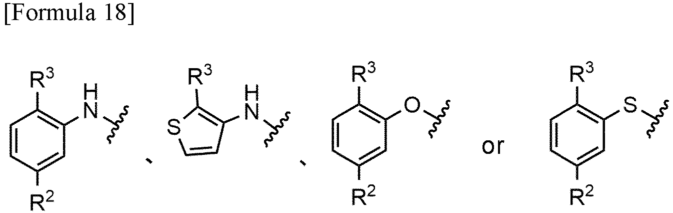 , R³ is neither pyridine bound at position 2 and substituted with a C₁-C₃ alkoxy group at position 3 nor pyridine bound at position 5 and substituted with a C₁-C₃ alkoxy group at position 3].

5. The compound or pharmacologically acceptable salt thereof according to claim 4, wherein R³ is selected from the group consisting of the following formulas: 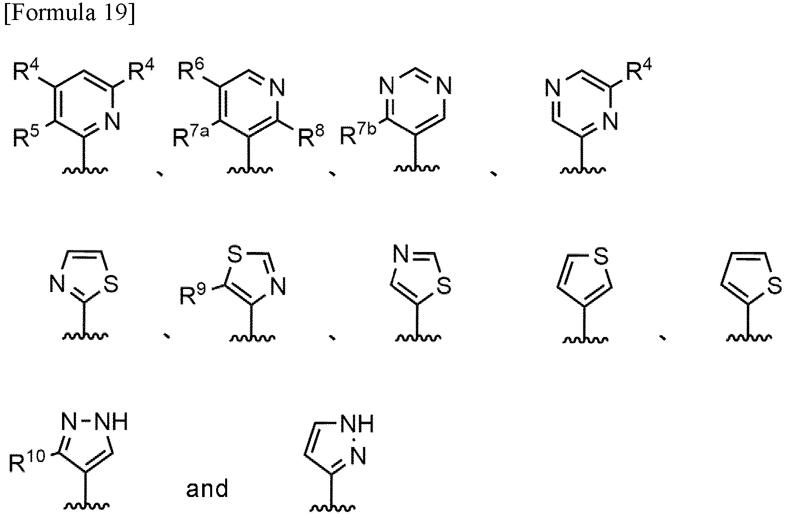
[wherein R⁴ each independently represents a hydrogen atom or a methoxy group,
R⁵ represents a hydrogen atom, a fluorine atom, or a methoxy group,
R⁶ represents a hydrogen atom, a fluorine atom, or a methoxy group,
R^{7a} represents a hydrogen atom, a methoxy group, or a cyclopropyl group,
R^{7b} represents a hydrogen atom, a methyl group, or a methoxy group,
R⁸ represents a hydrogen atom or a methoxy group,
R⁹ represents a hydrogen atom or a methyl group,
R¹⁰ represents a hydrogen atom, a methyl group, a methoxy group, or a cyano group, and
when R⁶ is a methoxy group, R^{7a} and R⁸ are hydrogen atoms; when R⁶ is a fluorine atom, R⁸ is a hydrogen atom; and when R⁸ is a methoxy group, R⁶ is a hydrogen atom or a fluorine atom, and R^{7a} is a hydrogen atom,
provided that when Z is: 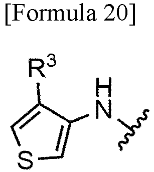
, R³ is not 3-methylpyrazole bound at position 4;
when Z is: 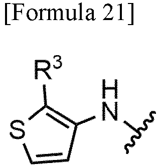
, R³ is neither non-substituted pyridine bound at position 3, 3-methylpyrazole bound at position 4, 2-methoxypyridine bound at position 3, 4-methoxypyridine bound at position 3, nor non-substituted thiophene bound at position 2;
when Z is: 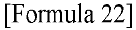 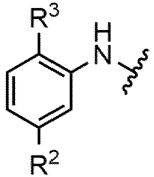 , R³ is neither non-substituted pyrimidine bound at position 5, 3-methylpyrazole bound at position 4, 2-methoxypyridine bound at position 3, 4-methoxypyridine bound at position 3, nor non-substituted thiophene bound at position 2;
when Z is: 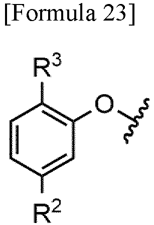 , and R² is a methyl group, R³ is neither non-substituted pyridine bound at position 3, 2-methoxypyridine bound at position 3, 4-methoxypyridine bound at position 3, nor non-substituted thiophene bound at position 2;
when Z is the same as described above, and R² is a hydrogen atom, R³ is neither non-substituted pyridine bound at position 3, 3-methylpyrazole bound at position 4, nor non-substituted thiophene bound at position 2;
when Z is the same as described above, and R² is a fluorine atom, R³ is neither non-substituted pyridine bound at position 3, 4-methylpyrimidine bound at position 5, 2-methoxypyridine bound at position 3, nor non-substituted thiophene bound at position 2; and
when Z is: 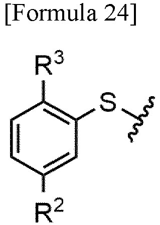
, R³ is neither non-substituted pyridine bound at position 3, 3-methylpyrazole bound at position 4, 2-methoxypyridine bound at position 3, 4-methoxypyridine bound at position 3, nor non-substituted thiophene bound at position 2].

6. The compound or pharmacologically acceptable salt thereof according to claim 5, wherein R³ is selected from the group consisting of the following formulas: 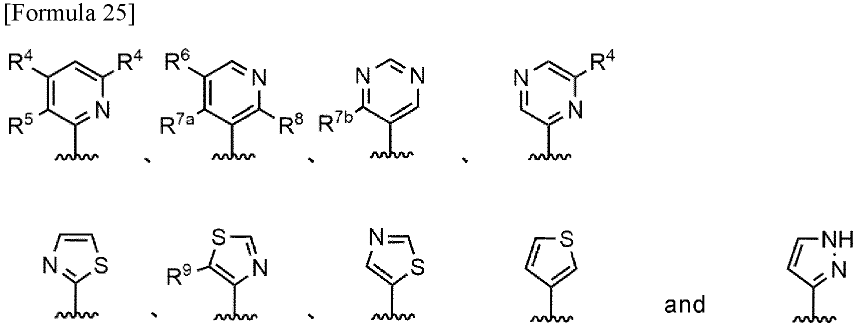
[wherein R⁴ each independently represents a hydrogen atom or a methoxy group,
R⁵ represents a hydrogen atom, a fluorine atom, or a methoxy group,
R⁶ represents a hydrogen atom, a fluorine atom, or a methoxy group,
R^{7a} represents a hydrogen atom, a methoxy group, or a cyclopropyl group,
R^{7b} represents a methoxy group,
R⁸ represents a hydrogen atom or a methoxy group,
R⁹ represents a hydrogen atom or a methyl group,
when R⁶ is a methoxy group, R^{7a} and R⁸ are hydrogen atoms; when R⁶ is a fluorine atom, R⁸ is a hydrogen atom; and when R^{7a} is a methoxy group, R⁶ is a fluorine atom, and R⁸ is a hydrogen atom,
provided that when Z is: 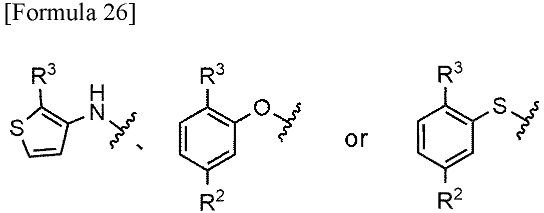 , R³ is not non-substituted pyridine bound at position 3].

7. The compound or pharmacologically acceptable salt thereof according to claims 2 to 6, wherein in the above general formula (I), Z is selected from the group consisting of the following formulas.

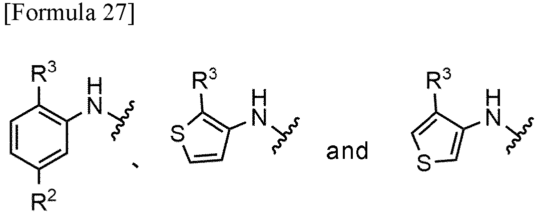

8. The compound or pharmacologically acceptable salt thereof according to claims 2 to 6, wherein in the above general formula (I), Z is selected from the group consisting of the following formulas.

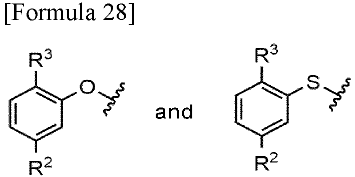

9. The compound or pharmacologically acceptable salt thereof according to claim 7, wherein in the above general formula (I), Z is the following formula: 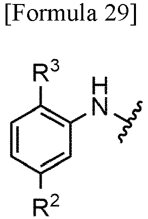
[wherein R² represents a hydrogen atom, a methyl group, or a fluorine atom, and
R³ is selected from the group consisting of the following formulas: 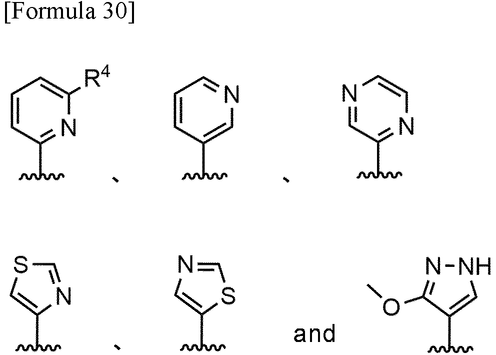
wherein R⁴ represents a hydrogen atom or a methoxy group].

10. The compound or pharmacologically acceptable salt thereof according to claim 8, wherein in the above general formula (I), Z is the following formula: 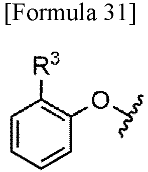
[wherein R³ is selected from the group consisting of the following formulas: 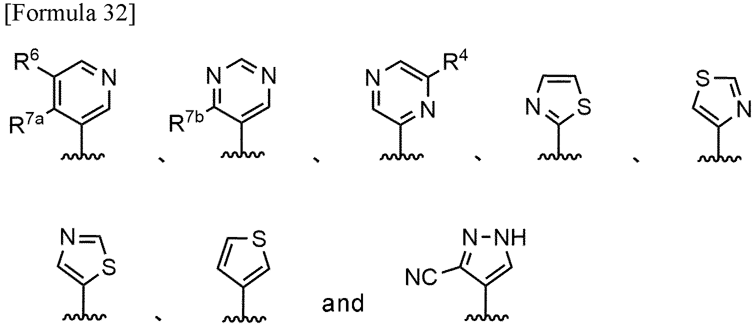
wherein R⁴ represents a hydrogen atom or a methoxy group
R⁶ represents a hydrogen atom or a fluorine atom,
R^{7a} represents a hydrogen atom, a methyl group, a methoxy group, or a cyclopropyl group,
R^{7b} represents a hydrogen atom, a methyl group, a methoxy group,
when R⁶ is a hydrogen atom, R^{7a} represents a methoxy group or a cyclopropyl group; when R^{7a} is a methyl group, R⁶ is a fluorine atom; and when R^{7a} is a cyclopropyl group, R⁶ is a hydrogen atom].

11. The compound or pharmacologically acceptable salt thereof according to claim 2, selected from the group consisting of the following compounds:
N-((1H-imidazol-4-yl)methyl)-[2,3'-bithiophen]-4'-amine;
N-((1H-imidazol-4-yl)methyl)-[2,2'-bithiophen]-3-amine;
N-((1H-imidazol-4-yl)methyl)-4-(pyridin-3-yl)thiophen-3-amine;
N-((1H-imidazol-4-yl)methyl)-4-(4-methoxypyridin-3-yl)thiophen-3-amine;
N-((1H-imidazol-4-yl)methyl)-4-(5-methoxypyridin-3-yl)thiophen-3-amine;
N-((1H-imidazol-4-yl)methyl)-4-(5-fluoropyridin-3-yl)thiophen-3-amine;
N-((1H-imidazol-4-yl)methyl)-2-(pyridin-3-yl)thiophen-3-amine;
N-((1H-imidazol-4-yl)methyl)-4-(1H-pyrazol-5-yl)thiophen-3-amine;
N-((1H-imidazol-4-yl)methyl)-2-(pyridin-3-yl)aniline;
N-((1H-imidazol-4-yl)methyl)-2-(pyrimidin-5-yl)aniline;
N-((1H-imidazol-4-yl)methyl)-2-(thiazol-5-yl)aniline;
N-((1H-imidazol-4-yl)methyl)-4-(thiazol-2-yl)thiophen-3-amine;
N-((1H-imidazol-4-yl)methyl)-4-(pyrazin-2-yl)thiophen-3-amine;
N-((1H-imidazol-4-yl)methyl)-4-(thiazol-4-yl)thiophen-3-amine;
N-((1H-imidazol-4-yl)methyl)-2-(thiazol-4-yl)aniline;
N-((1H-imidazol-4-yl)methyl)-4-(3-methylpyridin-2-yl)thiophen-3-amine;
N-((1H-imidazol-4-yl)methyl)-4-(3-methoxypyridin-2-yl)thiophen-3-amine;
N-((1H-imidazol-4-yl)methyl)-4-(4-methylthiazol-5-yl)thiophen-3-amine;
N-((1H-imidazol-4-yl)methyl)-2-(4-methylpyrimidin-5-yl)aniline;
N-((1H-imidazol-4-yl)methyl)-5-methyl-2-(4-methylpyrimidin-5-yl)aniline;
N-((1H-imidazol-4-yl)methyl)-4-(4-methoxypyridin-2-yl)thiophen-3-amine;2
N-((1H-imidazol-4-yl)methyl)-4-(6-methoxypyridin-2-yl)thiophen-3-amine;2
N-((1H-imidazol-4-yl)methyl)-4-(4-methoxypyrimidin-5-yl)thiophen-3-amine;
N-((1H-imidazol-4-yl)methyl)-4-(5-methylthiazol-4-yl)thiophen-3-amine;
N-((1H-imidazol-4-yl)methyl)-5-fluoro-2-(4-methylpyrimidin-5-yl)aniline;
N-((1H-imidazol-4-yl)methyl)-2-(pyrazin-2-yl)aniline;
N-((1H-imidazol-4-yl)methyl)-2-(pyridin-2-yl)aniline;
N-((1H-imidazol-4-yl)methyl)-2-(3-methoxypyridin-2-yl)aniline;
N-((1H-imidazol-4-yl)methyl)-4-(3-fluoropyridin-2-yl)thiophen-3-amine;
N-((1H-imidazol-4-yl)methyl)-4-(pyridin-2-yl)thiophen-3-amine;
N-((1H-imidazol-4-yl)methyl)-4-(6-methoxypyrazin-2-yl)thiophen-3-amine;
N-((1H-imidazol-4-yl)methyl)-2-(6-methoxypyridin-2-yl)aniline;
N-((1H-imidazol-4-yl)methyl)-2-(3-methoxy-1H-pyrazol-4-yl)-5-methylaniline;
N-((1H-imidazol-4-yl)methyl)-5-fluoro-2-(3-methoxy-1H-pyrazol-4-yl)aniline;
(+)-N-(1-(1H-imidazol-4-yl)ethyl)-2-(pyridin-3-yl)aniline;
(+)-N-(1-(1H-imidazol-4-yl)ethyl)-2-(6-methoxypyridin-2-yl)aniline;
(+)-N-(1-(1H-imidazol-4-yl)ethyl)-2-(thiazol-4-yl)aniline;
N-((1H-imidazol-4-yl)methyl)-4-(furan-3-yl)thiophen-3-amine;
N-((1H-imidazol-4-yl)methyl)-4-(pyrimidin-5-yl)thiophen-3-amine;
N-((1H-imidazol-4-yl)methyl)-4-(2-methoxypyridin-3-yl)thiophen-3-amine;
4-((2-(thiophen-3-yl)phenoxy)methyl)-1H-imidazole;
4-((2-(furan-2-yl)phenoxy)methyl)-1H-imidazole;
4-((2-(thiophen-2-yl)phenoxy)methyl)-1H-imidazole;
4-(2-((1H-imidazol-4-yl)methoxy)phenyl)-1H-pyrazole;
3-(2-((1H-imidazol-4-yl)methoxy)phenyl)pyridine;
4-(2-((1H-imidazol-4-yl)methoxy)phenyl)-3-methyl-1H-pyrazole;
4-(2-((1H-imidazol-4-yl)methoxy)phenyl)-1H-pyrazole-3-carbonitrile;
3-(2-((1H-imidazol-4-yl)methoxy)phenyl)-5-fluoropyridine;
3-(2-((1H-imidazol-4-yl)methoxy)phenyl)-2-methoxypyridine;
3-(2-((1H-imidazol-4-yl)methoxy)phenyl)-5-methoxypyridine;
3-(2-((1H-imidazol-4-yl)methoxy)phenyl)-4-methoxypyridine;
4-(2-((1H-imidazol-4-yl)methoxy)-4-methylphenyl)-1H-pyrazole;
4-(2-((1H-imidazol-4-yl)methoxy)-4-methylphenyl)-1H-pyrazole-3-carbonitrile;
3-(2-((1H-imidazol-4-yl)methoxy)-4-methylphenyl)-4-methylpyridine;
4-(2-((1H-imidazol-4-yl)methoxy)-4-fluorophenyl)-3-methyl-1H-pyrazole;
4-(2-((1H-imidazol-4-yl)methoxy)-4-methylphenyl)-3-methyl-1H-pyrazole;
3-(2-((1H-imidazol-4-yl)methoxy)phenyl)-5-fluoro-4-methylpyridine;
3-(2-((1H-imidazol-4-yl)methoxy)phenyl)-5-fluoro-2-methoxypyridine;
3-(2-((1H-imidazol-4-yl)methoxy)-4-methylphenyl)-4-methoxypyridine;
3-(2-((1H-imidazol-4-yl)methoxy)-4-fluorophenyl)-4-methoxypyridine;
5-(2-((1H-imidazol-4-yl)methoxy)-4-fluorophenyl)-4-methylpyrimidine;
5-(2-((1H-imidazol-4-yl)methoxy)-4-methylphenyl)-4-methylpyrimidine;
2-(2-((1H-imidazol-4-yl)methoxy)-4-methylphenyl)pyrazine;
4-(2-((1H-imidazol-4-yl)methoxy)phenyl)thiazole;
2-(2-((1H-imidazol-4-yl)methoxy)phenyl)pyrazine;
5-(2-((1H-imidazol-4-yl)methoxy)phenyl)pyrimidine;
2-(2-((1H-imidazol-4-yl)methoxy)phenyl)thiazole;
5-(2-((1H-imidazol-4-yl)methoxy)phenyl)thiazole;
3-(2-((1H-imidazol-4-yl)methoxy)phenyl)-4-methylpyridine;
3-(2-((1H-imidazol-4-yl)methoxy)phenyl)-4-cyclopropylpyridine;
3-(2-((1H-imidazol-4-yl)methoxy)phenyl)-5-fluoro-4-methoxypyridine;
5-(2-((1H-imidazol-4-yl)methoxy)phenyl)-4-methoxypyrimidine;
5-(2-((1H-imidazol-4-yl)methoxy)phenyl)-4-methylpyrimidine;
2-(2-((1H-imidazol-4-yl)methoxy)phenyl)-6-methoxypyrazine;
(+)-4-(1-(2-(thiophen-2-yl)phenoxy)ethyl)-1H-imidazole;
3-(2-(((1H-imidazol-4-yl)methyl)thio)phenyl)pyridine;
4-(2-(((1H-imidazol-4-yl)methyl)thio)phenyl)-1H-pyrazole; and
4-(((2-(thiophen-3-yl)phenyl)thio)methyl)-1H-imidazole.

12. The compound or pharmacologically acceptable salt thereof according to claim 4, selected from the group consisting of the following compounds:
N-((1H-imidazol-4-yl)methyl)-[2,3'-bithiophen]-4'-amine;
N-((1H-imidazol-4-yl)methyl)-[2,2'-bithiophen]-3-amine;
N-((1H-imidazol-4-yl)methyl)-4-(pyridin-3-yl)thiophen-3-amine;
N-((1H-imidazol-4-yl)methyl)-4-(4-methoxypyridin-3-yl)thiophen-3-amine;
N-((1H-imidazol-4-yl)methyl)-4-(5-methoxypyridin-3-yl)thiophen-3-amine;
N-((1H-imidazol-4-yl)methyl)-4-(5-fluoropyridin-3-yl)thiophen-3-amine;
N-((1H-imidazol-4-yl)methyl)-2-(pyridin-3-yl)thiophen-3-amine;
N-((1H-imidazol-4-yl)methyl)-4-(1H-pyrazol-5-yl)thiophen-3-amine;
N-((1H-imidazol-4-yl)methyl)-2-(pyridin-3-yl)aniline;
N-((1H-imidazol-4-yl)methyl)-2-(pyrimidin-5-yl)aniline;
N-((1H-imidazol-4-yl)methyl)-2-(thiazol-5-yl)aniline;
N-((1H-imidazol-4-yl)methyl)-4-(thiazol-2-yl)thiophen-3-amine;
N-((1H-imidazol-4-yl)methyl)-4-(pyrazin-2-yl)thiophen-3-amine;
N-((1H-imidazol-4-yl)methyl)-4-(thiazol-4-yl)thiophen-3-amine;
N-((1H-imidazol-4-yl)methyl)-2-(thiazol-4-yl)aniline;
N-((1H-imidazol-4-yl)methyl)-4-(3-methoxypyridin-2-yl)thiophen-3-amine;
N-((1H-imidazol-4-yl)methyl)-4-(4-methylthiazol-5-yl)thiophen-3-amine;
N-((1H-imidazol-4-yl)methyl)-2-(4-methylpyrimidin-5-yl)aniline;
N-((1H-imidazol-4-yl)methyl)-5-methyl-2-(4-methylpyrimidin-5-yl)aniline;
N-((1H-imidazol-4-yl)methyl)-4-(4-methoxypyridin-2-yl)thiophen-3-amine;
N-((1H-imidazol-4-yl)methyl)-4-(6-methoxypyridin-2-yl)thiophen-3-amine;
N-((1H-imidazol-4-yl)methyl)-4-(4-methoxypyrimidin-5-yl)thiophen-3-amine;
N-((1H-imidazol-4-yl)methyl)-4-(5-methylthiazol-4-yl)thiophen-3-amine;
N-((1H-imidazol-4-yl)methyl)-5-fluoro-2-(4-methylpyrimidin-5-yl)aniline;
N-((1H-imidazol-4-yl)methyl)-2-(pyrazin-2-yl)aniline;
N-((1H-imidazol-4-yl)methyl)-2-(pyridin-2-yl)aniline;
N-((1H-imidazol-4-yl)methyl)-4-(3-fluoropyridin-2-yl)thiophen-3-amine;
N-((1H-imidazol-4-yl)methyl)-4-(pyridin-2-yl)thiophen-3-amine;
N-((1H-imidazol-4-yl)methyl)-4-(6-methoxypyrazin-2-yl)thiophen-3-amine;
N-((1H-imidazol-4-yl)methyl)-2-(6-methoxypyridin-2-yl)aniline;
N-((1H-imidazol-4-yl)methyl)-2-(3-methoxy-1H-pyrazol-4-yl)-5-methylaniline;
N-((1H-imidazol-4-yl)methyl)-5-fluoro-2-(3-methoxy-1H-pyrazol-4-yl)aniline;
(+)-N-(1-(1H-imidazol-4-yl)ethyl)-2-(pyridin-3-yl)aniline;
(+)-N-(1-(1H-imidazol-4-yl)ethyl)-2-(6-methoxypyridin-2-yl)aniline;
(+)-N-(1-(1H-imidazol-4-yl)ethyl)-2-(thiazol-4-yl)aniline;
N-((1H-imidazol-4-yl)methyl)-4-(furan-3-yl)thiophen-3-amine;
N-((1H-imidazol-4-yl)methyl)-4-(2-methoxypyridin-3-yl)thiophen-3-amine;
4-((2-(thiophen-3-yl)phenoxy)methyl)-1H-imidazole;
4-((2-(thiophen-2-yl)phenoxy)methyl)-1H-imidazole;
3-(2-((1H-imidazol-4-yl)methoxy)phenyl)pyridine;
4-(2-((1H-imidazol-4-yl)methoxy)phenyl)-3-methyl-1H-pyrazole;
4-(2-((1H-imidazol-4-yl)methoxy)phenyl)-1H-pyrazole-3-carbonitrile;
3-(2-((1H-imidazol-4-yl)methoxy)phenyl)-5-fluoropyridine;
3-(2-((1H-imidazol-4-yl)methoxy)phenyl)-2-methoxypyridine;
3-(2-((1H-imidazol-4-yl)methoxy)phenyl)-4-methoxypyridine;
4-(2-((1H-imidazol-4-yl)methoxy)-4-methylphenyl)-1H-pyrazole;
4-(2-((1H-imidazol-4-yl)methoxy)-4-methylphenyl)-1H-pyrazole-3-carbonitrile;
4-(2-((1H-imidazol-4-yl)methoxy)-4-fluorophenyl)-3-methyl-1H-pyrazole;
4-(2-((1H-imidazol-4-yl)methoxy)-4-methylphenyl)-3-methyl-1H-pyrazole;
3-(2-((1H-imidazol-4-yl)methoxy)phenyl)-5-fluoro-4-methylpyridine;
3-(2-((1H-imidazol-4-yl)methoxy)phenyl)-5-fluoro-2-methoxypyridine;
3-(2-((1H-imidazol-4-yl)methoxy)-4-methylphenyl)-4-methoxypyridine;
3-(2-((1H-imidazol-4-yl)methoxy)-4-fluorophenyl)-4-methoxypyridine;
5-(2-((1H-imidazol-4-yl)methoxy)-4-fluorophenyl)-4-methylpyrimidine;
2-(2-((1H-imidazol-4-yl)methoxy)-4-methylphenyl)pyrazine;
4-(2-((1H-imidazol-4-yl)methoxy)phenyl)thiazole;
2-(2-((1H-imidazol-4-yl)methoxy)phenyl)pyrazine;
5-(2-((1H-imidazol-4-yl)methoxy)phenyl)pyrimidine;
2-(2-((1H-imidazol-4-yl)methoxy)phenyl)thiazole;
5-(2-((1H-imidazol-4-yl)methoxy)phenyl)thiazole;
3-(2-((1H-imidazol-4-yl)methoxy)phenyl)-4-cyclopropylpyridine;
3-(2-((1H-imidazol-4-yl)methoxy)phenyl)-5-fluoro-4-methoxypyridine;
5-(2-((1H-imidazol-4-yl)methoxy)phenyl)-4-methoxypyrimidine;
5-(2-((1H-imidazol-4-yl)methoxy)phenyl)-4-methylpyrimidine;
2-(2-((1H-imidazol-4-yl)methoxy)phenyl)-6-methoxypyrazine;
(+)-4-(1-(2-(thiophen-2-yl)phenoxy)ethyl)-1H-imidazole; and
3-(2-(((1H-imidazol-4-yl)methyl)thio)phenyl)pyridine.

13. The compound or pharmacologically acceptable salt thereof according to claim 5, selected from the group consisting of the following compounds:
N-((1H-imidazol-4-yl)methyl)-[2,3'-bithiophen]-4'-amine;
N-((1H-imidazol-4-yl)methyl)-[2,2'-bithiophen]-3-amine;
N-((1H-imidazol-4-yl)methyl)-4-(pyridin-3-yl)thiophen-3-amine;
N-((1H-imidazol-4-yl)methyl)-4-(4-methoxypyridin-3-yl)thiophen-3-amine;
N-((1H-imidazol-4-yl)methyl)-4-(5-methoxypyridin-3-yl)thiophen-3-amine;
N-((1H-imidazol-4-yl)methyl)-4-(5-fluoropyridin-3-yl)thiophen-3-amine;
N-((1H-imidazol-4-yl)methyl)-4-(1H-pyrazol-5-yl)thiophen-3-amine;
N-((1H-imidazol-4-yl)methyl)-2-(pyridin-3-yl)aniline;
N-((1H-imidazol-4-yl)methyl)-2-(thiazol-5-yl)aniline;
N-((1H-imidazol-4-yl)methyl)-4-(thiazol-2-yl)thiophen-3-amine;
N-((1H-imidazol-4-yl)methyl)-4-(pyrazin-2-yl)thiophen-3-amine;
N-((1H-imidazol-4-yl)methyl)-4-(thiazol-4-yl)thiophen-3-amine;
N-((1H-imidazol-4-yl)methyl)-2-(thiazol-4-yl)aniline;
N-((1H-imidazol-4-yl)methyl)-4-(3-methoxypyridin-2-yl)thiophen-3-amine;
N-((1H-imidazol-4-yl)methyl)-5-methyl-2-(4-methylpyrimidin-5-yl)aniline;
N-((1H-imidazol-4-yl)methyl)-4-(4-methoxypyridin-2-yl)thiophen-3-amine;
N-((1H-imidazol-4-yl)methyl)-4-(6-methoxypyridin-2-yl)thiophen-3-amine;
N-((1H-imidazol-4-yl)methyl)-4-(4-methoxypyrimidin-5-yl)thiophen-3-amine;
N-((1H-imidazol-4-yl)methyl)-4-(5-methylthiazol-4-yl)thiophen-3-amine;
N-((1H-imidazol-4-yl)methyl)-5-fluoro-2-(4-methylpyrimidin-5-yl)aniline;
N-((1H-imidazol-4-yl)methyl)-2-(pyrazin-2-yl)aniline;
N-((1H-imidazol-4-yl)methyl)-2-(pyridin-2-yl)aniline;
N-((1H-imidazol-4-yl)methyl)-4-(3-fluoropyridin-2-yl)thiophen-3-amine;
N-((1H-imidazol-4-yl)methyl)-4-(pyridin-2-yl)thiophen-3-amine;
N-((1H-imidazol-4-yl)methyl)-4-(6-methoxypyrazin-2-yl)thiophen-3-amine;
N-((1H-imidazol-4-yl)methyl)-2-(6-methoxypyridin-2-yl)aniline;
N-((1H-imidazol-4-yl)methyl)-2-(3-methoxy-1H-pyrazol-4-yl)-5-methylaniline;
N-((1H-imidazol-4-yl)methyl)-5-fluoro-2-(3-methoxy-1H-pyrazol-4-yl)aniline;
(+)-N-(1-(1H-imidazol-4-yl)ethyl)-2-(pyridin-3-yl)aniline;
(+)-N-(1-(1H-imidazol-4-yl)ethyl)-2-(6-methoxypyridin-2-yl)aniline;
(+)-N-(1-(1H-imidazol-4-yl)ethyl)-2-(thiazol-4-yl)aniline;
N-((1H-imidazol-4-yl)methyl)-4-(2-methoxypyridin-3-yl)thiophen-3-amine;
4-((2-(thiophen-3-yl)phenoxy)methyl)-1H-imidazole;
4-(2-((1H-imidazol-4-yl)methoxy)phenyl)-1H-pyrazole-3-carbonitrile;
3-(2-((1H-imidazol-4-yl)methoxy)phenyl)-5-fluoropyridine;
3-(2-((1H-imidazol-4-yl)methoxy)phenyl)-4-methoxypyridine;
4-(2-((1H-imidazol-4-yl)methoxy)-4-methylphenyl)-1H-pyrazole;
4-(2-((1H-imidazol-4-yl)methoxy)-4-methylphenyl)-1H-pyrazole-3-carbonitrile;
4-(2-((1H-imidazol-4-yl)methoxy)-4-fluorophenyl)-3-methyl-1H-pyrazole;
4-(2-((1H-imidazol-4-yl)methoxy)-4-methylphenyl)-3-methyl-1H-pyrazole;
3-(2-((1H-imidazol-4-yl)methoxy)phenyl)-5-fluoro-4-methylpyridine;
3-(2-((1H-imidazol-4-yl)methoxy)-4-fluorophenyl)-4-methoxypyridine;
2-(2-((1H-imidazol-4-yl)methoxy)-4-methylphenyl)pyrazine;
4-(2-((1H-imidazol-4-yl)methoxy)phenyl)thiazole;
2-(2-((1H-imidazol-4-yl)methoxy)phenyl)pyrazine;
5-(2-((1H-imidazol-4-yl)methoxy)phenyl)pyrimidine;
2-(2-((1H-imidazol-4-yl)methoxy)phenyl)thiazole;
5-(2-((1H-imidazol-4-yl)methoxy)phenyl)thiazole;
3-(2-((1H-imidazol-4-yl)methoxy)phenyl)-4-cyclopropylpyridine;
3-(2-((1H-imidazol-4-yl)methoxy)phenyl)-5-fluoro-4-methoxypyridine;
5-(2-((1H-imidazol-4-yl)methoxy)phenyl)-4-methoxypyrimidine;
5-(2-((1H-imidazol-4-yl)methoxy)phenyl)-4-methylpyrimidine; and
2-(2-((1H-imidazol-4-yl)methoxy)phenyl)-6-methoxypyrazine.

14. A pharmaceutical composition comprising the compound or pharmacologically acceptable salt thereof according to any of claims 1 to 12 as an active ingredient.

15. The pharmaceutical composition according to claim 14, for preventing or treating a disease selected from the group consisting of orthostatic hypotension, essential hypotension, acute hypotension associated with various diseases or conditions, and urinary incontinence.
